(19) Europäisches Patentamt
European Patent Office
Office européen des brevets

(11) **EP 4 365 159 B1**

(12) **EUROPEAN PATENT SPECIFICATION**

(45) Date of publication and mention of the grant of the patent:
**03.12.2025 Bulletin 2025/49**

(21) Application number: **23205510.3**

(22) Date of filing: **24.10.2023**

(51) International Patent Classification (IPC):
***C07C 17/263*** *(2006.01)* ***C07C 29/09*** *(2006.01)*
***C07C 67/10*** *(2006.01)* ***C07F 9/54*** *(2006.01)*

(52) Cooperative Patent Classification (CPC):
(C-Sets available)
**C07F 9/5442; C07C 17/2635; C07C 29/095; C07C 67/10** (Cont.)

(54) **ORGANOHALOGEN COMPOUND HAVING CONJUGATED DOUBLE BOND, PROCESS FOR PREPARING ACETATE COMPOUND AND ALCOHOL COMPOUND THEREOF, AND COMPOUND USED TO SYNTHESIZE THE ORGANOHALOGEN COMPOUND**

ORGANOHALOGENVERBINDUNG MIT KONJUGIERTER DOPPELBINDUNG, VERFAHREN ZUR HERSTELLUNG EINER ACETATVERBINDUNG UND EINER ALKOHOLVERBINDUNG DAVON UND VERBINDUNG, DIE VERWENDET WIRD, UM DIE ORGANOHALOGENVERBINDUNG ZU SYNTHETISIEREN

COMPOSÉ ORGANOHALOGÉNÉ AYANT UNE DOUBLE LIAISON CONJUGUÉE, PROCÉDÉ DE PRÉPARATION D'UN COMPOSÉ ACÉTATE ET D'UN COMPOSÉ ALCOOL DE CELUI-CI, ET COMPOSÉ UTILISÉ POUR SYNTHÉTISER LE COMPOSÉ ORGANOHALOGÉNÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **26.10.2022 JP 2022171337**

(43) Date of publication of application:
**08.05.2024 Bulletin 2024/19**

(73) Proprietor: **Shin-Etsu Chemical Co., Ltd.**
**Tokyo 100-0005 (JP)**

(72) Inventors:
- **YAMASHITA, Miyoshi**
**Niigata, 942-8601 (JP)**
- **WATANABE, Takeru**
**Niigata, 942-8601 (JP)**

(74) Representative: **De Vries & Metman**
**Overschiestraat 180**
**1062 XK Amsterdam (NL)**

(56) References cited:
**EP-A1- 3 556 742 WO-A2-2018/150379**

- **BUCHTA EMIL ET AL: "�ber eine Synthese des all-trans -1.1.10.10-Tetracarb�thoxy-3.8-dimethyl-decatriens-(3.5.7) und des all-trans -4.9-Dimethyl-dodecapentaen-(2.4.6.8.10)-dis�ure-(1.12)-dimethylesters", JUSTUS LIEBIGS ANNALEN DER CHEMIE, vol. 640, no. 1, 2 March 1961 (1961-03-02), DE, pages 29 - 37, XP093142076, ISSN: 0075-4617, DOI: 10.1002/jlac.19616400106**
- **YEN YAO-PIN ET AL: "Synthesis of the Sex Pheromone of Cocoa Pod Borer Moth, Conopomorpha Cramerella", vol. 22, no. 11, 1 June 1992 (1992-06-01), US, pages 1567 - 1581, XP093142074, ISSN: 0039-7911, Retrieved from the Internet <URL:https://dx.doi.org/10.1080/00397919208021630> DOI: 10.1080/00397919208021630**
- **DATABASE CAPLUS [online] 1 January 1992 (1992-01-01), YEN PIN: "Synthesis of the sex pheromone of cocoa pod borer moth, Conopomorpha cramerella", XP093142075, Database accession no. 1992:591523**

EP 4 365 159 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 17/2635, C07C 21/19;**
**C07C 17/2635, C07C 21/215;**
**C07C 17/2635, C07C 22/04;**
**C07C 29/095, C07C 33/02;**
**C07C 67/10, C07C 69/145;**
**C07C 67/10, C07C 69/157**

## Description

### TECHNICAL FIELD

The present invention relates to an organohalogen compound having a conjugated double bond, and a process for preparing an acetate compound and an alcohol compound thereof. The present invention also provides a (ω-halo-2-alkenyl)triphenylphosphonium halide compound used to synthesize the organohalogen compound.

### BACKGROUND ART

The Cocoa pod borer (scientific name: *Conopomorpha cramerella*) belonging to the Gracillariidae family of Lepidoptera is known as one of the most serious pests of Cacao (scientific name: *Theobroma cacao*) grown in Southeast Asia. The Cocoa pod borer lays eggs on the surfaces of cocoa pods. The hatched larvae penetrate to the pod interior and feed on the pulp, thereby reducing cacao bean yield and quality.

Pesticides are commonly used to control the Cocoa pod borer, but are insufficiently effective because the larvae penetrate into the pod directly after hatching.

Due to the adverse effects of pesticides on the environment and human health, there is recently a demand for the development of new eco-friendly and safe pest control technology, such as mating disruption and mass trapping using insect sex pheromones. The development of such pest control technology requires economical industrial preparation of sex pheromones in large quantities.

The sex pheromone of the Cocoa pod borer is reported to be a mixture of sex pheromones having a component ratio of 60:40:6:4:10 of (4*E*,6*E*,10*Z*)-4,6,10-hexadecatrienyl acetate, (4*E*,6*Z*,10*Z*)-4,6,10-hexadecatrienyl acetate, (4*E*,6*E*,10*Z*)-4,6,10-hexadecatrienyl alcohol, (4*E*,6*Z*,10*Z*)-4,6,10-hexadecatrienyl alcohol, and n-hexadecyl alcohol (Non-Patent Literature 1 below).

Among these sex pheromones, the acetate compounds, (4*E*,6*E*,10*Z*)-4,6,10-hexadecatrienyl acetate and (4*E*,6*Z*,10*Z*)-4,6,10-hexadecatrienyl acetate, are widely known to be synthesized by using a Wittig reaction to construct the double bond of position 6. The acetate compounds thus obtained can be hydrolyzed to synthesize (4*E*,6*E*,10*Z*)-4,6,10-hexadecatrienyl alcohol and (4*E*,6*Z*,10*Z*)-4,6,10-hexadecatrienyl alcohol.

Non-Patent Literature 1 below, for example, reports a process for synthesizing (4*E*,6*Z*,10*Z*)-4,6,10-hexadecatrienyl acetate by preparing phosphorus ylide by reacting (4*Z*)-4-decenyltriphenylphosphonium bromide with potassium *t*-butoxide as a base, and then subjecting the phosphorus ylide to a Wittig reaction with (2*E*)-6-[(tetrahydropyranyl)oxy]-2-hexenal. Non-Patent Literature 1 also reports a process for synthesizing (4*E*,6*E*,10*Z*)-4,6,10-hexadecatrienyl acetate by isomerizing the geometric structure of the double bond of the obtained (4*E*,6*Z*,10*Z*)-4,6,10-hexadecatrienyl acetate under sunlight with iodine as a catalyst, and by separating the resulting mixture of isomers by using liquid chromatography or a cheletropic reaction with liquid sulfur dioxide.

Non-Patent Literature 2 below reports a process for synthesizing (4*E*,6*Z*,10*Z*)-4,6,10-hexadecatrienyl acetate similar to that of Non-Patent Literature 1; and a process for synthesizing (4*E*,6*E*,10*Z*)-4,6,10-hexadecatrienyl acetate by preparing an ylide by reacting (2*E*)-6-[(tetrahydropyranyl)oxy]-2-hexenyltriphenylphosphonium bromide with n-butyllithium as a base, and then subjecting the ylide to a Wittig reaction with (4*Z*)-4-decenal.

Non-Patent Literature 3 below describes the synthesis of *all-trans*-1,1,10,10-tetracarbethoxy-3,8-dimethyldecatriene-(3,5,7) by condensation of 2 mole Na-diethyl malonate with *all-trans*-1,8-dibromo-2,7-dimethyl-octatriene-(2,4,6). A Wittig reaction between *all-trans*-2,7-dimethyl-octatriene-(2,4,6)-dial-(1,8) and 2 mole carbomethoxymethylene-triphenylphosphorane leads to the formation of *all-trans*-4,9-dimethyl-dodecapentaene-(2,4,6,8,10)-dicarboxy-(1,12)-dimethylester with 72% yield.

Patent Literature 1 below provides a process for preparing a 1-haloalkadiene of the general formula (1): $CH_3-(CH_2)_3-CH=CH-CH-CH-(CH_2)_a-X$, comprising a step of conducting a Wittig reaction between a haloalkenal of the general formula (2): $OHC-CH=CH-(CH_2)_a-X$, and a triarylphosphonium pentylide of the general formula (3): $CH_3-(CH_2)_3-CH^{-}-P^{+}Ar_3$, to obtain the 1-haloalkadiene. Patent Literature 1 also provides the use of a (7E,9Z)-1-halo-7,9-tetradecadiene obtained by the process for a process of preparing (9E,11Z)-9,11-hexadecadienyl acetate.

### PRIOR ART

[Non-Patent Literatures]

[Non-Patent Literature 1] P. S. Beevor et al., J. Chem. Ecol., 12, 1986, 1-23.
[Non-Patent Literature 2] Yao-Pin Yen et al., Synth. Commun., 22, 1992, 1567-1581.

[Non-Patent Literature 3] E. Buchta et al., Justus Liebigs Annalen der Chemie, 640, 1, 29-37

[Patent Literatures]

[Patent Literature 1] European patent application No. EP 3 556 742 A1

OBJECT OF THE INVENTION

However, the main product obtained by the processes for synthesizing (4*E*,6*Z*,10*Z*)-4,6,10-hexadecatrienyl acetate described in Non-Patent Literature 1 and Non-Patent Literature 2 is a geometric isomer in which the geometric structure of the double bond moiety is (4*E*,6*Z*,10*Z*). To obtain the geometric isomer ratio of the sex pheromone of the Cocoa pod borer, it is necessary to separately synthesize (4*E*,6*E*,10*Z*)-4,6,10-hexadecatrienyl acetate and mix to obtain the desired geometric isomer ratio.

Furthermore, the processes for synthesizing (4*E*,6*E*,10*Z*)-4,6,10-hexadecatrienyl acetate described in Non-Patent Literature 1 are problematic in that the method of using liquid chromatography to separate (4*E*,6*E*,10*Z*)-4,6,10-hexadecatrienyl acetate from a mixture of isomers obtained by an isomerization reaction of (4*E*,6*Z*,10*Z*)-4,6,10-hexadecatrienyl acetate has an extremely low yield; and in the separation method using a cheletropic reaction with liquid sulfur dioxide, the sulfur dioxide is highly toxic, and the reaction does not progress well when scaled up.

Moreover, the process for synthesizing (4*E*,6*E*,10*Z*)-4,6,10-hexadecatrienyl acetate described in Non-Patent Literature 2 is problematic in that the use of an ignitable organolithium compound requires a reaction at a cryogenic temperature of -78°C.

Thus, it is impossible to economically and industrially prepare large quantities using conventional technology.

The present invention has been made in view of the aforementioned circumstances, and aims to solve the problems of conventional art, and provide an economic industrial process for preparing synthetic intermediates, (4*E*,6*E*,10*Z*)-4,6,10-hexadecatrienyl halide and (4*E*,6*Z*,10*Z*)-4,6,10-hexadecatrienyl halide, of the sex pheromone of the Cocoa pod borer.

The present invention also aims to provide an economic industrial process for preparing the sex pheromone, (4*E*,6*E*,10*Z*)-4,6,10-hexadecatrienyl acetate, (4*E*,6*Z*,10*Z*)-4,6,10-hexadecatrienyl acetate, (4*E*,6*E*,10*Z*)-4,6,10-hexadecatrienyl alcohol, and (4*E*,6*Z*,10*Z*)-4,6,10-hexadecatrienyl alcohol, of the Cocoa pod borer by using the aforesaid organohalogen compound as an intermediate.

SUMMARY OF THE INVENTION

As a result of intensive research, the present inventors provided a novel compound, (ω-halo-2-alkenyl)triphenylphosphonium halide compound, and found that an organohalogen compound having a conjugated double bond can be prepared with good yield, without using toxic or ignitable starting materials, and in an industrially feasible reaction temperature range by subjecting the (ω-halo-2-alkenyl)triphenylphosphonium halide compound to a phosphorus ylide preparation reaction with alkali metal alkoxide as a base in the presence of lithium halide to obtain a reaction product mixture, and then subjecting the reaction product mixture to a Wittig reaction with an aldehyde compound, and thus have completed the present invention.

Also, the present inventors found that an acetate compound having a conjugated double bond can be industrially prepared efficiently and with fewer steps by subjecting the obtained organohalogen compound to an acetoxylation reaction, and thus have completed the present invention.

Furthermore, the present inventors found that an alcohol compound having a conjugated double bond can be industrially prepared efficiently and with fewer steps by subjecting the aforesaid acetate compound to a hydrolysis reaction, and thus have completed the present invention.

According to an aspect of the present invention, the present invention provides a process for preparing an organohalogen compound of the following general formula (5):

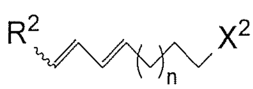
(5)

wherein $R^2$ represents a linear, branched, or aromatic monovalent hydrocarbon group having 1 to 10 carbon atoms, n is the number of methylene groups of 1 to 10, and $X^2$ represents a halogen atom,

the process comprising:

obtaining a reaction product mixture by subjecting a (ω-halo-2-alkenyl)triphenylphosphonium halide compound of the following general formula (1):

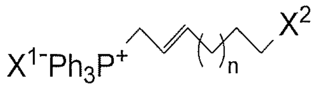

(1)

wherein n is as defined for the general formula (5) above, $X^1$ and $X^2$ represent, independently of each other, a halogen atom, and Ph represents a phenyl group,
to a phosphorus ylide preparation reaction with an alkali metal alkoxide of the following general formula (3):

$R^1OM$ (3)

wherein $R^1$ represents a linear or branched alkyl group having 1 to 6 carbon atoms, and M represents an alkali metal atom,
in the presence of a lithium halide of the following general formula (2):

$LiX^3$ (2)

wherein $X^3$ represents a halogen atom,
and then subjecting the aforesaid reaction product mixture to a Wittig reaction with an aldehyde compound of the following general formula (4):

$R^2CHO$ (4)

wherein $R^2$ is as defined for the general formula (5) above,
to form the organohalogen compound of the general formula (5) above.

According to another aspect of the present invention, the present invention provides a process for preparing an acetate compound having a conjugated double bond of the following general formula (6):

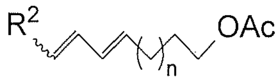

(6)

wherein $R^2$ and n are as defined for the general formula (5) above, and Ac represents an acetyl group,
the process comprising:

the aforesaid process for preparing the organohalogen compound (5), and
subjecting the organohalogen compound (5) to an acetoxylation reaction to form the acetate compound (6).

According to another aspect of the present invention, the present invention provides a process for preparing an alcohol compound having a conjugated double bond of the following general formula (7):

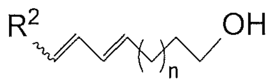

(7)

wherein $R^2$ and n are as defined for the general formula (5) above,
the process comprising:

the aforesaid process for preparing the acetate compound (6), and
subjecting the acetate compound (6) to a hydrolysis reaction to form the alcohol compound (7).

According to another aspect of the present invention, the present invention provides a process for preparing a (ω-halo-2-alkenyl)triphenylphosphonium halide compound of the following general formula (1):

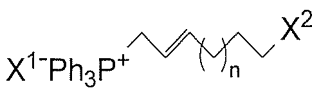

(1)

wherein n is the number of methylene groups of 1 to 10, $X^1$ and $X^2$ represent, independently of each other, a halogen atom, and Ph represents a phenyl group,

the process comprising subjecting a ω-halo-2-alkenyl halide compound of the following general formula (8):

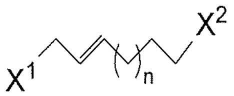

(8)

wherein *n*, $X^1$, and $X^2$ are as defined for the general formula (1) above,

to a substitution reaction with triphenylphosphine to form the (ω-halo-2-alkenyl)triphenylphosphonium halide compound (1).

According to an aspect of the present invention, the present invention provides a (ω-halo-2-alkenyl)triphenylphosphonium halide compound of the following general formula (1):

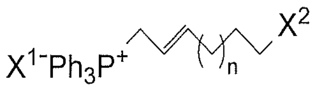

(1)

wherein *n* is the number of methylene groups of 1 to 10, $X^1$ and $X^2$ represent, independently of each other, a halogen atom, and Ph represents a phenyl group.

According to the present invention, an organohalogen compound having a conjugated double bond can be prepared with good yield without using toxic or ignitable starting materials, and in an industrially feasible reaction temperature range. According to the present invention, an acetate compound having a conjugated double bond can be industrially prepared efficiently and with fewer steps by subjecting the obtained organohalogen compound to an acetoxylation reaction. According to the present invention, an alcohol compound having a conjugated double bond can be industrially prepared efficiently and with fewer steps by subjecting the obtained acetate compound to a hydrolysis reaction.

The aforesaid novel compound, (ω-halo-2-alkenyl)triphenylphosphonium halide compound (1), also is effective as a synthetic intermediate of the sex pheromone, a mixture of (10*E*,12*E*)-10,12-tetradecadienyl acetate and (10*E*,12*Z*)-10,12-tetradecadienyl acetate, of the Western avocado leafroller (scientific name: *Amorbia cuneana*), one type of Tortricidae; and as a synthetic intermediate of an attractant, a mixture of (7*E*,9*E*)-7,9-decadienyl acetate and (7*E*,9*Z*)-7,9-decadienyl acetate, of the Tortricidae pest (scientific name: *Eucosma derelicta*).

## DETAILED DESCRIPTION OF THE INVENTION

Embodiments of the present invention will be described in detail below. It should be noted that the present invention is not limited to or by the embodiments but the invention is defined by the claims.

A. A novel compound, (ω-halo-2-alkenyl)triphenylphosphonium halide compound, of the following general formula (1) will be described in detail below.

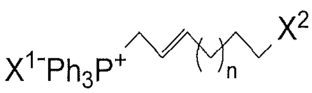

(1)

In the general formula (1) above, *n* is the number of methylene groups of 1 to 10, $X^1$ and $X^2$ represent, independently of each other, a halogen atom, and Ph represents a phenyl group.

When *n* is 1, 2, 3, 4, 5, 6, 7, 8, 9, and 10 in the general formula (1) above, examples of the compound include (6-halo-2-hexenyl)triphenylphosphonium halide (*n* = 1), (7-halo-2-heptenyl)triphenylphosphonium halide *(n* = 2), (8-halo-2-octenyl)triphenylphosphonium halide (*n* = 3), (9-halo-2-nonenyl)triphenylphosphonium halide (*n* = 4), (10-halo-2-decenyl) triphenylphosphonium halide (*n* = 5), (11-halo-2-undecenyl)triphenylphosphonium halide (*n* = 6), (12-halo-2-dodecenyl) triphenylphosphonium halide (*n* = 7), (13-halo-2-tridecenyl)triphenylphosphonium halide (*n* = 8), (14-halo-2-tetradecenyl) triphenylphosphonium halide (*n* = 9), and (15-halo-2-pentadecenyl)triphenylphosphonium halide (*n* = 10). *n* is preferably 1 to 8, and more preferably 1 to 4. By using said preferable *n* and said more preferable *n,* a preferable structural effectiveness of the obtained product and a more preferable structural effectiveness of the obtained product may be ensured.

In the general formula (1) above, $X^1$ and $X^2$ represent, independently of each other, a halogen atom. Examples of the halogen atom include a chlorine atom, a bromine atom, and an iodine atom.

In the general formula (1) above, examples of the compound, in which the halogen atoms of $X^1$ and $X^2$ in the combinations are independent of each other, include (ω-halo-2-alkenyl)triphenylphosphonium chloride compounds such as (ω-chloro-2-alkenyl)triphenylphosphonium chloride ($X^1$ = Cl, $X^2$ = Cl), (ω-bromo-2-alkenyl)triphenylphosphonium chloride ($X^1$ = Cl, $X^2$ = Br), and (ω-iodo-2-alkenyl)triphenylphosphonium chloride ($X^1$ = Cl, $X^2$ = I); (ω-halo-2-alkenyl) triphenylphosphonium bromide compounds such as (ω-chloro-2-alkenyl)triphenylphosphonium bromide ($X^1$ = Br, $X^2$ = Cl), (ω-bromo-2-alkenyl)triphenylphosphonium bromide ($X^1$ = Br, $X^2$ = Br), and (ω-iodo-2-alkenyl)triphenylphosphonium bromide ($X^1$ = Br, $X^2$ = I); and (ω-halo-2-alkenyl)triphenylphosphonium iodide compounds such as (ω-chloro-2-alkenyl) triphenylphosphonium iodide ($X^1$ = I, $X^2$ = Cl), (ω-bromo-2-alkenyl)triphenylphosphonium iodide ($X^1$ = I, $X^2$ = Br), and (ω-iodo-2-alkenyl)triphenylphosphonium iodide ($X^1$ = I, $X^2$ = I). The aforesaid compound is preferably (ω-chloro-2-alkenyl) triphenylphosphonium chloride ($X^1$ = Cl, $X^2$ = Cl), (ω-chloro-2-alkenyl)triphenylphosphonium bromide ($X^1$ = Br, $X^2$ = Cl), (ω-bromo-2-alkenyl)triphenylphosphonium bromide ($X^1$ = Br, $X^2$ = Br), (ω-chloro-2-alkenyl)triphenylphosphonium iodide ($X^1$ = I, $X^2$ = Cl), (ω-bromo-2-alkenyl)triphenylphosphonium iodide ($X^1$ = I, $X^2$ = Br), and (ω-iodo-2-alkenyl)triphenylphosphonium iodide ($X^1$ = I, $X^2$ = I). By using said (ω-chloro-2-alkenyl)triphenylphosphonium chloride ($X^1$ = Cl, $X^2$ = Cl), (ω-chloro-2-alkenyl)triphenylphosphonium bromide ($X^1$ = Br, $X^2$ = Cl), (ω-bromo-2-alkenyl)triphenylphosphonium bromide ($X^1$ = Br, $X^2$ = Br), (ω-chloro-2-alkenyl)triphenylphosphonium iodide ($X^1$ = I, $X^2$ = Cl), (ω-bromo-2-alkenyl)triphenylphosphonium iodide ($X^1$ = I, $X^2$ = Br), and (ω-iodo-2-alkenyl)triphenylphosphonium iodide ($X^1$ = I, $X^2$ = I), a preferable reactivity and/or selectivity may be ensured.

Examples of the (ω-halo-2-alkenyl)triphenylphosphonium halide compound (1) include the following compounds:

(ω-chloro-2-alkenyl)triphenylphosphonium chloride compounds such as (6-chloro-2-hexenyl)triphenylphosphonium chloride, (7-chloro-2-heptenyl)triphenylphosphonium chloride, (8-chloro-2-octenyl)triphenylphosphonium chloride, (9-chloro-2-nonenyl)triphenylphosphonium chloride, (10-chloro-2-decenyl)triphenylphosphonium chloride, (11-chloro-2-undecenyl)triphenylphosphonium chloride, (12-chloro-2-dodecenyl)triphenylphosphonium chloride, (13-chloro-2-tridecenyl)triphenylphosphonium chloride, (14-chloro-2-tetradecenyl)triphenylphosphonium chloride, and (15-chloro-2-pentadecenyl)triphenylphosphonium chloride;

(ω-bromo-2-alkenyl)triphenylphosphonium chloride compounds such as (6-bromo-2-hexenyl)triphenylphosphonium chloride, (7-bromo-2-heptenyl)triphenylphosphonium chloride, (8-bromo-2-octenyl)triphenylphosphonium chloride, (9-bromo-2-nonenyl)triphenylphosphonium chloride, (10-bromo-2-decenyl)triphenylphosphonium chloride, (11-bromo-2-undecenyl)triphenylphosphonium chloride, (12-bromo-2-dodecenyl)triphenylphosphonium chloride, (13-bromo-2-tridecenyl)triphenylphosphonium chloride, (14-bromo-2-tetradecenyl)triphenylphosphonium chloride, and (15-bromo-2-pentadecenyl)triphenylphosphonium chloride;

(ω-iodo-2-alkenyl)triphenylphosphonium chloride compounds such as (6-iodo-2-hexenyl)triphenylphosphonium chloride, (7-iodo-2-heptenyl)triphenylphosphonium chloride, (8-iodo-2-octenyl)triphenylphosphonium chloride, (9-iodo-2-nonenyl)triphenylphosphonium chloride, (10-iodo-2-decenyl)triphenylphosphonium chloride, (11-iodo-2-undecenyl)triphenylphosphonium chloride, (12-iodo-2-dodecenyl)triphenylphosphonium chloride, (13-iodo-2-tridecenyl)triphenylphosphonium chloride, (14-iodo-2-tetradecenyl)triphenylphosphonium chloride, and (15-iodo-2-pentadecenyl)triphenylphosphonium chloride;

(ω-chloro-2-alkenyl)triphenylphosphonium bromide compounds such as (6-chloro-2-hexenyl)triphenylphosphonium bromide, (7-chloro-2-heptenyl)triphenylphosphonium bromide, (8-chloro-2-octenyl)triphenylphosphonium bromide, (9-chloro-2-nonenyl)triphenylphosphonium bromide, (10-chloro-2-decenyl)triphenylphosphonium bromide, (11-chloro-2-undecenyl)triphenylphosphonium bromide, (12-chloro-2-dodecenyl)triphenylphosphonium bromide, (13-chloro-2-tridecenyl)triphenylphosphonium bromide, (14-chloro-2-tetradecenyl)triphenylphosphonium bromide, and (15-chloro-2-pentadecenyl)triphenylphosphonium bromide;

(ω-bromo-2-alkenyl)triphenylphosphonium bromide compounds such as (6-bromo-2-hexenyl)triphenylphosphonium bromide, (7-bromo-2-heptenyl)triphenylphosphonium bromide, (8-bromo-2-octenyl)triphenylphosphonium bromide, (9-bromo-2-nonenyl)triphenylphosphonium bromide, (10-bromo-2-decenyl)triphenylphosphonium bromide, (11-bromo-2-undecenyl)triphenylphosphonium bromide, (12-bromo-2-dodecenyl)triphenylphosphonium bromide, (13-bromo-2-tridecenyl)triphenylphosphonium bromide, (14-bromo-2-tetradecenyl)triphenylphosphonium bromide, and (15-bromo-2-pentadecenyl)triphenylphosphonium bromide;

(ω-iodo-2-alkenyl)triphenylphosphonium bromide compounds such as (6-iodo-2-hexenyl)triphenylphosphonium bromide, (7-iodo-2-heptenyl)triphenylphosphonium bromide, (8-iodo-2-octenyl)triphenylphosphonium bromide, (9-iodo-2-nonenyl)triphenylphosphonium bromide, (10-iodo-2-decenyl)triphenylphosphonium bromide, (11-iodo-2-undecenyl)triphenylphosphonium bromide, (12-iodo-2-dodecenyl)triphenylphosphonium bromide, (13-iodo-2-tridecenyl)triphenylphosphonium bromide, (14-iodo-2-tetradecenyl)triphenylphosphonium bromide, and (15-iodo-2-pentadecenyl)triphenylphosphonium bromide;

(ω-chloro-2-alkenyl)triphenylphosphonium iodide compounds such as (6-chloro-2-hexenyl)triphenylphosphonium iodide, (7-chloro-2-heptenyl)triphenylphosphonium iodide, (8-chloro-2-octenyl)triphenylphosphonium iodide, (9-chloro-2-nonenyl)triphenylphosphonium iodide, (10-chloro-2-decenyl)triphenylphosphonium iodide, (11-chloro-2-undecenyl)triphenylphosphonium iodide, (12-chloro-2-dodecenyl)triphenylphosphonium iodide, (13-chloro-2-tridecenyl)triphenylphosphonium iodide, (14-chloro-2-tetradecenyl)triphenylphosphonium iodide, and (15-chloro-2-pentadecenyl)triphenylphosphonium iodide;

(ω-bromo-2-alkenyl)triphenylphosphonium iodide compounds such as (6-bromo-2-hexenyl)triphenylphosphonium iodide, (7-bromo-2-heptenyl)triphenylphosphonium iodide, (8-bromo-2-octenyl)triphenylphosphonium iodide, (9-bromo-2-nonenyl)triphenylphosphonium iodide, (10-bromo-2-decenyl)triphenylphosphonium iodide, (11-bromo-2-undecenyl)triphenylphosphonium iodide, (12-bromo-2-dodecenyl)triphenylphosphonium iodide, (13-bromo-2-tridecenyl)triphenylphosphonium iodide, (14-bromo-2-tetradecenyl)triphenylphosphonium iodide, and (15-bromo-2-pentadecenyl)triphenylphosphonium iodide; and

(ω-iodo-2-alkenyl)triphenylphosphonium iodide compounds such as (6-iodo-2-hexenyl)triphenylphosphonium iodide, (7-iodo-2-heptenyl)triphenylphosphonium iodide, (8-iodo-2-octenyl)triphenylphosphonium iodide, (9-iodo-2-nonenyl)triphenylphosphonium iodide, (10-iodo-2-decenyl)triphenylphosphonium iodide, (11-iodo-2-undecenyl)triphenylphosphonium iodide, (12-iodo-2-dodecenyl)triphenylphosphonium iodide, (13-iodo-2-tridecenyl)triphenylphosphonium iodide, (14-iodo-2-tetradecenyl)triphenylphosphonium iodide, and (15-iodo-2-pentadecenyl)triphenylphosphonium iodide.

Further examples of the (ω-halo-2-alkenyl)triphenylphosphonium halide compound (1) include mixtures of *cis-trans* isomers of the double bond moiety.

Next, a process for preparing the (ω-halo-2-alkenyl)triphenylphosphonium halide compound (1) will be described.

The (ω-halo-2-alkenyl)triphenylphosphonium halide compound (1) is obtained by, for example, subjecting a ω-halo-2-alkenyl halide compound of the following general formula (8) to a substitution reaction with triphenylphosphine as in the following reaction formula.

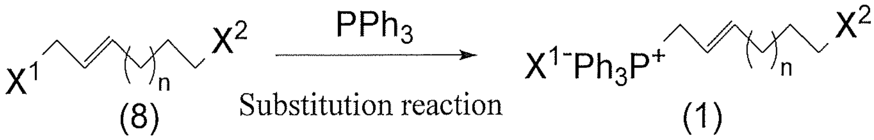

(8) (1)

The aforesaid process for synthesizing the (ω-halo-2-alkenyl)triphenylphosphonium halide compound (1) will be described in detail below.

The starting material, ω-halo-2-alkenyl halide compound (8), will be described.

In the general formula (8) above, *n*, $X^1$, and $X^2$ are as defined for the general formula (1) above.

When n is 1, 2, 3, 4, 5, 6, 7, 8, 9, and 10 in the general formula (8) above, examples of the compound include 6-halo-2-hexenyl halide (*n* = 1), 7-halo-2-heptenyl halide (*n* = 2), 8-halo-2-octenyl halide (*n* = 3), 9-halo-2-nonenyl halide (*n* = 4), 10-halo-2-decenyl halide (*n* = 5), 11-halo-2-undecenyl halide (*n* = 6), 12-halo-2-dodecenyl halide (*n* = 7), 13-halo-2-tridecenyl halide (*n* = 8), 14-halo-2-tetradecenyl halide (*n* = 9), and 15-halo-2-pentadecenyl halide (*n* = 10).

In the general formula (8) above, examples of the compound, in which the halogen atoms of $X^1$ and $X^2$ in the combinations are independent of each other, include ω-halo-2-alkenyl chloride compounds such as ω-chloro-2-alkenyl chloride ($X^1$ = Cl, $X^2$ = Cl), ω-bromo-2-alkenyl chloride ($X^1$ = Cl, $X^2$ = Br), and ω-iodo-2-alkenyl chloride ($X^1$ = Cl, $X^2$ = I); ω-halo-2-alkenyl bromide compounds such as ω-chloro-2-alkenyl bromide ($X^1$ = Br, $X^2$ = Cl), ω-bromo-2-alkenyl bromide ($X^1$ = Br, $X^2$ = Br), and ω-iodo-2-alkenyl bromide ($X^1$ = Br, $X^2$ = I); and ω-halo-2-alkenyl iodide compounds such as ω-chloro-2-alkenyl iodide ($X^1$ = I, $X^2$ = Cl), ω-bromo-2-alkenyl iodide ($X^1$ = I, $X^2$ = Br), and ω-iodo-2-alkenyl iodide ($X^1$ = I, $X^2$ = I). ω-Chloro-2-alkenyl chloride ($X^1$ = Cl, $X^2$ = Cl), ω-chloro-2-alkenyl bromide ($X^1$ = Br, $X^2$ = Cl), ω-bromo-2-alkenyl bromide ($X^1$ = Br, $X^2$ = Br), ω-chloro-2-alkenyl iodide ($X^1$ = I, $X^2$ = Cl), ω-bromo-2-alkenyl iodide ($X^1$ = I, $X^2$ = Br), and ω-iodo-2-alkenyl iodide ($X^1$ = I, $X^2$ = I) are preferred. By using said ω-chloro-2-alkenyl chloride ($X^1$ = Cl, $X^2$ = Cl), ω-chloro-2-alkenyl bromide ($X^1$ = Br, $X^2$ = Cl), ω-bromo-2-alkenyl bromide ($X^1$ = Br, $X^2$ = Br), ω-chloro-2-alkenyl iodide ($X^1$ = I, $X^2$ = Cl), ω-bromo-2-alkenyl iodide ($X^1$ = I, $X^2$ = Br), and ω-iodo-2-alkenyl iodide ($X^1$ = I, $X^2$ = I), a preferable reactivity and/or selectivity may be ensured.

Examples of the ω-halo-2-alkenyl halide compound (8) include the following compounds:

ω-chloro-2-alkenyl chloride compounds such as 6-chloro-2-hexenyl chloride, 7-chloro-2-heptenyl chloride, 8-chloro-2-octenyl chloride, 9-chloro-2-nonenyl chloride, 10-chloro-2-decenyl chloride, 11-chloro-2-undecenyl chloride, 12-chloro-2-dodecenyl chloride, 13-chloro-2-tridecenyl chloride, 14-chloro-2-tetradecenyl chloride, and 15-chloro-2-pentadecenyl chloride;

ω-bromo-2-alkenyl chloride compounds such as 6-bromo-2-hexenyl chloride, 7-bromo-2-heptenyl chloride, 8-bromo-2-octenyl chloride, 9-bromo-2-nonenyl chloride, 10-bromo-2-decenyl chloride, 11-bromo-2-undecenyl chloride, 12-bromo-2-dodecenyl chloride, 13-bromo-2-tridecenyl chloride, 14-bromo-2-tetradecenyl chloride, and 15-bromo-2-pentadecenyl chloride;

ω-iodo-2-alkenyl chloride compounds such as 6-iodo-2-hexenyl chloride, 7-iodo-2-heptenyl chloride, 8-iodo-2-octenyl chloride, 9-iodo-2-nonenyl chloride, 10-iodo-2-decenyl chloride, 11-iodo-2-undecenyl chloride, 12-iodo-2-dodecenyl chloride, 13-iodo-2-tridecenyl chloride, 14-iodo-2-tetradecenyl chloride, and 15-iodo-2-pentadecenyl chloride;

ω-chloro-2-alkenyl bromide compounds such as 6-chloro-2-hexenyl bromide, 7-chloro-2-heptenyl bromide, 8-chloro-2-octenyl bromide, 9-chloro-2-nonenyl bromide, 10-chloro-2-decenyl bromide, 11-chloro-2-undecenyl bromide, 12-chloro-2-dodecenyl bromide, 13-chloro-2-tridecenyl bromide, 14-chloro-2-tetradecenyl bromide, and 15-chloro-2-pentadecenyl bromide;

ω-bromo-2-alkenyl bromide compounds such as 6-bromo-2-hexenyl bromide, 7-bromo-2-heptenyl bromide, 8-bromo-2-octenyl bromide, 9-bromo-2-nonenyl bromide, 10-bromo-2-decenyl bromide, 11-bromo-2-undecenyl bromide, 12-bromo-2-dodecenyl bromide, 13-bromo-2-tridecenyl bromide, 14-bromo-2-tetradecenyl bromide, and 15-bromo-2-pentadecenyl bromide;

ω-iodo-2-alkenyl bromide compounds such as 6-iodo-2-hexenyl bromide, 7-iodo-2-heptenyl bromide, 8-iodo-2-octenyl bromide, 9-iodo-2-nonenyl bromide, 10-iodo-2-decenyl bromide, 11-iodo-2-undecenyl bromide, 12-iodo-2-dodecenyl bromide, 13-iodo-2-tridecenyl bromide, 14-iodo-2-tetradecenyl bromide, and 15-iodo-2-pentadecenyl bromide;

ω-chloro-2-alkenyl iodide compounds such as 6-chloro-2-hexenyl iodide, 7-chloro-2-heptenyl iodide, 8-chloro-2-octenyl iodide, 9-chloro-2-nonenyl iodide, 10-chloro-2-decenyl iodide, 11-chloro-2-undecenyl iodide, 12-chloro-2-dodecenyl iodide, 13-chloro-2-tridecenyl iodide, 14-chloro-2-tetradecenyl iodide, and 15-chloro-2-pentadecenyl iodide;

ω-bromo-2-alkenyl iodide compounds such as 6-bromo-2-hexenyl iodide, 7-bromo-2-heptenyl iodide, 8-bromo-2-octenyl iodide, 9-bromo-2-nonenyl iodide, 10-bromo-2-decenyl iodide, 11-bromo-2-undecenyl iodide, 12-bromo-2-dodecenyl iodide, 13-bromo-2-tridecenyl iodide, 14-bromo-2-tetradecenyl iodide, and 15-bromo-2-pentadecenyl iodide; and

ω-iodo-2-alkenyl iodide compounds such as 6-iodo-2-hexenyl iodide, 7-iodo-2-heptenyl iodide, 8-iodo-2-octenyl iodide, 9-iodo-2-nonenyl iodide, 10-iodo-2-decenyl iodide, 11-iodo-2-undecenyl iodide, 12-iodo-2-dodecenyl iodide, 13-iodo-2-tridecenyl iodide, 14-iodo-2-tetradecenyl iodide, and 15-iodo-2-pentadecenyl iodide.

Further examples of the ω-halo-2-alkenyl halide compound (8) include mixtures of *cis-trans* isomers of the double bond moiety.

The ω-halo-2-alkenyl halide compound (8) may be a commercially available one or may be synthesized in house.

The substitution reaction of the ω-halo-2-alkenyl halide compound (8) with triphenylphosphine may be carried out with heating or cooling, if necessary.

Triphenylphosphine used in the substitution reaction may be one after purification of a commercial one or a commercial one as-is.

The amount of triphenylphosphine used in the substitution reaction is preferably 0.2 mol to 5.0 mol, more preferably 0.5 mol to 3.0 mol, and most preferably 0.8 to 2.0 mol, per mol of the ω-halo-2-alkenyl halide compound (8). By using said more preferred amount and said most preferred amount, a more preferable yield and/or reactivity and a most preferable yield and/or reactivity may be ensured.

The solvent used in the substitution reaction may be any solvent that has no adverse effect on the substitution reaction. Examples of the solvent include halogen-based solvents such as methylene chloride, chloroform, carbon tetrachloride, and 1,2-dichloroethane; ether solvents such as diethyl ether, di-n-butyl ether, di-*t*-butyl ether, tetrahydrofuran, 2-methyltetrahydrofuran, 4-methyltetrahydropyran, 1,4-dioxane, and diethyleneglycol dimethyl ether; hydrocarbon solvents such as hexane, heptane, benzene, toluene, and xylene; ketone solvents such as acetone, methyl ethyl ketone, isobutyl methyl ketone, and cyclohexanone; alcoholic solvents such as methyl alcohol, ethyl alcohol, n-propyl alcohol, isopropyl alcohol, isobutyl alcohol, ethylene glycol, propylene glycol, and benzyl alcohol; ester solvents such as ethyl acetate, n-propyl acetate, n-butyl acetate, and isobutyl acetate; nitrile solvents such as acetonitrile; and aprotic polar solvents such as *N,N*-dimethylformamide, *N,N*-dimethylacetamide, dimethyl sulfoxide, and hexamethylphosphoric triamide. By using said ether solvents, hydrocarbon solvents, nitrile solvents and aprotic polar solvents, a preferable reactivity may be ensured.

The solvent may be used alone or in combination thereof, if necessary, and may be optionally determined while considering the reactivity and/or selectivity of the ω-halo-2-alkenyl halide compound (8). The solvent used may be one after purification of a commercial one or a commercial one as-is.

The amount of the solvent used in the substitution reaction may be optionally determined while considering the reactivity of the ω-halo-2-alkenyl halide compound (8). The amount of the solvent is, for example, preferably 100 g to

10,000 g, more preferably 200 g to 5,000 g, and most preferably 300 g to 2,000 g, per mol of the ω-halo-2-alkenyl halide compound (8). By using said more preferred amount and said most preferred amount, a more preferable reactivity and/or economy and a most preferable reactivity and/or economy may be ensured.

The reaction temperature of the substitution reaction may be optionally determined while considering the reactivity and/or the yield of the ω-halo-2-alkenyl halide compound (8). The reaction temperature is, for example, preferably -30°C to 200°C, more preferably 0°C to 150°C, and most preferably 20°C to 100°C. By using said more preferred reaction temperature and said most preferred reaction temperature, a more preferable reactivity and/or by-production of an impurity and a most preferred reactivity and/or by-production of an impurity may be ensured.

The reaction time of the substitution reaction is preferably optimized, depending on the reactivity of the ω-halo-2-alkenyl halide compound (8) by monitoring the reaction progress with, for example, gas chromatography and/or thin layer chromatography and/or nuclear magnetic resonance spectrum to confirm the disappearance of the ω-halo-2-alkenyl halide compound (8) and/or triphenylphosphine. The reaction time is typically, for example, preferably 0.5 hours to 72 hours, more preferably 0.5 hours to 24 hours, and most preferably 0.5 hours to 12 hours. By using said more preferred reaction time and said most preferred reaction time, a more preferable yield and/or by-production of an impurity and a most preferred yield and/or by-production of an impurity may be ensured.

The (ω-halo-2-alkenyl)triphenylphosphonium halide compound (1) obtained by the substitution reaction may be suitably isolated and/or purified in any purification method used in usual organic synthesis, such as distillation at a reduced pressure and/or recrystallization and/or various chromatography. Recrystallization is preferred. By using said recrystallization, a preferable physical property and/or industrial economy of the (ω-halo-2-alkenyl)triphenylphosphonium halide compound (1) may be ensured. When the (ω-halo-2-alkenyl)triphenylphosphonium halide compound (1) obtained by the substitution reaction has a sufficient purity, the crude product comprising the (ω-halo-2-alkenyl)triphenylphosphonium halide compound (1) may be used as such, without isolation or purification, in a subsequent step. After the substitution reaction, for example, the solution and/or the suspension which are reaction product mixtures may be used as such in a subsequent step.

B. A process for preparing an organohalogen compound of the following general formula (5) will be described in detail below.

As shown in the following reaction formula, the aforesaid (ω-halo-2-alkenyl)triphenylphosphonium halide compound (1) is subjected to a phosphorus ylide preparation reaction with alkali metal alkoxide of the following general formula (3) in the presence of lithium halide of the following general formula (2) to obtain a reaction product mixture, and then the reaction product mixture is subjected to a Wittig reaction with an aldehyde compound of the following general formula (4) to form the organohalogen compound (5).

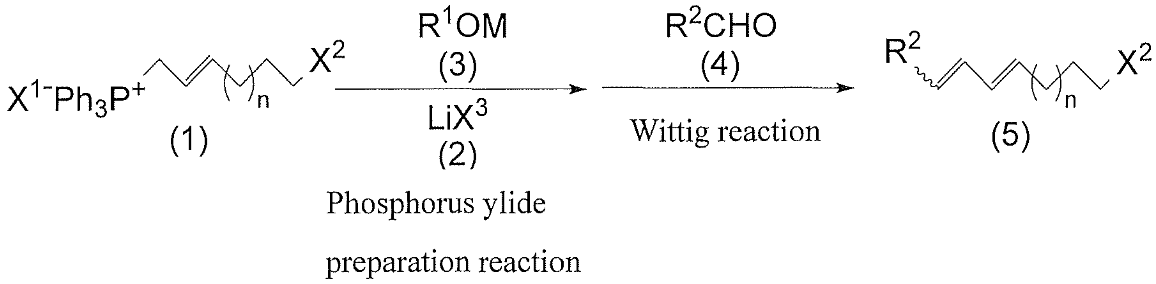


The starting material, (ω-halo-2-alkenyl)triphenylphosphonium halide compound (1), is as mentioned above.

The organohalogen compound (5) obtained by the phosphorus ylide preparation reaction and the subsequent Wittig reaction will be described in detail below.

In the general formula (5) above, $R^2$ represents a linear, branched, or aromatic monovalent hydrocarbon group of 1 to 10 carbon atoms, and *n* and $X^2$ are as defined for the general formula (1) above.

Examples of $R^2$ in the general formula (5) above include linear saturated monovalent hydrocarbon groups such as a methyl group, an ethyl group, an n-propyl group, an n-butyl group, an n-pentyl group, an n-heptyl group, and an n-nonyl group; linear unsaturated monovalent hydrocarbon groups such as a vinyl group, a 1-propenyl group, a 1-butenyl group, a 5-hexenyl group, and a (3*Z*)-3-nonenyl group; branched saturated monovalent hydrocarbon groups such as a 2-methylpropyl group, a 2-methyl-2-propyl group, a 2-pentyl group, and a 3-methylheptyl group; branched unsaturated monovalent hydrocarbon groups such as a 2-methyl-1-propenyl group and a 3-methyl-3-butenyl group; and aromatic monovalent hydrocarbon groups such as a phenyl group.

When the organohalogen compound (5) is synthesized from the (ω-halo-2-alkenyl)triphenylphosphonium halide compound (1), *n* and $X^2$ in the general formula (5) are as selected in the general formula (1). When the organohalogen compound (5) is synthesized from the aldehyde compound (4), $R^2$ in the general formula (5) is as selected in the general formula (4).

Examples of the organohalogen compound (5) include the following compounds:

linear organochlorine compounds such as (10*Z*)-4,6,10-hexadecatrienyl chloride, 4,6-hexadecadienyl chloride, 7,9-tetradecadienyl chloride, 7,9-dodecadienyl chloride, (11*E*)-7,9,11-tridecatrienyl chloride, and 11,13-hexadecadienyl chloride;
branched organochlorine compounds such as 10-methyl-4,6,10-undecatrienyl chloride, 8,8-dimethyl-4,6-nonadienyl chloride, 11-methyl-7,9-tetradecadienyl chloride, 12-methyl-7,9,11-tridecatrienyl chloride, and 14-methyl-9,11,13-pentadecatrienyl chloride;
aromatic organochlorine compounds such as 7-phenyl-4,6-heptadienyl chloride and 10-phenyl-7,9-decadienyl chloride;
linear organobromine compounds such as (10*Z*)-4,6,10-hexadecatrienyl bromide, 4,6-hexadecadienyl bromide, 7,9-tetradecadienyl bromide, 7,9-dodecadienyl bromide, (11*E*)-7,9,11-tridecatrienyl bromide, and 11,13-hexadecadienyl bromide;
branched organobromine compounds such as 10-methyl-4,6,10-undecatrienyl bromide, 8,8-dimethyl-4,6-nonadienyl bromide, 11-methyl-7,9-tetradecadienyl bromide, 12-methyl-7,9,11-tridecatrienyl bromide, and 14-methyl-9,11,13-pentadecatrienyl bromide;
aromatic organobromine compounds such as 7-phenyl-4,6-heptadienyl bromide and 10-phenyl-7,9-decadienyl bromide;
linear organoiodine compounds such as (10*Z*)-4,6,10-hexadecatrienyl iodide, 4,6-hexadecadienyl iodide, 7,9-tetradecadienyl iodide, 7,9-dodecadienyl iodide, (11*E*)-7,9,11-tridecatrienyl iodide, and 11,13-hexadecadienyl iodide;
branched organoiodine compounds such as 10-methyl-4,6,10-undecatrienyl iodide, 8,8-dimethyl-4,6-nonadienyl iodide, 11-methyl-7,9-tetradecadienyl iodide, 12-methyl-7,9,11-tridecatrienyl iodide, and 14-methyl-9,11,13-pentadecatrienyl iodide; and
aromatic organoiodine compounds such as 7-phenyl-4,6-heptadienyl iodide and 10-phenyl-7,9-decadienyl iodide.

The organohalogen compound (5) may be its enantiomers, diastereomers, and a mixture of such stereoisomers in the same or different amounts.
The aforesaid phosphorus ylide preparation reaction may be performed using alkali metal alkoxide (3) in the presence of lithium halide (2), and may be carried out with heating or cooling, if necessary.
$X^3$ in the general formula (2) above represents a halogen atom. Examples of the halogen atom include a chlorine atom, a bromine atom, and an iodine atom.
Examples of the lithium halide (2) include lithium chloride, lithium bromide, and lithium iodide. Lithium chloride and lithium bromide are preferred. By using said lithium chloride and lithium bromide, a preferable reactivity and/or economy may be ensured. The lithium halide (2) may be used alone or in combination thereof, if necessary. The lithium halide (2) used may be one after purification of a commercial one or a commercial one as-is.
The amount of the lithium halide (2) used varies, depending on the reactivity of the starting material, (ω-halo-2-alkenyl)triphenylphosphonium halide compound (1), to be used. The amount of the lithium halide (2) is preferably 0.1 mol to 5.0 mol, more preferably 0.5 mol to 3.0 mol, and most preferably 0.8 mol to 2.0 mol, per mol of the (ω-halo-2-alkenyl) triphenylphosphonium halide compound (1). By using said more preferred amount and said most preferred amount, more preferable economy and most preferred economy may be ensured.
In the general formula (3) above, $R^1$ represents a linear or branched alkyl group having 1 to 6 carbon atoms, and M represents an alkali metal atom.
Examples of $R^1$ in the general formula (3) above preferably include linear alkyl groups such as a methyl group and an ethyl group; and branched alkyl groups such as a *t*-butyl group, a 2-methyl-2-butyl group and a cyclohexyl group. In view of the availability and/or the ease of the preparation, the groups above may be preferred.
Examples of the alkali metal atom include a lithium atom, a sodium atom, and a potassium atom.
Examples of the alkali metal alkoxide (3) include lithium alkoxides such as lithium methoxide, lithium ethoxide, lithium *t*-butoxide, lithium 2-methyl-2-butoxide, and lithium cyclohexanoxide; sodium alkoxides such as sodium methoxide, sodium ethoxide, sodium t-butoxide, sodium 2-methyl-2-butoxide, and sodium cyclohexanoxide; and potassium alkoxides such as potassium methoxide, potassium ethoxide, potassium *t*-butoxide, potassium 2-methyl-2-butoxide, and potassium cyclohexanoxide. Sodium *t*-butoxide and potassium *t*-butoxide are more preferred. By using said sodium *t*-butoxide and potassium *t*-butoxide, a more preferred reactivity and/or yield may be ensured.
The alkali metal alkoxide (3) may be used alone or in combination thereof, if necessary. The alkali metal alkoxide (3) used may be one after purification of a commercial one or a commercial one as-is.
The alkali metal alkoxide (3) may be dissolved in, for example, a solvent and used as a solution.
Examples of the solvent include alcoholic solvents such as methyl alcohol, ethyl alcohol, *t*-butyl alcohol, 2-methyl-2-butyl alcohol, and cyclohexyl alcohol; ether solvents such as diethyl ether, di-n-butyl ether, di-*t*-butyl ether, tetrahydrofuran, 2-methyltetrahydrofuran, 4-methyltetrahydropyran, 1,4-dioxane, and diethyleneglycol dimethyl ether;

and aromatic hydrocarbon solvents such as benzene, toluene, and xylene. The solvent may be used alone or in combination thereof, if necessary.

The amount of the alkali metal alkoxide (3) used in the phosphorus ylide preparation reaction varies, depending on the reactivity of the starting material, (ω-halo-2-alkenyl)triphenylphosphonium halide compound (1), to be used. The amount of the alkali metal alkoxide (3) is preferably 0.1 mol to 5.0 mol, more preferably 0.5 mol to 3.0 mol, and most preferably 0.8 mol to 2.0 mol, per mol of the (ω-halo-2-alkenyl)triphenylphosphonium halide compound (1). By using said more preferred amount and said most preferred amount, more preferably economy and most preferred economy may be ensured.

The solvent used in the phosphorus ylide preparation reaction may be any solvent that has no adverse effect on the phosphorus ylide preparation reaction. Examples of the solvent used in the phosphorus ylide preparation reaction include ether solvents such as diethyl ether, di-n-butyl ether, di-*t*-butyl ether, tetrahydrofuran, 2-methyltetrahydrofuran, 4-methyltetrahydropyran, 1,4-dioxane, and diethyleneglycol dimethyl ether; hydrocarbon solvents such as hexane, heptane, benzene, toluene, and xylene; alcoholic solvents such as methyl alcohol, ethyl alcohol, n-propyl alcohol, isopropyl alcohol, isobutyl alcohol, *t*-butyl alcohol, 2-methyl-2-butyl alcohol, cyclohexyl alcohol, ethylene glycol, propylene glycol, and benzyl alcohol; ester solvents such as ethyl acetate, n-propyl acetate, n-butyl acetate, and isobutyl acetate; nitrile solvents such as acetonitrile; and aprotic polar solvents such as *N,N*-dimethylformamide, *N,N*-dimethylacetamide, dimethyl sulfoxide, and hexamethylphosphoric triamide. Ether solvent, hydrocarbon solvent, alcoholic solvent, and aprotic polar solvent are preferred. By using said ether solvent, hydrocarbon solvent, alcoholic solvent, and aprotic polar solvent, a preferable reactivity may be ensured.

The solvent may be used alone or in combination thereof, if necessary. The solvent used may be optionally determined while considering the reactivity and/or the yield of the (ω-halo-2-alkenyl)triphenylphosphonium halide compound (1), and may be one after purification of a commercial one or a commercial one as-is.

When the (ω-halo-2-alkenyl)triphenylphosphonium halide compound (1) is used in the phosphorus ylide preparation reaction as such, without isolation or purification, the solvent used may be a solvent used in the substitution reaction as such. The same solvent used in the substitution reaction or any solvent different from the solvent used in the substitution reaction may be added to the phosphorus ylide preparation reaction to increase the reactivity and/or adjust the concentration.

The amount of the solvent used in the phosphorus ylide preparation reaction may be optionally determined while considering the reactivity of the (ω-halo-2-alkenyl)triphenylphosphonium halide compound (1). The amount of the solvent is, for example, preferably 100 g to 10,000 g, more preferably 500 g to 5,000 g, and most preferably 800 g to 3,000 g, per mol of the (ω-halo-2-alkenyl)triphenylphosphonium halide compound (1). By using said more preferred amount and said most preferred amount, a more preferred reactivity and/or economy and a most preferred reactivity and/or economy may be ensured.

The reaction temperature of the phosphorus ylide preparation reaction may be optionally determined while considering the reactivity and/or the yield of the (ω-halo-2-alkenyl)triphenylphosphonium halide compound (1). The reaction temperature is, for example, preferably -35°C to 80°C, more preferably -20°C to 50°C, and most preferably -10°C to 20°C. By using said more preferred reaction temperature and said most preferred reaction temperature, a more preferred yield and/or by-production of an impurity and a most preferred yield and/or by-production of an impurity may be ensured.

The reaction time of the phosphorus ylide preparation reaction is preferably optimized, depending on the reactivity of the (ω-halo-2-alkenyl)triphenylphosphonium halide compound (1) by monitoring the reaction progress with, for example, gas chromatography and/or thin layer chromatography and/or nuclear magnetic resonance spectrum to confirm the disappearance of the (ω-halo-2-alkenyl)triphenylphosphonium halide compound (1). The reaction time is typically, for example, preferably 0.5 hours to 24 hours, more preferably 0.5 hours to 12 hours, and most preferably 0.5 hours to 6 hours. By using said more preferred reaction time and said most preferred reaction time, a more preferred yield and/or by-production of an impurity and a most preferred yield and/or by-production of an impurity may be ensured.

A reaction product mixture is obtained by the phosphorus ylide preparation reaction. A phosphorus ylide compound of the following general formula (9) is thought to be formed as the reaction product mixture in the reaction system.

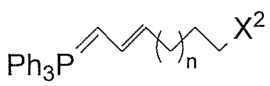

(9)

In the general formula (9) above, *n*, $X^2$, and Ph are as defined for the general formula (1) above.

The phosphorus ylide compound may be suitably isolated and/or purified in any purification method used in usual organic synthesis, such as distillation at a reduced pressure and/or recrystallization and/or various chromatography, and subsequently used in a subsequent Wittig reaction. When the properties of the phosphorus ylide compound make it difficult

to isolate and/or purify, the phosphorus ylide compound is preferably used as such in a subsequent Wittig reaction.

The obtained reaction product mixture may be considered to include at least the phosphorus ylide compound (9). The reaction product mixture optionally may include at least one of the (ω-halo-2-alkenyl)triphenylphosphonium halide compound (1), lithium halide (2), or alkali metal alkoxide (3), which is the starting material, of the phosphorus ylide preparation reaction. The reaction product mixture may be used as such in a subsequent step. The reaction product mixture after the phosphorus ylide preparation reaction, for example, may be used as such in the subsequent step. Or, the reaction product mixture may be purified and then used in the subsequent step. Unless otherwise noted in the present specification, the term "reaction product mixture" includes both purified and unpurified solutions or suspensions after the reaction.

The aforesaid Wittig reaction may be carried out using the phosphorus ylide compound (9) and the aldehyde compound (4) obtained by the phosphorus ylide preparation reaction, and may be carried out with heating or cooling, if necessary.

$R^2$ in the general formula (4) above is as defined for the general formula (5) above.

Examples of the aldehyde compound (4) include linear saturated aldehyde compounds such as acetaldehyde, n-propanal, n-butanal, n-pentanal, n-octanal, and n-decanal; linear unsaturated aldehyde compounds such as acrolein, (2*E*)-2-butenal, (2*E*)-2-pentenal, 6-heptenal, and (4*Z*)-4-decenal; branched saturated aldehyde compounds such as 3-methylbutanal, 2,2-dimethylpropanal, 2-methylpentanal, and 4-methyloctanal; branched unsaturated aldehyde compounds such as 3-methyl-2-butenal and 4-methyl-4-pentenal; and aromatic aldehyde compounds such as benzaldehyde.

The amount of the aldehyde compound (4) used in the Wittig reaction varies, depending on the reactivity of the phosphorus ylide compound (9) and/or the aldehyde compound (4) to be used. The amount of the aldehyde compound (4) is preferably 0.1 mol to 5.0 mol, more preferably 0.5 mol to 3.0 mol, and most preferably 0.8 mol to 2.0 mol, per mol of the (ω-halo-2-alkenyl)triphenylphosphonium halide compound (1) or the phosphorus ylide compound (9). By using said more preferred amount and said most preferred amount, a more preferred yield and/or economy and a most preferred yield and/or economy may be ensured.

The solvent used in the Wittig reaction may be any solvent that has no adverse effect on the Wittig reaction. Examples of the solvent used in the Wittig reaction include ether solvents such as diethyl ether, di-n-butyl ether, di-*t*-butyl ether, tetrahydrofuran, 2-methyltetrahydrofuran, 4-methyltetrahydropyran, 1,4-dioxane, and diethyleneglycol dimethyl ether; hydrocarbon solvents such as hexane, heptane, benzene, toluene, and xylene; alcoholic solvents such as methyl alcohol, ethyl alcohol, n-propyl alcohol, isopropyl alcohol, isobutyl alcohol, *t*-butyl alcohol, 2-methyl-2-butyl alcohol, cyclohexyl alcohol, ethylene glycol, propylene glycol, and benzyl alcohol; ester solvents such as ethyl acetate, n-propyl acetate, n-butyl acetate, and isobutyl acetate; nitrile solvents such as acetonitrile; and aprotic polar solvents such as *N,N*-dimethylformamide, *N,N*-dimethylacetamide, dimethyl sulfoxide, and hexamethylphosphoric triamide. Ether solvents, hydrocarbon solvents, alcoholic solvents, and aprotic polar solvents are preferred. By using said ether solvents, hydrocarbon solvents, alcoholic solvents, and aprotic polar solvents, a preferable reactivity may be ensured.

The solvent may be used alone or in combination thereof, if necessary, and may be optionally determined while considering the reactivity and/or the yield of the phosphorus ylide compound (9) and/or the aldehyde compound (4). The solvent used may be one after purification of a commercial one or a commercial one as-is.

When the phosphorus ylide compound (9) is used in the Wittig reaction as such, without isolation or purification, a solvent used in the phosphorus ylide preparation reaction may be used as such. The same solvent used in the phosphorus ylide preparation reaction or any solvent different from the solvent used in the phosphorus ylide preparation reaction may be added to the Wittig reaction to increase the reactivity and/or adjust the concentration.

The amount of the solvent used in the Wittig reaction may be optionally determined while considering the reactivity of the phosphorus ylide compound (9) and/or the aldehyde compound (4). The amount of the solvent is, for example, preferably 100 g to 10,000 g, more preferably 300 g to 5,000 g, and most preferably 500 g to 3,000 g, per mol of the (ω-halo-2-alkenyl)triphenylphosphonium halide compound (1) or the phosphorus ylide compound (9). By using said more preferred amount and said most preferred amount, a more preferred reactivity and/or economy and a most preferred reactivity and/or economy may be ensured.

The reaction temperature of the Wittig reaction may be optionally determined while considering the reactivity and/or the yield of the phosphorus ylide compound (9) and/or the aldehyde compound (4). The reaction temperature is, for example, preferably -35°C to 80°C, more preferably -20°C to 50°C, and most preferably -10°C to 30°C. By using said more preferred amount and said most preferred amount, a more preferred yield and/or by-production of an impurity and a most preferred yield and/or by-production of an impurity may be ensured.

The reaction time of the Wittig reaction is preferably optimized, depending on the reactivity of the phosphorus ylide compound (9) and/or the aldehyde compound (4) by monitoring the reaction progress with, for example, gas chromatography and/or thin layer chromatography and/or nuclear magnetic resonance spectrum to confirm the disappearance of the phosphorus ylide compound (9) and/or the aldehyde compound (4). The reaction time is typically, for example, preferably 0.5 hours to 24 hours, more preferably 0.5 hours to 12 hours, and most preferably 0.5 hours to 6 hours. By using said more preferred reaction time and said most preferred reaction time, a more preferred yield and/or by-production of an impurity and a most preferred yield and/or by-production of an impurity may be ensured.

The organohalogen compound (5) obtained by the phosphorus ylide preparation reaction and the subsequent Wittig reaction may be suitably isolated and/or purified in any purification method used in usual organic synthesis, such as distillation at a reduced pressure and/or various chromatography. Distillation at a reduced pressure is preferred. By using said distillation at a reduced pressure, preferable industrial economy may be ensured. When the organohalogen compound (5) has a sufficient purity, the crude product comprising the organohalogen compound (5) may be used as such, without isolation or purification, in a subsequent step.

C. A process for preparing an acetate compound of the following general formula (6) will be described in detail below.

The acetate compound (6) is obtained by subjecting the aforesaid organohalogen compound (5) to an acetoxylation reaction as shown in the following reaction formula.

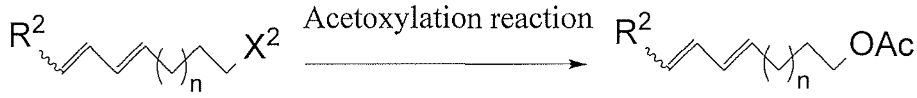


(5) (6)

The starting material, organohalogen compound (5), is as mentioned above.

The acetate compound (6) obtained by the aforesaid acetoxylation reaction will be described in detail below.

In the general formula (6) above, *n* is as defined for the general formula (1) above, $R^2$ is as defined for the general formula (5) above, and Ac represents an acetyl group. When the acetate compound (6) is synthesized from the organohalogen compound (5), $R^2$ and *n* in the general formula (6) above are as selected in the general formula (5) above.

Examples of the acetate compound (6) include linear acetate compounds such as (10*Z*)-4,6,10-hexadecatrienyl acetate, 4,6-hexadecadienyl acetate, 7,9-tetradecadienyl acetate, 7,9-dodecadienyl acetate, (11*E*)-7,9,11-tridecatrienyl acetate, and 11,13-hexadecadienyl acetate; branched acetate compounds such as 10-methyl-4,6,10-undecatrienyl acetate, 8,8-dimethyl-4,6-nonadienyl acetate, 11-methyl-7,9-tetradecadienyl acetate, 12-methyl-7,9,11-tridecatrienyl acetate, and 14-methyl-9, 11,13-pentadecatrienyl acetate; and aromatic acetate compounds such as 7-phenyl-4,6-heptadienyl acetate and 10-phenyl-7,9-decadienyl acetate.

The acetate compound (6) may be its enantiomers, diastereomers, and a mixture of such stereoisomers in the same or different amounts.

The acetoxylation reaction may be carried out using a known acetoxylating agent, and may be carried out with heating or cooling, if necessary.

Examples of the acetoxylating agent used in the acetoxylation reaction include acetic acid or acetates. When acetic acid is used as the acetoxylating agent, the acetoxylation reaction may be carried out in the presence of a base.

Examples of the acetate used as the acetoxylating agent include metal acetates such as sodium acetate, lithium acetate, potassium acetate, silver acetate(I), copper acetate(I), lead acetate(II), and tri-n-butyltin acetate; and ammonium acetates such as tetramethylammonium acetate, tetraethylammonium acetate, tetra-n-butylammonium acetate, and 1-ethyl-3-methylimidazolium acetate. Sodium acetate and potassium acetate are preferred, and may be used alone or in combination thereof, if necessary. By using said sodium acetate and potassium acetate, a preferable yield and/or economy may be ensured.

The amount of the acetoxylating agent used is preferably 0.1 mol to 30.0 mol, more preferably 0.5 mol to 10.0 mol, and most preferably 1.0 mol to 5.0 mol. By using said more preferred amount and said most preferred amount, more preferably economy and most preferred economy may be ensured.

Examples of a base when acetic acid is used as the acetoxylating agent include alkali metal hydroxide salts such as sodium hydroxide, lithium hydroxide, and potassium hydroxide; alkali metal carbonates such as sodium carbonate, lithium carbonate, and potassium carbonate; and amines such as triethylamine and diisopropylethylamine. Sodium hydroxide, potassium hydroxide, and potassium carbonate are preferred. By using said sodium hydroxide, potassium hydroxide, and potassium carbonate, a preferable reactivity and/or yield may be ensured.

The base may be used alone or in combination thereof, if necessary, and may be optionally determined while considering the type and/or the reactivity of the organohalogen compound (5).

The amount of the base used is preferably 0.1 mol to 50.0 mol, more preferably 0.5 to 30.0 mol, and most preferably 1.0 mol to 10 mol, per mol of the organohalogen compound (5). By using said more preferred amount and said most preferred amount, more preferably economy and most preferred economy may be ensured.

A metal halide compound may be added to the acetoxylation reaction to increase the reactivity. Examples of the metal halide compound include metal iodide compounds such as sodium iodide, lithium iodide, and potassium iodide; and metal bromide compounds such as sodium bromide, lithium bromide, and potassium bromide. Sodium iodide, potassium iodide, sodium bromide, and potassium bromide are preferred. By using said sodium iodide, potassium iodide, sodium

bromide, and potassium bromide, a preferable reactivity and/or economy may be ensured.

The amount of the metal halide compound used is preferably 0.01 mol to 1.0 mol, more preferably 0.03 to 0.5 mol, and most preferably 0.05 mol to 0.3 mol, per mol of the organohalogen compound (5). By using said more preferred amount and said most preferred amount, more preferably economy and most preferred economy may be ensured.

The solvent used in the acetoxylation reaction may be any solvent that has no adverse effect on the acetoxylation reaction. Examples of the solvent include water; halogen-based solvents such as methylene chloride, chloroform, carbon tetrachloride, and 1,2-dichloroethane; ether solvents such as diethyl ether, di-n-butyl ether, di-t-butyl ether, tetrahydrofuran, 2-methyltetrahydrofuran, 4-methyltetrahydropyran, 1,4-dioxane, and diethyleneglycol dimethyl ether; hydrocarbon solvents such as hexane, heptane, benzene, toluene, and xylene; ketone solvents such as acetone, methyl ethyl ketone, isobutyl methyl ketone, and cyclohexanone; alcoholic solvents such as methyl alcohol, ethyl alcohol, n-propyl alcohol, isopropyl alcohol, isobutyl alcohol, ethylene glycol, propylene glycol, and benzyl alcohol; ester solvents such as ethyl acetate, n-propyl acetate, n-butyl acetate, and isobutyl acetate; nitrile solvents such as acetonitrile; and aprotic polar solvents such as *N,N*-dimethylformamide, *N,N*-dimethylacetamide, *N*-methyl-2-pyrrolidone, 1,3-dimethyl-2-imidazolidinone, dimethyl sulfoxide, and hexamethylphosphoric triamide. Ether solvents, hydrocarbon solvents, nitrile solvents, and aprotic polar solvents are preferred. By using said ether solvents, hydrocarbon solvents, nitrile solvents, and aprotic polar solvents, a preferable reactivity may be ensured.

The solvent may be used alone or in combination thereof, if necessary, and may be optionally determined while considering the reactivity and/or economy of the organohalogen compound (5). The solvent used may be one after purification of a commercial one or a commercial one as-is.

The amount of the solvent used in the acetoxylation reaction may be optionally determined while considering the reactivity of the organohalogen compound (5). The amount of the solvent is, for example, preferably 50 g to 5,000 g, more preferably 100 g to 2,000 g, and most preferably 200 g to 1,000 g, per mol of the organohalogen compound (5). By using said more preferred amount and said most preferred amount, a more preferred reactivity and/or by-production of an impurity and a most preferred reactivity and/or by-production of an impurity may be ensured.

The reaction temperature of the acetoxylation reaction may be optionally determined while considering the reactivity and/or the yield of the organohalogen compound (5). The reaction temperature is, for example, preferably 0°C to 200°C, more preferably 25°C to 180°C, and most preferably 50°C to 150°C. By using said more preferred reaction temperature and said most preferred reaction temperature, a more preferred reactivity and/or by-production of an impurity and a most preferred reactivity and/or by-production of an impurity may be ensured.

The reaction time of the acetoxylation reaction is preferably optimized, depending on the reactivity of the organohalogen compound (5) by monitoring the reaction progress with, for example, gas chromatography and/or thin layer chromatography and/or nuclear magnetic resonance spectrum to confirm the disappearance of the organohalogen compound (5). The reaction time is typically, for example, preferably 0.5 hours to 72 hours, more preferably 0.5 hours to 24 hours, and most preferably 0.5 hours to 12 hours. By using said more preferred reaction time and said most preferred reaction time, a more preferred yield and/or by-production of an impurity and a most preferred yield and/or by-production of an impurity may be ensured.

The acetate compound (6) obtained by the acetoxylation reaction may be suitably isolated and/or purified in any purification method used in usual organic synthesis, such as distillation at a reduced pressure and/or various chromatography. Distillation at a reduced pressure is preferred. By using said distillation at a reduced pressure, preferable industrial economy may be ensured. When the acetate compound (6) obtained by the acetoxylation reaction has a sufficient purity, the crude product comprising the acetate compound (6) may be used as such, without isolation or purification.

D. A process for preparing an alcohol compound of the following general formula (7) will be described in detail below.

The alcohol compound (7) is obtained by subjecting the aforesaid acetate compound (6) to a hydrolysis reaction as shown in the following reaction formula.

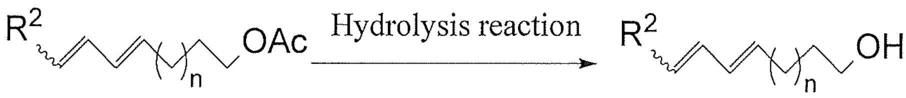


(6) (7)

The starting material, acetate compound (6), is as mentioned above.

The alcohol compound (7) obtained by the hydrolysis reaction will be described in detail below.

In the general formula (7) above, n is as defined for the general formula (1) above, and $R^2$ is as defined for the general formula (6) above. When the alcohol compound (7) is synthesized from the acetate compound (6), $R^2$ and *n* in the general formula (7) above are as selected in the general formula (6) above.

Examples of the alcohol compound (7) include linear alcohol compounds such as (10*Z*)-4,6,10-hexadecatrienyl

alcohol, 4,6-hexadecadienyl alcohol, 7,9-tetradecadienyl alcohol, 7,9-dodecadienyl alcohol, (11*E*)-7,9,11-tridecatrienyl alcohol, and 11,13-hexadecadienyl alcohol; branched alcohol compounds such as 10-methyl-4,6,10-undecatrienyl alcohol, 8,8-dimethyl-4,6-nonadienyl alcohol, 11-methyl-7,9-tetradecadienyl alcohol, 12-methyl-7,9,11-tridecatrienyl alcohol, and 14-methyl-9,11,13-pentadecatrienyl alcohol; and aromatic alcohol compounds such as 7-phenyl-4,6-heptadienyl alcohol and 10-phenyl-7,9-decadienyl alcohol.

The alcohol compound (7) may be its enantiomers, diastereomers, and a mixture of such stereoisomers in the same or different amounts.

The hydrolysis reaction may be carried out using a known hydrolysis reaction, and may be carried out with heating or cooling, if necessary.

The hydrolysis reaction may be carried out, for example, in a basic condition in the presence of a base, in an acidic condition in the presence of an acid, or in a neutral condition in the presence of a salt or a halogenated silane.

Examples of the base used in the hydrolysis in a basic condition include hydroxide salts such as sodium hydroxide, lithium hydroxide, potassium hydroxide, and barium hydroxide; carbonates or bicarbonates such as sodium carbonate, potassium carbonate, sodium bicarbonate, and potassium bicarbonate; and metal alkoxides such as sodium methoxide, sodium ethoxide, sodium *t*-butoxide, lithium methoxide, lithium ethoxide, lithium *t*-butoxide, potassium methoxide, potassium ethoxide, and potassium *t*-butoxide.

The base may be used alone or in combination thereof, if necessary, and may be optionally determined while considering the reactivity and/or the yield of the acetate compound (6). The base used may be one after purification of a commercial one or a commercial one as-is.

Examples of the acid used in the hydrolysis in an acidic condition include inorganic acids such as hydrochloric acid, hydrobromic acid, sulfuric acid, and nitric acid; organic acids such as acetic acid, formic acid, oxalic acid, trifluoroacetic acid, methanesulfonic acid, benzenesulfonic acid, and *p*-toluenesulfonic acid; and Lewis acids such as aluminum trichloride, aluminum ethoxide, aluminum isopropoxide, boron trifluoride, boron trichloride, boron tribromide, tin tetrachloride, tin tetrabromide, dibutyltin dichloride, dibutyltin dimethoxide, dibutyltin oxide, titanium tetrachloride, titanium tetrabromide, titanium(IV) methoxide, titanium(IV) ethoxide, and titanium(IV) isopropoxide.

The acid may be used alone or in combination thereof, if necessary, and may be optionally determined while considering the reactivity and/or the yield of the acetate compound (6). The acid used may be one after purification of a commercial one or a commercial one as-is.

Examples of the salt used in the hydrolysis in a neutral condition include lithium iodide, lithium bromide, sodium cyanide, potassium cyanide, lithium methanethiolate, and sodium benzenethiolate.

Examples of the halogenated silane used in the hydrolysis in a neutral condition include trimethylsilyl iodide and trimethylsilyl bromide.

The salt or the halogenated silane may be used alone, or if necessary, as a combination of salts, as a combination of halogenated silanes, or as any combination of salts and halogenated silanes, and may be optionally determined while considering the reactivity and/or the yield of the acetate compound (6). The salt or the halogenated silane used may be one after purification of a commercial one or a commercial one as-is.

The hydrolysis reaction is preferably hydrolysis in a basic condition, and more preferably a hydrolysis reaction with a hydroxide salt, a carbonate, or a bicarbonate. By using said hydrolysis reaction with a hydroxide salt, a carbonate, or a bicarbonate, a more preferred yield and/or by-production of an impurity may be ensured.

The amount of the base, acid, salt, or halogenated silane used in the hydrolysis reaction may be arbitrarily set in the range from a very low catalytic amount to a large excess, depending on the reactivity of the acetate compound (6) and is, for example, preferably 0.1 mol to 50 mol, more preferably 0.3 mol to 30 mol, and most preferably 0.5 mol to 10 mol, per mol of the acetate compound (6). By using said more preferred amount and said most preferred amount, a more preferred reaction time and/or yield and a most preferred reaction time and/or yield may be ensured.

The solvent used in the hydrolysis reaction may be any solvent that has no adverse effect on the hydrolysis reaction. Examples of the solvent include water; halogen-based solvents such as methylene chloride, chloroform, carbon tetrachloride, and 1,2-dichloroethane; ether solvents such as diethyl ether, di-n-butyl ether, di-*t*-butyl ether, tetrahydrofuran, 2-methyltetrahydrofuran, 4-methyltetrahydropyran, 1,4-dioxane, and diethyleneglycol dimethyl ether; hydrocarbon solvents such as hexane, heptane, benzene, toluene, and xylene; ketone solvents such as acetone, methyl ethyl ketone, isobutyl methyl ketone, and cyclohexanone; alcoholic solvents such as methyl alcohol, ethyl alcohol, n-propyl alcohol, isopropyl alcohol, isobutyl alcohol, ethylene glycol, propylene glycol, and benzyl alcohol; nitrile solvents such as acetonitrile; and aprotic polar solvents such as *N,N*-dimethylformamide, *N,N*-dimethylacetamide, *N*-methyl-2-pyrrolidone, 1,3-dimethyl-2-imidazolidinone, dimethyl sulfoxide, and hexamethylphosphoric triamide. Water, ether solvents, and alcoholic solvents are preferred. By using said water, ether solvents, and alcoholic solvents, a preferable reactivity may be ensured.

The solvent may be used alone or in combination thereof, if necessary, and may be optionally determined while considering the reactivity and/or the yield of the acetate compound (6). The solvent used may be one after purification of a commercial one or a commercial one as-is.

The amount of the solvent used may be optionally determined while considering the reactivity and/or the solubility of the acetate compound (6). The amount of the solvent is, for example, preferably 30 g to 10,000 g, more preferably 50 g to 5,000 g, and most preferably 100 g to 1,000 g, per mol of the acetate compound (6). By using said more preferred amount and said most preferred amount, a more preferred reactivity and/or economy and a most preferred reactivity and/or economy may be ensured.

The reaction temperature of the hydrolysis reaction may be optionally determined while considering the reactivity and/or by-production of an impurity of the acetate compound (6). The reaction temperature is, for example, preferably -30°C to 250°C, more preferably 0°C to 150°C, and most preferably 25°C to 100°C. By using said more preferred reaction temperature and said most preferred reaction temperature, a more preferred reactivity and/or by-production of an impurity and a most preferred reactivity and/or by-production of an impurity may be ensured.

The reaction time of the hydrolysis reaction is preferably optimized, depending on the reactivity of the acetate compound (6) by monitoring the reaction progress with, for example, gas chromatography and/or thin layer chromatography and/or nuclear magnetic resonance spectrum to confirm the disappearance of the substrate, acetate compound (6). The reaction time is typically, for example, preferably 1 hour to 72 hours, more preferably 1 hour to 24 hours, and most preferably 1 hour to 12 hours. By using said more preferred reaction time and said most preferred reaction time, a more preferred yield and/or by-production of an impurity and a most preferred yield and/or by-production of an impurity may be ensured.

The alcohol compound (7) obtained by the hydrolysis reaction may be suitably isolated and/or purified in any purification method used in usual organic synthesis, such as distillation at a reduced pressure and/or various chromatography. Distillation at a reduced pressure is preferred. By using said distillation at a reduced pressure, preferable industrial economy may be ensured. When the target compound, alcohol compound (7), has a sufficient purity, the crude product comprising the alcohol compound (7) may be used as such, without purification.

Thus, the organohalogen compound (5) can be industrially prepared with good yield and with fewer steps without using toxic or ignitable starting materials and in an industrially feasible reaction temperature range by subjecting the (ω-halo-2-alkenyl)triphenylphosphonium halide compound (1) to a phosphorus ylide preparation reaction with alkali metal alkoxide (3) in the presence of lithium halide (2) to obtain a reaction product mixture, and then subjecting the reaction product mixture to a Wittig reaction with the aldehyde compound (4). The acetate compound (6) can be prepared from the organohalogen compound (5), and the alcohol compound (7) can be prepared from the acetate compound (6) with good yield and fewer steps.

## EXAMPLES

The present invention will be described with reference to the following Examples and Comparative Examples. It should be construed that the present invention is not limited to or by the Examples.

The term "purity" as used herein means an area percentage obtained by gas chromatography (hereinafter referred to also as "GC"), unless otherwise specified. The term "product ratio" means a ratio of area percentages in GC.

The term "yield" is calculated from the area percentages determined by GC.

The yield was calculated by the following equation in consideration of purities (% GC) of a starting material and a product.

Yield (%) = {[(weight of a product obtained by a reaction × % GC)/molecular weight of a product] = [(weight of a starting material in a reaction × % GC)/molecular weight of a starting material]} × 100

GC conditions were as follows.

GC conditions for determination of "purity" and "product ratio": GC: capillary gas chromatograph GC-2010 plus (Shimadzu Corporation); column: DB-WAX, 0.25 μm×0.25 mmϕ×30 m, carrier gas: He (1.55 mL/min); detector: FID; column temperature: 150°C, kept for 3 minutes, elevated at a rate of 5°C/min, up to 230°C.

## Example 1

The following Examples 1-1 to 1-3 describe the preparation of a (ω-halo-2-alkenyl)triphenylphosphonium halide compound of the following general formula (1) according to the following reaction formula.

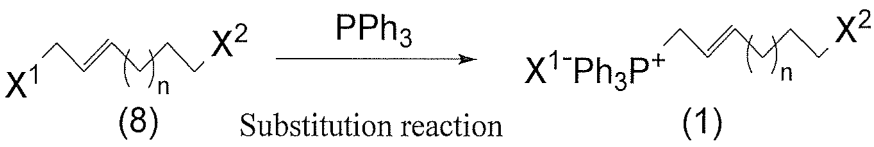

(8) (1)

Example 1-1: Preparation of (2*E*)- (6-chloro-2-hexenyl)triphenylphosphonium bromide (1: $X^1$ = Br, $X^2$ = Cl, *n* = 1)

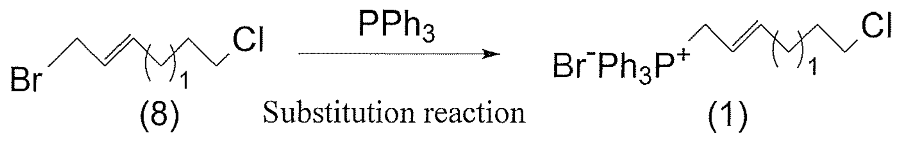

(8) (1)

Air in a reactor equipped with a stirrer, a condenser, and a thermometer was purged with nitrogen. Triphenylphosphine ($PPh_3$) (275.40 g: 1.05 mol) and acetonitrile ($CH_3CN$) (850.0 g) were placed in the reactor, and the liquid temperature was adjusted to 25°C to 28°C. (2*E*)-6-Chloro-2-hexenyl bromide (8: $X^1$ = Br, $X^2$ = Cl, *n* = 1) (197.50 g: 1.00 mol, purity 96.7%) was added dropwise to the mixture over 2 hours at a liquid temperature of 28°C to 30°C. After the completion of the dropwise addition, the reaction mixture was stirred for 7 hours at a liquid temperature of 30°C to 35°C. After the completion of the stirring, the solvent was removed from the reaction mixture at a reduced pressure, then toluene (700.0 g) followed by n-hexane (700.0 g) were added, and crystals were precipitated. The suspension was stirred for 1 hour at a liquid temperature of 20°C to 25°C, after which the crystals were filtered off and washed with n-hexane (30.0 g).

Subsequently, the crystals were dried at a reduced pressure to obtain (2E)- (6-chloro-2-hexenyl)triphenylphosphonium bromide (1: $X^1$ = Br, $X^2$ = Cl, *n* = 1) (460.25 g: 0.98 mol, yield 98.3%, purity 98.2%). The yield and the purity were determined by $^1$H-NMR using 1,4-bis(trimethylsilyl)benzene-*d*4 as an internal standard substance.

The following are the various spectrum data of (2*E*)-(6-chloro-2-hexenyl)triphenylphosphonium bromide (1: $X^1$ = Br, $X^2$ = Cl, *n* = 1) thus prepared.

Nuclear magnetic resonance spectrum: $^1$H-NMR (500 MHz, $CDCl_3$): δ 1.63 (2H, quin, J = 6.8 Hz), 2.19 (2H, dt, J = 12.5, 6.1 Hz), 3.24 (2H, t, J = 6.5 Hz), 4.69 (2H.q, J = 7.4 Hz), 5.31 (1H, dt, J = 21.6, 6.2 Hz), 5.96 (1H, dt, J = 21.7, 6.3 Hz), 7.63-7.67 (6H, m), 7.73-7.81 (9H, m) ppm.

$^{13}$C-NMR(126 MHz, $CDCl_3$): δ 27.48, 27.87, 29.60, 29.62, 30.83, 30.86, 43.95, 115.34, 115.42, 117.54, 118.23, 130.18, 130.29, 133.75, 133.83, 134.92, 134.94, 140.70, 140.80 ppm.

Mass spectrum: ESI (Positive): m/z 382.2, 380.2, 379.1 ($M^+$).

Infrared absorption spectrum: (ATR): ν ($cm^{-1}$) 493, 500, 545, 692, 718, 749, 843, 994, 1114, 1161, 1268, 1403, 1435, 1485, 1587, 2782, 2853, 2879, 2960, 2989, 3006, 305.

Example 1-2: Preparation of (2*E*)- (9-chloro-2-nonenyl)triphenylphosphonium bromide (1: $X^1$ = Br, $X^2$ = Cl, *n* = 4)

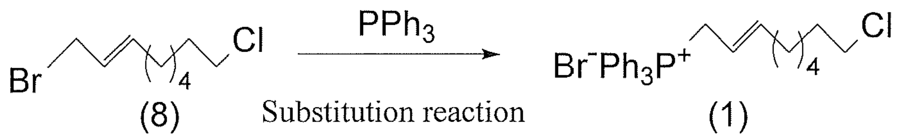

(8) (1)

The procedures of Example 1-1 were repeated, with the proviso that (2*E*)-9-chloro-2-nonenyl bromide (8: $X^1$ = Br, $X^2$ = Cl, *n* = 4) (239.58 g: 1.00 mol, purity 95.1%) was used instead of (2*E*)-6-chloro-2-hexenyl bromide (8: $X^1$ = Br, $X^2$ = Cl, *n* = 1) as the starting material. (2*E*)-(9-chloro-2-nonenyl)triphenylphosphonium bromide (1: $X^1$ = Br, $X^2$ = Cl, *n* = 4) (497.84 g: 0.99 mol, yield 99.0%, purity 99.8%) was obtained as a result. The yield and the purity were determined by $^1$H-NMR using 1,4-bis(trimethylsilyl)benzene-*d*4 as an internal standard substance.

The following are the various spectrum data of (2*E*)-(9-chloro-2-nonenyl)triphenylphosphonium bromide (1: $X^1$ = Br, $X^2$ = Cl, *n* = 4) thus prepared.

Nuclear magnetic resonance spectrum: $^1$H-NMR (500 MHz, $CD_3CN$): δ 1.08 (2H, quin, J = 7.6 Hz), 1.19 (2H, quin, J = 7.5 Hz), 1.29 (2H, quin, J = 7.6 Hz), 1.64 (2H.q, J = 7.1 Hz), 1.96 (2H, q, J = 6.1 Hz), 3.52 (2H, t, J = 6.7 Hz), 4.26 (2H, q, J = 7.4 Hz), 5.31-5.26 (1H, m), 5.76 (1H, dt, J = 21.0, 6.9 Hz), 7.68-7.76 (12H, m), 7.83-7.87 (3H, m) ppm.

$^{13}$C-NMR (126 MHz, $CD_3CN$): δ 27.11, 27.61, 28.01, 28.69, 29.09,29.11, 32.96, 32.99, 33.13, 46.17, 115.40, 118.79, 119.48, 130.98, 131.09, 134.94, 135.97, 136.00, 143.01, 143.11 ppm.

Mass spectrum: ESI (Positive): m/z 424.2, 422.2, 421.2 ($M^+$).

Infrared absorption spectrum: (ATR): ν ($cm^{-1}$) 502, 512, 538, 645, 692, 722, 742, 754, 850, 980, 995, 1112, 1163, 1184, 1437, 1485, 1587, 2777, 2866, 2932, 2986, 3003, 3041.

Example 1-3: Preparation of (2*E*)-(13-chloro-2-tridecenyl)triphenylphosphonium bromide (1: $X^1$ = Br, $X^2$ = Cl, *n* = 8)

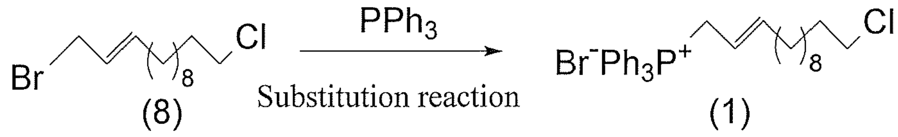


The procedures of Example 1-1 were repeated, with the proviso that (2E)-13-chloro-2-tridecenyl bromide (8: $X^1$ = Br, $X^2$ = Cl, *n* = 8) (295.69 g: 1.00 mol, purity 91.9%) was used instead of (2*E*)-6-chloro-2-hexenyl bromide (8: $X^1$ = Br, $X^2$ = Cl, *n* = 1) as the starting material. (2*E*)- (13-chloro-2-tridecenyl)triphenylphosphonium bromide (1: $X^1$ = Br, $X^2$ = Cl, *n* = 8) (497.84 g: 0.99 mol, yield 98.8%, purity 96.6%) was obtained as a result. The yield and the purity were determined by $^1$H-NMR using 1,4-bis(trimethylsilyl)benzene-d4 as an internal standard substance.

The following are the various spectrum data of (2E)- (13-chloro-2-tridecenyl)triphenylphosphonium bromide (1: $X^1$ = Br, $X^2$ = Cl, *n* = 8) thus prepared.

Nuclear magnetic resonance spectrum: $^1$H-NMR (500 MHz, $CD_3CN$): δ 1.02-1.08 (2H, m), 1.14-1.30 (10H, m), 1.38 (2H, quin, J = 7.4 Hz), 1.72 (2H.quin, J = 7.2 Hz), 1.96 (2H, q, J = 6.3 Hz), 3.56 (2H, t, J = 6.7 Hz), 4.22 (2H, q, J = 7.5 Hz), 5.33 (1H, dt, J = 21.3, 6.5 Hz), 5.76 (1H, dt, J = 20.0, 5.6 Hz), 7.67-7.76 (12H, m), 7.83-7.87 (3H, m) ppm.

$^{13}$C-NMR (126 MHz, $CD_3CN$): δ 27.44, 27.59, 27.99, 29.27, 29.30, 29.45, 29.49, 29.92, 30.05, 33.08, 33.10, 33.28, 46.25, 115.25, 115.33, 118.78, 119.47, 134.86, 134.93, 135.97, 136.00, 143.18, 143.29 ppm.

Mass spectrum: ESI (Positive): m/z 480.2, 478.2, 477.2 ($M^+$).

Infrared absorption spectrum: (ATR): ν ($cm^{-1}$) 490, 506, 544, 692, 723, 739, 753, 844, 996, 1113, 1161, 1436, 1485, 1588, 2778, 2852, 2923, 2987, 3055.

Example 2

The following Examples 2-1 to 2-17 describe preparation of an organohalogen compound of the following general formula (5) according to the following reaction formula.

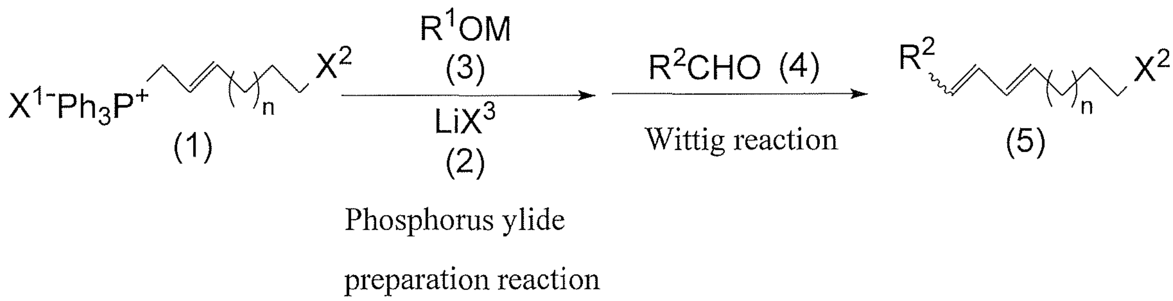


Example 2-1: Preparation of (4E,10Z)-4,6,10-hexadecatrienyl chloride (5: $X^2$ = Cl, *n* = 1, $R^2$ = (3Z)-3-nonenyl group)

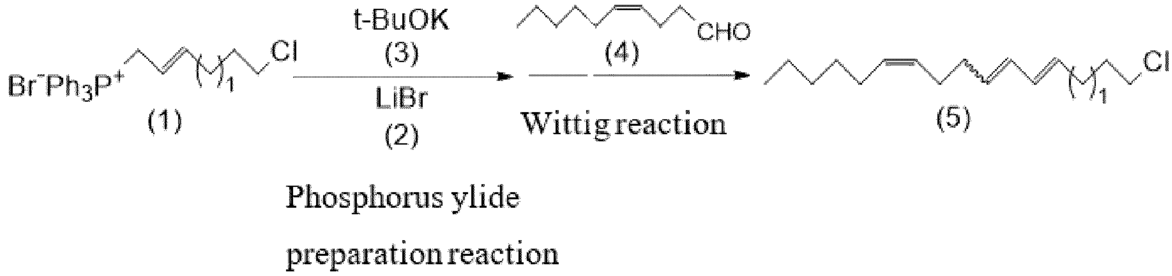


Air in a reactor equipped with a stirrer, a condenser, and a thermometer was purged with nitrogen. (2*E*)- (6-chloro-2-hexenyl)triphenylphosphonium bromide (1: $X^1$ = Br, $X^2$ = Cl, *n* = 1) (505.77 g: 1.10 mol, purity 98.2%) obtained as in Example 1-1, lithium bromide (LiBr) (2: $X^3$ = Br) (104.21 g, 1.20 mol), and tetrahydrofuran (THF) (1,800.0 g) were added

to the reactor, and the liquid temperature was cooled to -10°C to -5°C. Potassium *t*-butoxide (*t*-BuOK) (3: $R^1$ = *t*-Bu group, M = potassium) (122.31 g: 1.09 mol) was added to the mixture over 10 minutes at a liquid temperature of -5°C to 0°C, and then allowed to react for 1.5 hours at a liquid temperature of -5°C to 0°C.

After the completion of the reaction, the reaction liquid temperature was stabilized at -5°C, and (4Z)-4-decenal (4: $R^2$ = (3Z)-3-nonenyl group) (154.25 g, 1.00 mol, purity 97.5%) was added over 2 hours at a liquid temperature of -5°C to 0°C.

The reaction mixture was stirred for 1 hour at a liquid temperature of 0°C to 10°C. After the completion of the stirring, the reaction was quenched by adding water (600.0 g) to the reactor, and was separated into an organic layer and an aqueous layer. The solvent was removed from the resulting organic layer at a reduced pressure, followed by adding n-hexane (1,500.0 g), filtering, and separating into the filtrate and precipitated triphenylphosphine oxide. Filtrate was washed sequentially with an aqueous solution of 5% by weight sodium bicarbonate (800.0 g) and then an aqueous solution of 15% by weight sodium chloride (800.0 g), followed by removing the solvent from the organic layer at a reduced pressure.

The crude product thus obtained was purified by distillation at a reduced pressure to obtain (4*E*,10*Z*)-4,6,10-hexadecatrienyl chloride (5: $X^2$ = Cl, *n* = 1, $R^2$ = (3Z)-3-nonenyl group) (224.26 g: 0.88 mol, yield 87.9%, purity 93.1%).

It was verified from the gas chromatography analysis that (4*E*,10*Z*)-4,6,10-hexadecatrienyl chloride (5: $X^2$ = Cl, *n* = 1, $R^2$ = (3*Z*)-3-nonenyl group) thus prepared had two types of *cis-trans* isomers [isomer formation ratio = 39.9 (GC Retention time 14.78 min): 60.1 (GC Retention time 15.61 min)] at Retention times14.78 minutes and 15.61 minutes, respectively. The above yields of (4E,10Z)-4,6,10-hexadecatrienyl chloride (5: $X^2$ = Cl, *n* = 1, $R^2$ = (3Z)-3-nonenyl group) are represented as a mixture of the two types of *cis-trans* isomers.

The configurations of the two types of *cis-trans* isomers of (4*E*,10*Z*)-4,6,10-hexadecatrienyl chloride (5: $X^2$ = Cl, *n* = 1, $R^2$ = (3Z)-3-nonenyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 14.78 minutes was considered to be (4*E*,6*Z*,10*Z*)-4,6,10-hexadecatrienyl chloride, and the *cis-trans* isomer detected at GC Retention time 15.61 minutes was considered to be (4*E*,6*E*,10*Z*)-4,6,10-hexadecatrienyl chloride.

The following are the various spectrum data of (4E,102)-4,6,10-hexadecatrienyl chloride (5: $X^2$ = Cl, *n* = 1, $R^2$ = (3Z)-3-nonenyl group) thus prepared.

Nuclear magnetic resonance spectrum: $^1$H-NMR (600 MHz, $CDCl_3$): $\delta$ 0.89 (3H, t, J = 7.2 Hz), 1.24-1.37 (6H, m), 1.83-1.90 (2H, m), 2.02 (2H.quin, J = 7.1 Hz), 2.11-2.14 (3H, m), 2.22 (2H, q, J = 7.4 Hz), 2.26 (1H, q, J = 7.2 Hz), 3.52-3.56 (2H, m), 5.33-5.42 (2.4H, m), 5.52 (0.6H, dt, J = 18.4, 7.2 Hz), 5.59-5.64 (1H, m), 5.96 (0.4H, t, J = 11.1 Hz), 5.99-6.07 (1.2H, m), 6.36 (0.4H, dd, J = 15.0 Hz) ppm.

$^{13}$C-NMR (126 MHz, $CDCl_3$): $\delta$ 14.22, 22.72, 27.20, 27.38, 27.43, 28.02, 29.51, 29.53, 29.76, 30.04, 31.66, 32.25, 32.29, 32.86, 44.52, 127.13, 128.69, 128.91, 130.14, 130.33, 130.38, 130.72, 130.84, 131.84, 132.38, 132.78 ppm.

Spin coupling constant between double bonded hydrogen nuclei determined from $^1$H-NMR and two-dimensional NMR$^{13}$C non-irradiated HSQC slice data: (4*E*,6*Z*,10*Z*)-form: $J_{4,5}$ = 15.2 Hz (positions 4-5), $J_{6,7}$ = 10.8 Hz (positions 6-7), $J_{10,11}$ = 11.1 Hz (positions 10-11); (4*E*,6*E*,10*Z*)-form: $J_{4,5}$ = 14.9 Hz (positions 4-5), $J_{6,7}$ = 14.9 Hz (positions 6-7), $J_{10,11}$ = 11.1 Hz (positions 10-11).

Mass spectrum: EI (70 eV) (GC Retention time = 14.78 min) (4*E*,6*Z*,10*Z*)-form: m/z 254 ($M^+$), 197, 183, 163, 143, 107, 93, 79, 67, 55, 41, 21; (GC Retention time = 15.61 min) (4*E*,6*E*,10*Z*)-form: m/z 254 ($M^+$), 197, 183, 163, 143, 107, 93, 79, 67, 55, 41, 27.

Infrared absorption spectrum: (ATR): ν ($cm^{-1}$) 656, 727, 947, 987, 1308, 1442, 2855, 2927, 2956, 3007.

Example 2-2: Preparation of (4*E*,10*Z*)-4,6,10-hexadecatrienyl chloride (5: $X^2$ = Cl, *n* = 1, $R^2$ = (3*Z*)-3-nonenyl group)

The procedures of Example 2-1 were repeated, with the proviso that the amount of lithium bromide (LiBr) (2: $X^3$ = Br) used as lithium halide (2) in Example 2-1 was (138.94 g, 1.60 mol). (4*E*,10*Z*)-4,6,10-Hexadecatrienyl chloride (5: $X^2$ = Cl, *n* = 1, $R^2$ = (3Z)-3-nonenyl group) (214.83 g: 0.84 mol, yield 84.3%, purity 94.0%) was obtained as a result.

It was verified from the gas chromatography analysis that (4*E*,10*Z*)-4,6,10-hexadecatrienyl chloride (5: $X^2$ = Cl, *n* = 1, $R^2$ = (3Z)-3-nonenyl group) thus prepared had two types of *cis-trans* isomers [isomer formation ratio = 39.9 (GC Retention time 14.78 min): 60.1 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3*Z*)-3-nonenyl group) are represented as a mixture of the two types of *cis-trans* isomers.

The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-3: Preparation of (4*E*,10*Z*)-4,6,10-hexadecatrienyl chloride (5: $X^2$ = Cl, *n* = 1, $R^2$ = (3*Z*)-3-nonenyl group)

The procedures of Example 2-1 were repeated, with the proviso that the amount of lithium bromide (LiBr) (2: $X^3$ = Br) used as lithium halide (2) in Example 2-1 was (52.10 g, 0.6 mol). (4*E*,10*Z*)-4,6,10-Hexadecatrienyl chloride (5: $X^2$ = Cl, *n* = 1, $R^2$ = (3*Z*)-3-nonenyl group) (193.68 g: 0.76 mol, yield 76.1%, purity 94.9%) was obtained as a result.
It was verified from the gas chromatography analysis that (4*E*,10*Z*)-4,6,10-hexadecatrienyl chloride (5: $X^2$ = Cl, *n* = 1, $R^2$ = (3Z)-3-nonyl group) thus prepared had two types of *cis-trans* isomers [isomer formation ratio = 46.0 (GC Retention time 14.78 min): 54.0 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3Z)-3-nonenyl group) are represented as a mixture of the two types of *cis-trans* isomers.
The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-4: Preparation of (4*E*,10*Z*)-4,6,10-hexadecatrienyl chloride (5: $X^2$ = Cl, *n* = 1, $R^2$ = (3*Z*)-3-nonenyl group)

The procedures of Example 2-1 were repeated, with the proviso that (4*Z*)-4-decenal (4: $R^2$ = (3*Z*)-3-nonenyl group) was added as the aldehyde compound (4) of Example 2-1 at a liquid temperature of -25°C to -20°C instead of the liquid temperature of -5°C to 0°C. (4*E*,10*Z*)-4,6,10-Hexadecatrienyl chloride (5: $X^2$ = Cl, *n* = 1, $R^2$ = (3*Z*)-3-nonenyl group) (163.10 g: 0.64 mol, yield 64.0%, purity 92.5%) was obtained as a result.
It was verified from the gas chromatography analysis that (4*E*,10*Z*)-4,6,10-hexadecatrienyl chloride (5: $X^2$ = Cl, *n* = 1, $R^2$ = (3Z)-3-nonenyl group) thus prepared had two types of *cis-trans* isomers [isomer formation ratio = 39.0 (GC Retention time 14.78 min): 61.0 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3Z)-3-nonenyl group) are represented as a mixture of the two types of *cis-trans* isomers.
The various spectrum data of (4E,10Z)-4,6,10-hexadecatrienyl chloride (5: $X^2$ = Cl, *n* = 1, $R^2$ = (3Z)-3-nonenyl group) were the same as those of Example 2-1.

Example 2-5: Preparation of (4E,10Z)-4,6,10-hexadecatrienyl chloride (5: $X^2$ = Cl, *n* = 1, $R^2$ = (3Z)-3-nonenyl group)

The procedures of Example 2-1 were repeated, with the proviso that (4*Z*)-4-decenal (4: $R^2$ = (3Z)-3-nonenyl group) was added as the aldehyde compound (4) of Example 2-1 at a liquid temperature of 30°C to 35°C instead of the liquid temperature of -5°C to 0°C. (4E,10Z)-4,6,10-Hexadecatrienyl chloride (5: $X^2$ = Cl, *n* = 1, $R^2$ = (3Z)-3-nonenyl group) (226.81 g: 0.89 mol, yield 88.6%, purity 87.3%) was obtained as a result.
It was verified from the gas chromatography analysis that (4*E*,10*Z*)-4,6,10-hexadecatrienyl chloride (5: $X^2$ = Cl, *n* = 1, $R^2$ = (3*Z*)-3-nonenyl group) thus prepared had two types of *cis-trans* isomers [isomer formation ratio = 43.8 (GC Retention time 14.78 min): 56.2 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3*Z*)-3-nonenyl group) are represented as a mixture of the two types of *cis-trans* isomers.
The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-6: Preparation of (4*E*,10*Z*)-4,6,10-hexadecatrienyl chloride (5: $X^2$ = Cl, *n* = 1, $R^2$ = (3*Z*)-3-nonenyl group)

The procedures of Example 2-1 were repeated, with the proviso that acetonitrile ($CH_3CN$) (1,800.0 g) was used instead of tetrahydrofuran (THF) as the solvent used in Example 2-1. (4*E*,10*Z*)-4,6,10-Hexadecatrienyl chloride (5: $X^2$ = Cl, *n* = 1, $R^2$ = (3*Z*)-3-nonenyl group) (145.26 g: 0.57 mol, yield 56.8%, purity 92.8%) was obtained as a result.
It was verified from the gas chromatography analysis that (4*E*,10*Z*)-4,6,10-hexadecatrienyl chloride (5: $X^2$ = Cl, *n* = 1, $R^2$ = (3*Z*)-3-nonenyl group) thus prepared had two types of *cis-trans* isomers [isomer formation ratio = 52.6 (GC Retention time 14.78 min): 47.4 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3*Z*)-3-nonenyl group) are represented as a mixture of the two types of *cis-trans* isomers.
The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-7: Preparation of (4*E*,10*Z*)-4,6,10-hexadecatrienyl chloride (5: $X^2$ = Cl, *n* = 1, $R^2$ = (3*Z*)-3-nonenyl group)

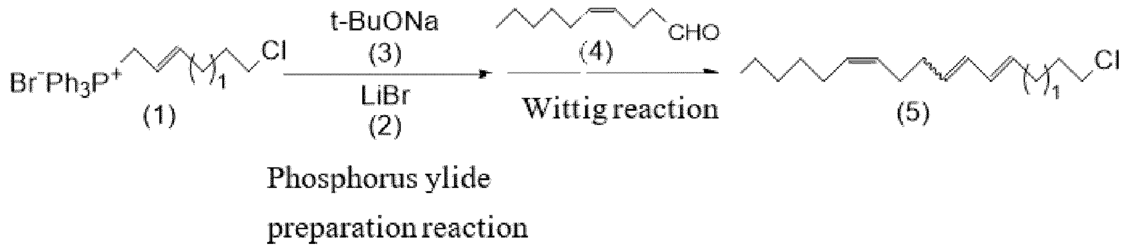


The procedures of Example 2-1 were repeated, with the proviso that sodium t-butoxide (*t*-BuONa) (3: $R^1$ = *t*-Bu group, M = sodium) (104.75 g: 1.09 mol) was used instead of potassium *t*-butoxide (*t*-BuOK) (3: $R^1$ = *t*-Bu group, M = potassium) as alkali metal alkoxide (3) used in Example 2-1. (4*E*,10*Z*)-4,6,10-Hexadecatrienyl chloride (5: $X^2$ = Cl, *n* = 1, $R^2$ = (3Z)-3-nonenyl group) (211.51 g: 0.83 mol, yield 83.1%, purity 96.1%) was obtained as a result.

It was verified from the gas chromatography analysis that (4*E*,10*Z*)-4,6,10-hexadecatrienyl chloride (5: $X^2$ = Cl, *n* = 1, $R^2$ = (3Z)-3-nonenyl group) thus prepared had two types of *cis-trans* isomers [isomer formation ratio = 40.7 (GC Retention time 14.78 min): 59.3 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3Z)-3-nonenyl group) are represented as a mixture of the two types of *cis-trans* isomers.

The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-8: Preparation of (4*E*,10*Z*)-4,6,10-hexadecatrienyl chloride (5: $X^2$ = Cl, *n* = 1, $R^2$ = (3Z)-3-nonenyl group)

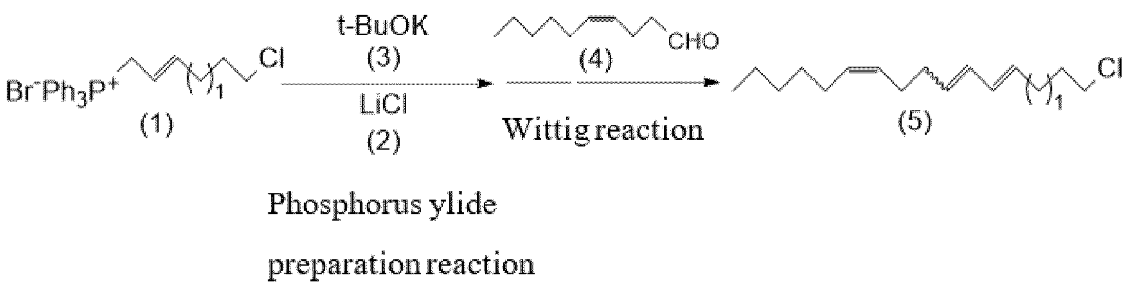


The procedures of Example 2-1 were repeated, with the proviso that lithium chloride (LiCl) (2: $X^3$ = Cl) (50.87 g, 1.20 mol) was used instead of lithium bromide (LiBr) (2: $X^3$ = Br) (104.21 g, 1.20 mol) as lithium halide (2) used in Example 2-1. (4E,10Z)-4,6,10-Hexadecatrienyl chloride (5: $X^2$ = Cl, *n* = 1, $R^2$ = (3Z)-3-nonenyl group) (187.97 g: 0.74 mol, yield 73.6%, purity 94.6%) was obtained as a result.

It was verified from the gas chromatography analysis that (4*E*,10*Z*)-4,6,10-hexadecatrienyl chloride (5: $X^2$ = Cl, *n* = 1, $R^2$ = (3Z)-3-nonenyl group) thus prepared had two types of *cis-trans* isomers [isomer formation ratio = 38.7 (GC Retention time 14.78 min): 61.3 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3Z)-3-nonenyl group) are represented as a mixture of the two types of *cis-trans* isomers.

The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-9: Preparation of (4*E*,10*Z*)-4,6,10-hexadecatrienyl chloride (5: $X^2$ = Cl, *n* = 1, $R^2$ = (3*Z*)-3-nonenyl group)

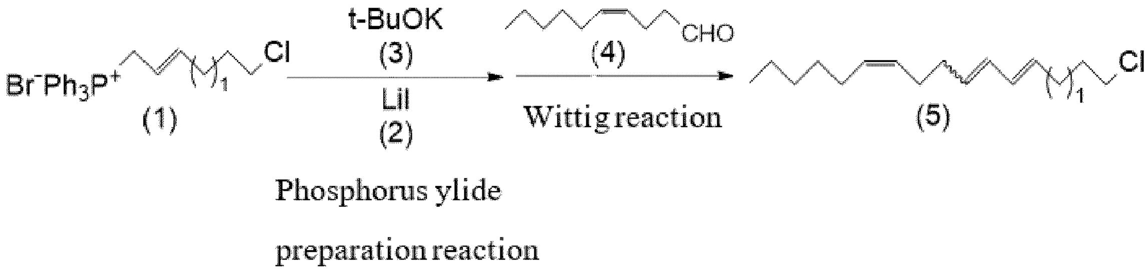


The procedures of Example 2-1 were repeated, with the proviso that lithium iodide (LiI) (2: $X^3$ = I) (160.62 g, 1.20 mol) was used instead of lithium bromide (LiBr) (2: $X^3$ = Br) (104.21 g, 1.20 mol) as lithium halide (2) used in Example 2-1. (4E,10Z)-4,6,10-Hexadecatrienyl chloride (5: $X^2$ = Cl, *n* = 1, $R^2$ = (3Z)-3-nonenyl group) (187.97 g: 0.71 mol, yield 71.3%, purity 95.2%) was obtained as a result.

It was verified from the gas chromatography analysis that (4*E*,10*Z*)-4,6,10-hexadecatrienyl chloride (5: $X^2$ = Cl, *n* = 1, $R^2$ = (3Z)-3-nonenyl group) thus prepared had two types of *cis-trans* isomers [isomer formation ratio = 46.1 (GC Retention time 14.78 min): 53.9 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3*Z*)-3-nonenyl group) are represented as a mixture of the two types of *cis-trans* isomers.

The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-10: Preparation of (4*E*,10*Z*)-4,6,10-hexadecatrienyl chloride (5: $X^2$ = Cl, *n* = 1, $R^2$ = (3*Z*)-3-nonenyl group)

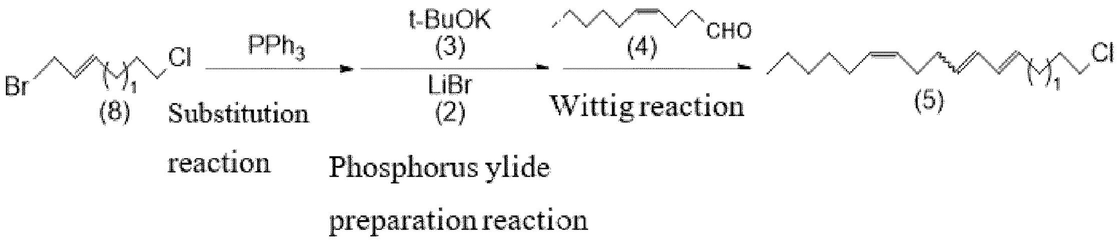


The procedures of Example 1-1 were repeated, with the proviso that tetrahydrofuran (THF) (500.0 g) was used instead of the acetonitrile ($CH_3CN$) as the solvent used in Example 1-1. The reaction mixture of (2*E*)-(6-chloro-2-hexenyl) triphenylphosphonium bromide (1: $X^1$ = Br, $X^2$ = Cl, *n* = 1) thus obtained was used as such, without isolation or purification, to prepare the organohalogen compound (5) of Example 2.

The procedures of Example 2-1 were repeated, with the proviso that the amount of tetrahydrofuran (THF) used as the solvent used in Example 2-1 was (600.0 g), and lithium bromide (LiBr) (2: $X^3$ = Br) and tetrahydrofuran (THF) were directly added respectively as lithium halide and a solvent to the reaction mixture of (2*E*)- (6-chloro-2-hexenyl)triphenylphosphonium bromide (1: $X^1$ = Br, $X^2$ = Cl, *n* = 1). (4*E*,10*Z*)-4,6,10-Hexadecatrienyl chloride (5: $X^2$ = Cl, *n* = 1, $R^2$ = (3Z)-3-nonenyl group) (221.71 g: 0.87 mol, yield 87.0%, purity 95.6%) was obtained as a result.

It was verified from the gas chromatography analysis that (4*E*,10*Z*)-4,6,10-hexadecatrienyl chloride (5: $X^2$ = Cl, *n* = 1, $R^2$ = (3Z)-3-nonenyl group) thus prepared had two types of *cis-trans* isomers [isomer formation ratio = 40.9 (GC Retention time 14.78 min): 59.1 (GC Retention time 15.61 min)] for (4*E*,6*Z*,10*Z*)-4,6,10-hexadecatrienyl chloride at Retention time 14.78 minutes and (4*E*,6*E*,10*Z*)-4,6,10-hexadecatrienyl chloride at Retention time 15.61 minutes. The above yields of (4*E*,10*Z*)-4,6,10-hexadecatrienyl chloride (5: $X^2$ = Cl, *n* = 1, $R^2$ = (3*Z*)-3-nonenyl group) are represented as a mixture of the two types of *cis-trans* isomers.

The various spectrum data of (4*E*,10*Z*)-4,6,10-hexadecatrienyl chloride (5: $X^2$ = Cl, *n* = 1, $R^2$ = (3*Z*)-3-nonenyl group) were the same as those of Example 2-1.

Example 2-11: Preparation of (4*E*)-4,6-hexadecadienyl chloride (5: $X^2$ = Cl, *n* = 1, $R^2$ = n-nonenyl group)

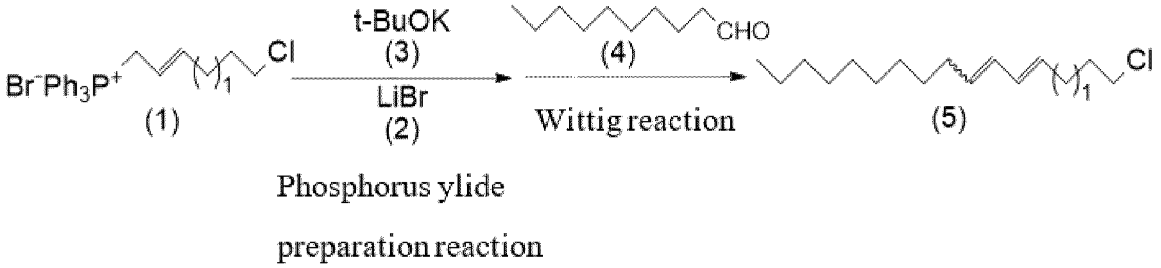


The procedures of Example 2-1 were repeated, with the proviso that n-decanal (4: $R^2$ = n-nonyl group) (154.25 g, 1.00 mol, purity 97.5%) was used instead of (4*Z*)-4-decenal (4: $R^2$ = (3*Z*)-3-nonenyl group) as the aldehyde compound (4) used in Example 2-1. (4*E*)-4,6-Hexadecadienyl chloride (5: $X^2$ = Cl, *n* = 1, $R^2$ = n-nonyl group) (228.61 g: 0.89 mol, yield 88.8%, purity 94.1%) was obtained as a result.

It was verified from the gas chromatography analysis that (4*E*)-4,6-hexadecadienyl chloride (5: $X^2$ = Cl, *n* = 1, $R^2$ = n-nonyl group) thus prepared had two types of *cis-trans* isomers [isomer formation ratio = 41.4 (GC Retention time 13.98 min): 58.6 (GC Retention time 14.89 min)] at Retention times13.98 minutes and 14.89 minutes, respectively. The above yields of (4*E*)-4,6-hexadecadienyl chloride (5: $X^2$ = Cl, *n* = 1, $R^2$ = n-nonyl group) are represented as a mixture of the two types of *cis-trans* isomers.

The configurations of the two types of *cis-trans* isomers of (4*E*)-4,6-hexadecadienyl chloride (5: $X^2$ = Cl, *n* = 1, $R^2$ = n-nonyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 13.98 minutes was considered to be (4*E*,6*Z*)-4,6-hexadecadienyl chloride, and the *cis-trans* isomer detected at GC Retention time 14.89 minutes was considered to be (4*E*,6*E*)-4,6-hexadecadienyl chloride.

The following are the various spectrum data of (4*E*)-4,6-hexadecadienyl chloride (5: $X^2$ = Cl, *n* = 1, $R^2$ = n-nonyl group) thus prepared.

Nuclear magnetic resonance spectrum: $^1$H-NMR (500 MHz, $CDCl_3$): δ 0.88 (3H, t, J = 7.1 Hz), 1.27-1.39 (14H, m), 1.83-1.91 (2H, m), 2.05 (2H.q, J = 7.1 Hz), 2.16 (0.8H, q, J = 7.3 Hz), 2.23 (1.2H, q, J = 7.9 Hz), 2.28 (0.8H, q, J = 6.7 Hz), 3.52-3.56 (2H, m), 5.35 (0.4H, dt, J = 11.0, 7.5 Hz), 5.51 (0.6H, dt, J = 14.6, 7.2 Hz), 5.60 (1H, dt, J = 14.5, 7.2 Hz), 5.95 (0.6H, dd, J = 15.7, 11.2 Hz), 5.99-6.08 (1H, m), 6.36 (0.4H, dd, J = 15.1, 11.0 Hz) ppm.

$^{13}$C-NMR (126 MHz, $CDCl_3$): δ 14.09, 22.67, 27.71, 29.21, 29.25, 29.31, 29.35, 29.51, 29.56, 29.58, 29.60, 29.68, 29.89, 31.89, 32.13, 32.16, 32.58, 127.03, 128.14, 129.61, 129.87, 131.05, 131.81, 131.88, 133.44 ppm.

Spin coupling constant between double bonded hydrogen nuclei determined from $^1$H-NMR and two-dimensional NMR$^{13}$C non-irradiated HSQC slice data: (4*E*,6*Z*)-form: $J_{4,5}$ = 15.1 Hz (positions 4-5), $J_{6,7}$ = 11.0 Hz (positions 6-7); (4*E*,6*E*)-form: $J_{4,5}$ = 15.1 Hz (positions 4-5), $J_{6,7}$ = 14.7 Hz (positions 6-7).

Mass spectrum: EI (70 eV) (GC Retention time = 13.98 min) (4*E*,6*Z*)-form: m/z 256 ($M^+$), 158, 144, 130, 109, 95, 81, 67, 54, 41, 27; (GC Retention time = 14.89 min) (4E,6E)-form: m/z 256 ($M^+$), 158, 144, 130, 109, 95, 81, 67, 54, 41, 27.

Infrared absorption spectrum: (ATR): ν ($cm^{-1}$) 656, 723, 948, 987, 1442, 1458, 2854, 2924, 2956, 3016.

Example 2-12: Preparation of (4*E*)-7-phenyl-4,6-heptadienyl chloride (5: $X^2$ = Cl, n = 1, $R^2$ = phenyl group)

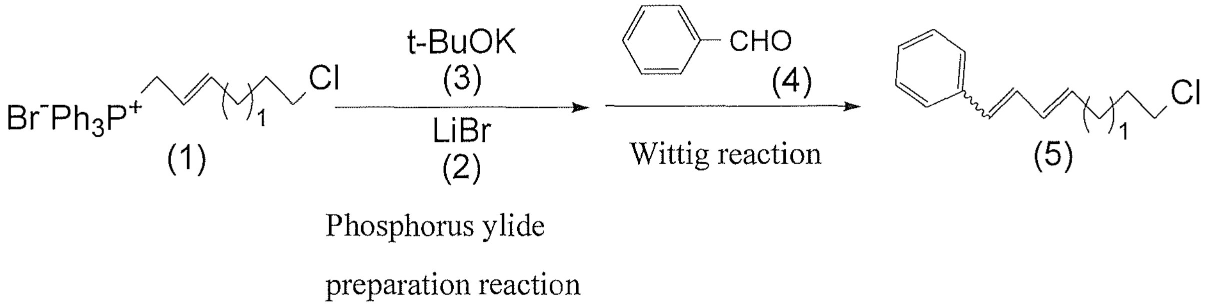


The procedures of Example 2-1 were repeated, with the proviso that benzaldehyde (4: $R^2$ = phenyl group) (106.12 g, 1.00 mol, purity 98.0%) was used instead of (4*Z*)-4-decenal (4: $R^2$ = (3*Z*)-3-nonenyl group) as the aldehyde compound (4) used in Example 2-1. (4*E*)-7-Phenyl-4,6-heptadienyl chloride (5: $X^2$ = Cl, *n* = 1, $R^2$ = phenyl group) (190.17 g: 0.92 mol, yield 91.8%, purity 96.2%) was obtained as a result.

It was verified from the gas chromatography analysis that (4*E*)-7-phenyl-4,6-heptadienyl chloride (5: $X^2$ = Cl, *n* = 1, $R^2$ = phenyl group) thus prepared had two types of *cis-trans* isomers [isomer formation ratio = 38.6 (GC Retention time 13.44 min): 61.4 (GC Retention time 16.80 min)] at Retention times13.44 minutes and 16.80 minutes, respectively. The above yields of (4*E*)-7-phenyl-4,6-heptadienyl chloride (5: $X^2$ = Cl, *n* = 1, $R^2$ = phenyl group) are represented as a mixture of the two types of *cis-trans* isomers.

The configurations of the two types of *cis-trans* isomers of (4*E*)-7-phenyl-4,6-heptadienyl chloride (5: $X^2$ = Cl, *n* = 1, $R^2$ = phenyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 13.44 minutes was considered to be (4*E*,6*Z*)-7-phenyl-4,6-heptadienyl chloride, and the *cis-trans* isomer detected at GC Retention time 16.80 minutes was considered to be (4*E*,6*E*)-7-phenyl-4,6-heptadienyl chloride.

The following are the various spectrum data of (4*E*)-7-phenyl-4,6-heptadienyl chloride (5: $X^2$ = Cl, *n* = 1, $R^2$ = phenyl group) thus prepared.

Nuclear magnetic resonance spectrum: $^1$H-NMR (600 MHz, $CDCl_3$): δ 1.91 (2H, sep, J = 7.1 Hz), 2.31 (2H, sext, J = 7.3 Hz), 3.56 (1.2H, t, 6.6 Hz), 3.57 (0.8H.t, J = 6.5 Hz), 5.75-5.86 (1H, m), 6.22 (0.6H, t, J = 11.4 Hz), 6.27 (0.4H, dd, J = 15.1, 8.7 Hz), 6.37 (0.4H, J = 11.5 Hz), 6.48 (0.6H, J = 15.6 Hz), 6.66 (0.4H, dd, J = 15.0, 11.0 Hz), 6.76 (0.6H, dd, J = 15.7, 8.7 Hz), 7.21-7.26 (1H, m), 7.30-7.39 (4H, m) ppm.

$^{13}$C-NMR (150 MHz, $CDCl_3$): δ 29.99, 30.08, 32.11, 32.16, 44.47, 126.33, 126.95, 127.41, 127.98, 128.38, 128.54, 128.71, 129.02, 129.06, 130.17, 130.94, 131.99, 133.30, 135.51, 137.57, 137.74 ppm.

Spin coupling constant between double bonded hydrogen nuclei determined from $^1$H-NMR and two-dimensional NMR$^{13}$C non-irradiated HSQC slice data: (4*E*,6*Z*)-form: $J_{4,5}$ = 14.4 Hz (positions 4-5), $J_{6,7}$ = 11.0 Hz (positions 6-7); (4*E*,6*E*)-form: $J_{4,5}$ = 15.1 Hz (positions 4-5), $J_{6,7}$ = 15.6 Hz (positions 6-7).

Mass spectrum: EI (70 eV) (GC Retention time = 13.44 min) (4*E*,6*Z*)-form: m/z 206 ($M^+$), 143, 129, 115, 91, 77, 65, 51, 39, 27; (GC Retention time = 16.80 min) (4*E*,6*E*)-form: m/z 208 ($M^+$), 143, 129, 115, 91, 77, 65, 51, 39, 27.

Infrared absorption spectrum: (ATR): ν (cm$^{-1}$) 508, 652, 692, 701, 746, 771, 916, 949, 989, 1028, 1072, 1295, 1446, 1492, 1596, 1643, 2841, 2936, 2955, 3022, 3057, 3078.

Example 2-13: Preparation of (7*E*)-7,9-tetradecadienyl chloride (5: $X^2$ = Cl, *n* = 4, $R^2$ = n-butyl group)

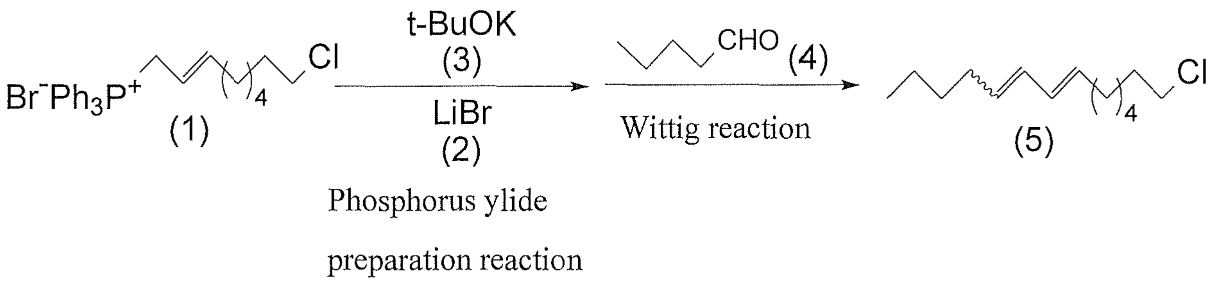


The procedures of Example 2-1 were repeated, with the proviso that (2*E*)- (9-chloro-2-nonenyl)triphenylphosphonium bromide (1: $X^1$ = Br, $X^2$ = Cl, *n* = 4) (552.06 g, 1.10 mol, purity 99.8%) obtained as in Example 1-2 was used instead of (2*E*)- (6-chloro-2-hexenyl)triphenylphosphonium bromide (1: $X^1$ = Br, $X^2$ = Cl, *n* = 1) as the (ω-halo-2-alkenyl) triphenylphosphonium halide compound (1) used in Example 2-1, and n-pentanal (4: $R^2$ = n-butyl group) (86.13 g, 1.00 mol, purity 95.0%) was used instead of (4*Z*)-4-decenal (4: $R^2$ = (3*Z*)-3-nonenyl group) as the aldehyde compound (4) used in Example 2-1. (7*E*)-7,9-Tetradecadienyl chloride (5: $X^2$ = Cl, *n* = 4, $R^2$ = n-butyl group) (205.92 g: 0.90 mol, yield 90.5%, purity 95.5%) was obtained as a result.

It was verified from the gas chromatography analysis that (7E)-7,9-tetradecadienyl chloride (5: $X^2$ = Cl, *n* = 4, $R^2$ = n-butyl group) thus prepared had two types of *cis-trans* isomers [isomer formation ratio = 41.9 (GC Retention time 10.43 min): 58.1 (GC Retention time 11.06 min)] at Retention times10.43 minutes and 11.06 minutes, respectively. The above yields of (7*E*)-7,9-tetradecadienyl chloride (5: $X^2$ = Cl, *n* = 4, $R^2$ = n-butyl group) are represented as a mixture of the two types of *cis-trans* isomers.

The configurations of the two types of *cis-trans* isomers of (7*E*)-7,9-tetradecadienyl chloride (5: $X^2$ = Cl, *n* = 4, $R^2$ = n-butyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 10.43 minutes was considered to be (7*E*,9*Z*)-7,9-tetradecadienyl chloride, and the *cis-trans* isomer detected at GC Retention time 11.06 minutes was considered to be (7*E*,9*E*)-7,9-tetradecadienyl chloride.

The following are the various spectrum data of (7*E*)-7,9-tetradecadienyl chloride (5: $X^2$ = Cl, *n* = 4, $R^2$ = n-butyl group) thus prepared.

Nuclear magnetic resonance spectrum: $^1$H-NMR (600 MHz, $CDCl_3$): δ 0.88-0.92 (3H, m), 1.28-1.46 (10H, m), 1.74-1.80 (2H, m), 2.06 (2.4H.q, J = 7.0 Hz), 2.10 (0.8H, q, J = 7.2 Hz), 2.16 (0.8H, q, J = 7.2 Hz), 3.53 (1.2H, t, J = 6.8 Hz), 3.53 (0.8H, t, J = 6.8 Hz), 5.31 (0.4H, dt, J = 10.7, 7.6 Hz), 5.51-5.60 (1.2H, m), 5.64 (0.4H, dt, J = 14.4, 7.3 Hz), 5.94 (0.4H, t, J = 11.0 Hz), 5.97-6.02 (1.2H, m), 6.30 (0.4H, dd, J = 15.1, 11.0 Hz) ppm.

$^{13}$C-NMR (150 MHz, $CDCl_3$): δ 14.08, 14.11, 22.39, 22.46, 26.87, 26.88, 27.54, 28.55, 29.33, 29.34, 31.71, 32.04, 32.41, 32.58, 32.72, 32.86, 45.27, 125.98, 128.64, 130.38, 130.40, 130.70, 132.11, 132.73, 134.37 ppm.

Spin coupling constant between double bonded hydrogen nuclei determined from $^1$H-NMR and two-dimensional NMR$^{13}$C non-irradiated HSQC slice data: (7*E*,9*Z*)-form: $J_{7,8}$ = 14.4 Hz (positions 7-8), $J_{9,10}$ = 10.8 Hz (positions 9-10); (7*E*,9*E*)-form: $J_{7,8}$ = 15.2 Hz (positions 7-8), $J_{9,10}$ = 15.5 Hz (positions 9-10).

Mass spectrum: EI (70 eV) (GC Retention time = 10.43 min) (7*E*,9*Z*)-form: m/z 228 ($M^+$), 185, 171, 157, 144, 123, 109, 95, 81, 67, 54, 41; (GC Retention time = 11.06 min) (7E,9E)-form: m/z 228 ($M^+$), 185, 171, 157, 144, 123, 109, 95, 81, 67, 54, 41.

Infrared absorption spectrum: (ATR): ν ($cm^{-1}$) 653, 729, 949, 987, 1464, 2856, 2928, 2956, 3015.

Example 2-14: Preparation of (7*E*)-7,9-dodecadienyl chloride (5: $X^2$ = Cl, *n* = 4, $R^2$ = ethyl group)

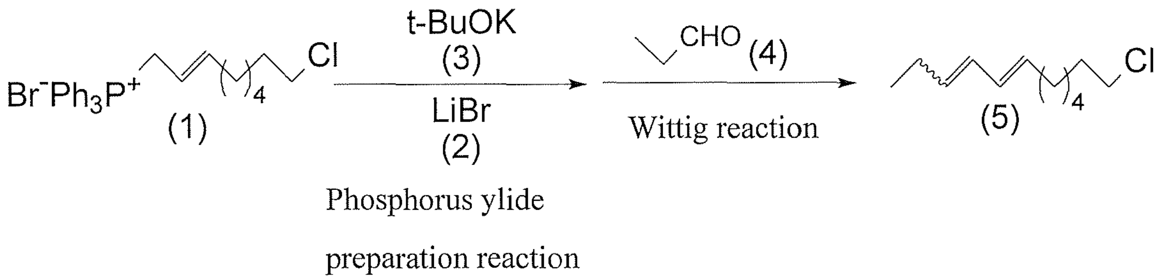


The procedures of Example 2-13 were repeated, with the proviso that propanal (4: $R^2$ = ethyl group) (58.08 g, 1.00 mol, purity 98.0%) was used instead of n-pentanal (4: $R^2$ = n-butyl group) as the aldehyde compound (4) used in Example 2-13. (7*E*)-7,9-Dodecadienyl chloride (5: $X^2$ = Cl, *n* = 4, $R^2$ = ethyl group) (194.73 g: 0.97 mol, yield 96.7%, purity 93.4%) was obtained as a result.

It was verified from the gas chromatography analysis that (7*E*)-7,9-dodecadienyl chloride (5: $X^2$ = Cl, *n* = 4, $R^2$ = ethyl group) thus prepared had two types of *cis-trans* isomers [isomer formation ratio = 44.8 (GC Retention time 12.98 min): 55.2 (GC Retention time 13.35 min)] at Retention times 12.98 minutes and 13.35 minutes, respectively. The above yields of (7*E*)-7,9-dodecadienyl chloride (5: $X^2$ = Cl, *n* = 4, $R^2$ = ethyl group) are represented as a mixture of the two types of *cis-trans* isomers.

The configurations of the two types of *cis-trans* isomers of (7E)-7,9-dodecadienyl chloride (5: $X^2$ = Cl, *n* = 4, $R^2$ = ethyl group) were determined by confirming the spin coupling constant between the double bonded hydrogen nuclei from $^1$H-NMR and two-dimensional NMR $^{13}$C non-irradiated HSQC slice data. As a result, the *cis-trans* isomer detected at GC Retention time 12.98 minutes was considered to be (7*E*,9*Z*)-7,9-dodecadienyl chloride, and the *cis-trans* isomer detected at GC Retention time 13.35 minutes was considered to be (7*E*,9*E*)-7,9-dodecadienyl chloride.

The following are the various spectrum data of (7*E*)-7,9-dodecadienyl chloride (5: $X^2$ = Cl, *n* = 4, $R^2$ = ethyl group) thus prepared.

Nuclear magnetic resonance spectrum: $^1$H-NMR (600 MHz, $CDCl_3$): δ 0.99 (1.2H, t, J = 7.5 Hz), 1.00 (1.8H, t, J = 7.4 Hz), 1.31-1.47 (6H, m), 1.74-1.79 (2H.m), 2.04-2.13 (3.2H, m), 2.17 (0.8H, quin, J = 7.4 Hz), 3.52 (1.2H, t, J = 6.8 Hz), 3.53 (0.8H, t, J = 6.8 Hz), 5.31 (0.4H, dt, J = 10.7, 7.5 Hz), 5.56 (0.6H, dt, J = 14.1, 7.0 Hz), 5.60-5.67 (1H, m), 5.91 (0.4H, t, J = 10.9 Hz), 5.97-6.03 (1.2H, m), 6.30 (0.4H, dd, J = 15.1, 11.0 Hz) ppm.

$^{13}$C-NMR (150 MHz, $CDCl_3$): δ 13.77, 14.46, 21.15, 25.72, 26.86, 26.87, 28.54, 28.57, 29.32, 29.35, 32.58, 32.71, 32.85, 45.26, 125.84, 128.07, 129.44, 130.68, 131.96, 132.19, 134.19, 134.44 ppm.

Spin coupling constant between double bonded hydrogen nuclei determined from $^1$H-NMR and two-dimensional NMR$^{13}$C non-irradiated HSQC slice data: (7*E*,9*Z*)-form: $J_{7,8}$ = 15.2 Hz (positions 7-8), $J_{9,10}$ = 10.8 Hz (positions 9-10); (7*E*,9*E*)-form: $J_{7,8}$ = 14.8 Hz (positions 7-8), $J_{9,10}$ = 14.9 Hz (positions 9-10).

Mass spectrum: EI (70 eV) (GC Retention time = 12.98 min) (7*E*,9*Z*)-form: m/z 200 ($M^+$), 171, 157, 144, 123, 109, 95, 82, 67, 55, 41, 27; (GC Retention time = 13.35 min) (7E,9E)-form: m/z 200 ($M^+$), 171, 157, 144, 123, 109, 95, 82, 67, 55, 41, 27.

Infrared absorption spectrum: (ATR): ν ($cm^{-1}$) 542, 652, 725, 947, 987, 1120, 1309, 1438, 1461, 2855, 2931, 2962, 3016.

Example 2-15: Preparation of (7E)-11-methyl-7,9-tetradecadienyl chloride (5: $X^2$ = Cl, *n* = 4, $R^2$ = 2-pentyl group)

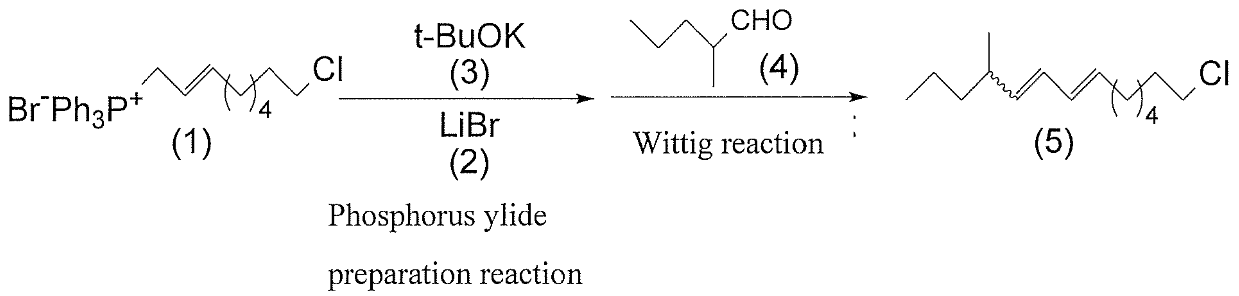

(1)
(5)

The procedures of Example 2-13 were repeated, with the proviso that 2-methylpentanal (4: $R^2$ = 2-pentyl group) (100.16 g, 1.00 mol, purity 92.4%) was used instead of n-pentanal (4: $R^2$ = n-butyl group) as the aldehyde compound (4) used in Example 2-13. (7*E*)-11-Methyl-7,9-tetradecadienyl chloride (5: $X^2$ = Cl, *n* = 4, $R^2$ = 2-pentyl group) (218.55 g: 0.90 mol, yield 90.3%, purity 96.9%) was obtained as a result.

It was verified from the gas chromatography analysis that (7*E*)-11-methyl-7,9-tetradecadienyl chloride (5: $X^2$ = Cl, *n* = 4, $R^2$ = 2-pentyl group) thus prepared had two types of *cis-trans* isomers [isomer formation ratio = 51.3 (GC Retention time 10.21 min): 48.7 (GC Retention time 11.43 min)] at Retention times 10.21 minutes and 11.43 minutes, respectively. The above yields of (7*E*)-1 1-methyl-7,9-tetradecadienyl chloride (5: $X^2$ = Cl, *n = 4,* $R^2$ = 2-pentyl group) are represented as a mixture of the two types of *cis-trans* isomers.

The configurations of the two types of *cis-trans* isomers of (7*E*)-11-methyl-7,9-tetradecadienyl chloride (5: $X^2$ = Cl, *n* = 4, $R^2$ = 2-pentyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 10.21 minutes was considered to be (7*E*,9*Z*)-11-methyl-7,9-dodecadienyl chloride, and the *cis-trans* isomer detected at GC Retention time 11.43 minutes was considered to be (7*E*,9*E*)-11-methyl-7,9-tetradecadienyl chloride.

The following are the various spectrum data of (7*E*)-11-methyl-7,9-tetradecadienyl chloride (5: $X^2$ = Cl, *n* = 4, $R^2$ = 2-pentyl group) thus prepared.

Nuclear magnetic resonance spectrum: $^1$H-NMR (600 MHz, $CDCl_3$): δ 0.87 (1.5H, t, J = 7.0 Hz), 0.88 (1.5H, t, J = 7.1 Hz), 0.96 (1.5H, J = 6.7 Hz), 0.98 (1.5H.d, J = 6.7 Hz), 1.26-1.36 (6H, m), 1.37-1.46 (4H, m), 1.74-1.80 (2H, m), 2.06 (1H, q, J = 7.4 Hz), 2.10 (1H, q, J = 7.2 Hz), 2.12-2.19 (0.5H, m), 2.56-2.64 (0.5H, m), 3.53 (1H, t, J = 6.8 Hz), 3.54 (1H, t, J = 6.8 Hz), 5.07 (0.5H, t, J = 10.4 Hz), 5.45 (0.5H, dd, J = 14.4, 7.9 Hz), 5.56 (0.5H, dt, J = 14.3, 7.1 Hz), 5.63 (0.5H, dt, J = 14.7, 7.3 Hz), 5.89 (0.5H, t, J = 10.9 Hz), 5.93-6.02 (1H, m), 6.28 (0.5H, dd, J = 15.0, 10.9 Hz) ppm.

$^{13}$C-NMR (150 MHz, $CDCl_3$): δ 14.31, 14.35, 20.58, 20.70, 20.72, 21.43, 26.86, 26.88, 28.55, 28.58, 29.30, 29.36, 32.06, 32.61, 32.72, 32.83, 36.57, 39.50, 39.98, 45.26, 126.20, 127.30, 128.51, 130.81, 132.16, 134.31, 136.85, 138.75 ppm.

Spin coupling constant between double bonded hydrogen nuclei determined from $^1$H-NMR and two-dimensional NMR$^{13}$C non-irradiated HSQC slice data: (7*E*,9*Z*)-form: $J_{7,8}$ = 14.7 Hz (positions 7-8), $J_{9,10}$ = 10.7 Hz (positions 9-10); (7*E*,9*E*)-form: $J_{7,8}$ = 14.3 Hz (positions 7-8), $J_{9,10}$ = 14.4 Hz (positions 9-10).

Mass spectrum: EI (70 eV) (GC Retention time = 10.21 min) (7*E*,9*Z*)-form: m/z 242 ($M^+$), 199, 171, 158, 143, 123, 109, 95, 81, 67, 55, 41; (GC Retention time = 11.43 min) (7E,9E)-form: m/z 242 ($M^+$), 199, 171, 158, 143, 123, 109, 95, 81, 67, 55, 41.

Infrared absorption spectrum: (ATR): ν ($cm^{-1}$) 654, 720, 949, 987, 1376, 1456, 2858, 2927, 2956, 3016.

Example 2-16: Preparation of (7*E*,11*E*)-7,9,11-tridecatrienyl chloride (5: $X^2$ = Cl, *n* = 4, $R^2$ = 1-propenyl group)

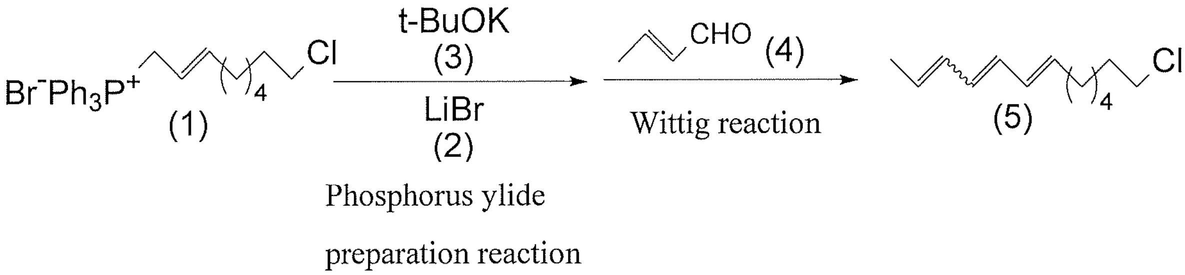


The procedures of Example 2-13 were repeated, with the proviso that (2*E*)-2-butenal (4: $R^2$ = 1-propenyl group) (70.09 g, 1.00 mol, purity 99.0%) was used instead of n-pentanal (4: $R^2$ = n-butyl group) as the aldehyde compound (4) used in Example 2-13. (7*E*,11*E*)-7,9,11-Tridecatrienyl chloride (5: $X^2$ = Cl, *n* = 4, $R^2$ = 1-propenyl group) (195.74 g: 0.92 mol, yield 91.9%, purity 96.9%) was obtained as a result.

It was verified from the gas chromatography analysis that (7*E*,11*E*)-7,9,11-tridecatrienyl chloride (5: $X^2$ = Cl, *n* = 4, $R^2$ = 1-propenyl group) thus prepared had two types of *cis-trans* isomers [isomer formation ratio = 53.0 (GC Retention time 10.62 min): 47.0 (GC Retention time 10.96 min)] at Retention times 10.62 minutes and 10.96 minutes, respectively. The above yields of (7*E*,11*E*)-7,9,11-tridecatrienyl chloride (5: $X^2$ = Cl, *n* = 4, $R^2$ = 1-propenyl group) are represented as a mixture of the two types of *cis-trans* isomers.

The configurations of the two types of *cis-trans* isomers of (7*E*,11*E*)-7,9,11-tridecatrienyl chloride (5: $X^2$ = Cl, *n* = 4, $R^2$ = 1-propenyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 10.62 minutes was considered to be (7E,9Z,11E)-7,9,11-tridecatrienyl chloride, and the *cis-trans* isomer detected at GC Retention time 10.96 minutes was considered to be (7*E*,9*E*,11*E*)-7,9,11-tridecatrienyl chloride.

The following are the various spectrum data of (7*E*,11*E*)-7,9,11-tridecatrienyl chloride (5: $X^2$ = Cl, *n* = 4, $R^2$ = 1-propenyl group) thus prepared.

Nuclear magnetic resonance spectrum: $^1$H-NMR (600 MHz, $CDCl_3$): δ 1.24-1.37 (2H, m), 1.38-1.48 (4H, m), 1.74-1.78 (3.5H, m), 1.80 (1.5H, J = 6.8 Hz), 2.09 (1H, q, J = 7.7 Hz), 2.13 (1H, q, J = 7.4 Hz), 3.52 (1H, t, J = 6.7 Hz), 3.53 (1H, t, J = 6.8 Hz), 5.62-5.73 (2H, m), 5.81-5.87 (1H, m), 6.01-6.09 (2H, m), 6.45-6.53 (1H, m) ppm.

$^{13}$C-NMR (150 MHz, $CDCl_3$): δ 18.41, 18.53, 26.86, 28.52, 28.56, 29.27, 32.70, 32.77, 32.92, 45.26, 126.08, 127.29, 127.46, 127.75, 129.02, 130.04, 130.63, 130.76, 130.99, 131.89, 134.17, 135.18 ppm.

Spin coupling constant between double bonded hydrogen nuclei determined from $^1$H-NMR and two-dimensional NMR$^{13}$C non-irradiated HSQC slice data: *(7E,9Z,11E)-form:* $J_{7,8}$ = 15.1 Hz (positions 7-8), $J_{9,10}$ = 11.4 Hz (positions 9-10), $J_{11,12}$ = 14.8 Hz (positions 11-12); (7E,9E,11E)-form: $J_{7,8}$ = 14.6 Hz (positions 7-8), $J_{9,10}$ = 14.5 Hz (positions 9-10), $J_{11,12}$ = 14.7 Hz (positions 11-12).

Mass spectrum: EI (70 eV) (GC Retention time = 10.62 min) *(7E,9Z,11E)-form:* m/z 212 ($M^+$), 144, 121, 107, 93, 79, 67, 55, 41; (GC Retention time = 10.96 min) *(7E,9E,11E)-form:* m/z 212 ($M^+$), 144, 121, 107, 93, 79, 67, 55, 41.

Infrared absorption spectrum: (ATR): ν ($cm^{-1}$) 542, 651, 724, 925, 963, 996, 1308, 1376, 1446, 2855, 2930, 3012.

Example 2-17: Preparation of (11E)-11,13-hexadecadienyl chloride (5: $X^2$ = Cl, *n* = 8, $R^2$ = ethyl group)

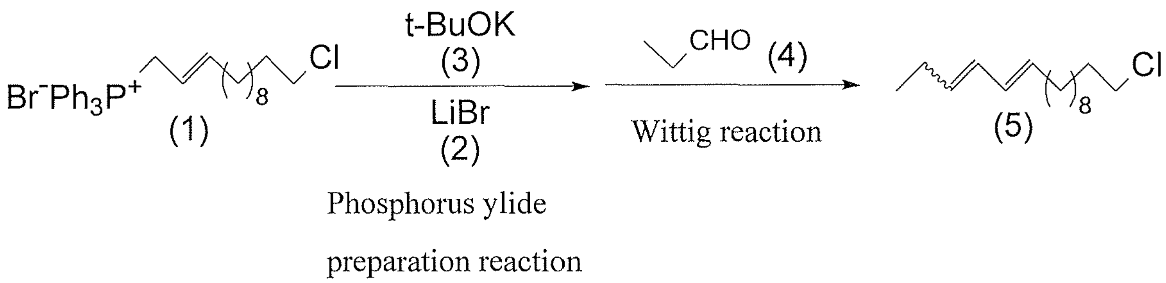


The procedures of Example 2-1 were repeated, with the proviso that (2E)- (13-chloro-2-tridecenyl)triphenylphosphonium bromide (1: $X^1$ = Br, $X^2$ = Cl, *n* = 8) (613.78 g, 1.10 mol, purity 96.6%) obtained as in Example 1-3 was used instead of (2E)- (6-chloro-2-hexenyl)triphenylphosphonium bromide (1: $X^1$ = Br, $X^2$ = Cl, *n* = 1) as the (ω-halo-2-alkenyl) triphenylphosphonium halide compound (1) used in Example 2-1, and propanal (4: $R^2$ = ethyl group) (58.08 g, 1.00 mol,

purity 98.0%) was used instead of (4Z)-4-decenal (4: $R^2$ = (3Z)-3-nonenyl group) as the aldehyde compound (4) used in Example 2-1. (11E)-11,13-Hexadecadienyl chloride (5: $X^2$ = Cl, *n* = 8, $R^2$ = ethyl group) (249.15 g: 0.97 mol, yield 96.6%, purity 88.5%) was obtained as a result.

It was verified from the gas chromatography analysis that (11E)-11,13-hexadecadienyl chloride (5: $X^2$ = Cl, *n* = 8, $R^2$ = ethyl group) thus prepared had two types of *cis-trans* isomers [isomer formation ratio = 44.4 (GC Retention time 15.38 min): 55.6 (GC Retention time 15.77 min)] at Retention times 15.38 minutes and 15.77 minutes, respectively. The above yields of (11E)-11,13-hexadecadienyl chloride (5: $X^2$ = Cl, n = 8, $R^2$ = ethyl group) are represented as a mixture of the two types of *cis-trans* isomers.

The configurations of the two types of *cis-trans* isomers of (11*E*)-11,13-hexadecadienyl chloride (5: $X^2$ = Cl, *n* = 8, $R^2$ = ethyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 15.38 minutes was considered to be (11*E*,13*Z*)-11,13-hexadecadienyl chloride, and the *cis-trans* isomer detected at GC Retention time 15.77 minutes was considered to be (11*E*,13*E*)-11,13-hexadecadienyl chloride.

The following are the various spectrum data of (11*E*)-11,13-hexadecadienyl chloride (5: $X^2$ = Cl, *n* = 8, $R^2$ = ethyl group) thus prepared.

Nuclear magnetic resonance spectrum: $^1$H-NMR (600 MHz, $CDCl_3$): $\delta$ 0.99 (1.3H, t, J = 7.5 Hz), 1.00 (1.7H, t, J = 7.5 Hz), 1.24-1.44 (14H, m), 1.76 (2H.quin, J = 7.2 Hz), 2.02-2.11 (3.1H, m), 2.18 (0.9H, quin, J = 7.4 Hz), 3.53 (2H, t, J = 6.9 Hz), 5.30 (0.4H, dt, J = 10.8, 7.2 Hz), 5.54-5.63 (1.2H, m), 5.66 (0.4H, dt, J = 14.7, 7.3 Hz), 5.92 (0.4H, t, J = 10.8 Hz), 5.97-6.03 (1.2H, m), 6.30 (0.4H, dd, J = 15.1, 10.8 Hz) ppm.

$^{13}$C-NMR(150 MHz, $CDCl_3$): $\delta$ 13.79, 14.47, 21.15, 25.73, 27.02, 29.02, 29.32, 29.35, 29.53, 29.57, 29.58, 29.59, 29.61, 32.74, 32.80, 33.01, 45.33, 125.63, 128.17, 129.53, 130.46, 131.78, 132.60, 134.00, 134.84 ppm.

Spin coupling constant between double bonded hydrogen nuclei determined from $^1$H-NMR and two-dimensional NMR$^{13}$C non-irradiated HSQC slice data: (11E,13Z)-form: $J_{11,12}$ = 14.9 Hz (positions 11-12), $J_{13,14}$ = 10.8 Hz (positions 13-14); (11E,13E)-form: $J_{11,12}$ = 14.3 Hz (positions 11-12), $J_{13,14}$ = 15.3 Hz (positions 13-14).

Mass spectrum: EI (70 eV) (GC Retention time = 15.38 min) *(11E,13Z)-form:* m/z 256 ($M^+$), 200, 172, 137, 123, 109, 96, 82, 64, 57, 41; (GC Retention time = 15.77 min) (11E,13E)-form: m/z 256 ($M^+$), 200, 172, 137, 123, 109, 96, 82, 64, 57, 41.

Example 3

The following Examples 3-1 to 3-8 describe preparation of an acetate compound of the following general formula (6) according to the following reaction formula.

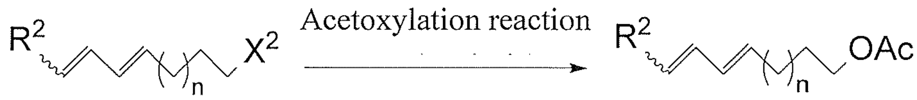


(5) (6)

Example 3-1: Preparation of (4*E*,10*Z*)-4,6,10-hexadecatrienyl acetate (6: *n* = 1, $R^2$ = (3Z)-3-nonenyl group)

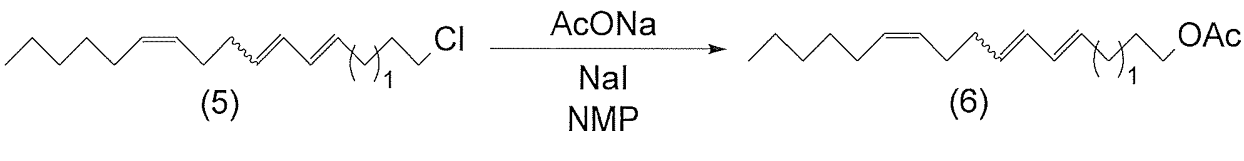


(5) (6)

Air in a reactor equipped with a stirrer, a condenser, and a thermometer was purged with nitrogen. (4*E*,10*Z*)-4,6,10-Hexadecatrienyl chloride (5: $X^2$ = Cl, *n* = 1, $R^2$ = (3*Z*)-3-nonenyl group) (254.84 g: 1.00 mol, purity 93.1%) obtained as in Example 2-1, sodium acetate (AcONa) (98.41 g, 1.20 mol), sodium iodide (NaI) (8.99 g, 0.06 mol), and *N*-methyl-2-pyrrolidone (NMP) (300.0 g) were placed in the reactor. The reaction mixture was allowed to react for 1.5 hours at a liquid temperature of 120°C to 130°C.

After the completion of the reaction, water (360.0 g) was added to the reactor to quench the reaction, followed by adding n-hexane (200.0 g) and separating into an organic layer and an aqueous layer. The organic layer thus obtained was washed sequentially with an aqueous solution of 10% by weight sodium chloride (280.0 g) followed by an aqueous solution of 8% by weight sodium bicarbonate (250.0 g), and then the solvent was removed from the organic layer at a reduced pressure.

The crude product thus obtained was purified by distillation at a reduced pressure to obtain (4E,10Z)-4,6,10-hexadecatrienyl acetate (6: *n* = 1, $R^2$ = (3Z)-3-nonenyl group) (256.16 g: 0.92 mol, yield 92.3%, purity 97.3%).

It was verified from the gas chromatography analysis that (4E,10Z)-4,6,10-hexadecatrienyl acetate (6: *n* = 1, $R^2$ = (3*Z*)-3-nonenyl group) thus prepared had two types of *cis-trans* isomers [isomer formation ratio = 40.7 (GC Retention time 18.68 min): 59.3 (GC Retention time 19.69 min)] at Retention times 18.68 minutes and 19.69 minutes, respectively. The above yields of (4*E*,10*Z*)-4,6,10-hexadecatrienyl acetate (6: *n* = 1, $R^2$ = (3*Z*)-3-nonenyl group) are represented as a mixture of the two types of *cis-trans* isomers.

The configurations of the two types of *cis-trans* isomers of (4*E*,10*Z*)-4,6,10-hexadecatrienyl acetate (6: *n* = 1, $R^2$ = (3*Z*)-3-nonenyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 18.68 minutes was considered to be (4*E*,6*Z*,10*Z*)-4,6,10-hexadecatrienyl acetate, and the *cis-trans* isomer detected at GC Retention time 19.69 minutes was considered to be (4*E*,6*E*,10*Z*)-4,6,10-hexadecatrienyl acetate.

The following are the various spectrum data of (4*E*,10*Z*)-4,6,10-hexadecatrienyl acetate (6: *n* = 1, $R^2$ = (3Z)-3-nonenyl group) thus prepared.

Nuclear magnetic resonance spectrum: $^1$H-NMR (500 MHz, $CDCl_3$): δ 0.88 (3H, t, J = 7.7 Hz), 1.23-1.36 (6H, m), 1.68-1.76 (2H, m), 2.03 (2H.quin, J = 6.6 Hz), 2.04 (1.8H, s), 2.05 (1.2H, s), 2.10-2.28 (6H, m), 4.06 (1.2H, t, J = 6.1 Hz), 4.07 (0.8H, t, J = 5.0 Hz), 5.31-5.45 (2.5H, m), 5.50-5.71 (1.5H, m), 5.95 (0.4H, t, J = 11.1 Hz), 5.98-6.04 (1.2H, m), 6.32 (0.4H, dd, J = 14.8, 11.5 Hz) ppm.

$^{13}$C-NMR (126 MHz, $CDCl_3$): δ 14.05, 20.95, 22.56, 27.04, 27.20, 27.27, 27.84, 28.26, 28.87, 29.17, 29.34, 29.36, 31.48, 32.68, 63.92, 63.94, 126.43, 128.58, 128.76, 129.95, 130.28, 130.51, 130.58, 131.19, 132.39, 132.89 ppm.

Spin coupling constant between double bonded hydrogen nuclei determined from $^1$H-NMR and two-dimensional NMR$^{13}$C non-irradiated HSQC slice data: (4*E*,6*Z*,10*Z*)-form: $J_{4,5}$ = 15.1 Hz (positions 4-5), $J_{6,7}$ = 10.6 Hz (positions 6-7), $J_{10,11}$ = 10.3 Hz (positions 10-11); (4*E*,6*E*,10*Z*)-form: $J_{4,5}$ = 15.1 Hz (positions 4-5), $J_{6,7}$ = 14.7 Hz (positions 6-7), $J_{10,11}$ = 10.9 Hz (positions 10-11).

Mass spectrum: EI (70 eV) (GC Retention time = 18.68 min) (4*E*,6*Z*,10*Z*)-form: m/z 278 ($M^+$), 218, 190, 175, 161, 147, 133, 119, 107, 93, 79, 65, 55, 43, 29; (GC Retention time = 19.69 min) (4*E*,6*E*,10*Z*)-form: m/z 278 ($M^+$), 218, 177, 161, 150, 133, 107, 93, 79, 65, 55, 43, 29.

Infrared absorption spectrum: (ATR): ν ($cm^{-1}$) 732, 947, 988, 1042, 1239, 1365, 1386, 1449, 1742, 2855, 2926, 2956, 3008.

Example 3-2: Preparation of (4*E*)-4,6-hexadecadienyl acetate (6: *n* = 1, $R^2$ = n-nonyl group)

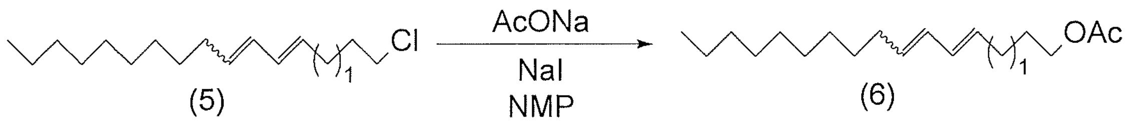


The procedures of Example 3-1 were repeated, with the proviso that (4*E*)-4,6-hexadecadienyl chloride (5: $X^2$ = Cl, *n* = 1, $R^2$ = n-nonyl group) (256.86 g: 1.00 mol, purity 94.1%) obtained as in Example 2-11 was used instead of (4*E*,10*Z*)-4,6,10-hexadecatrienyl chloride (5: $X^2$ = Cl, *n* = 1, $R^2$ = (3Z)-3-nonenyl group) as the organohalogen compound (5) used in Example 3-1. (4*E*)-4,6-Hexadecadienyl acetate (6: *n* = 1, $R^2$ = n-nonyl group) (249.15 g: 0.92 mol, yield 92.2%, purity 97.1%) was obtained as a result.

It was verified from the gas chromatography analysis that (4*E*)-4,6-hexadecadienyl acetate (6: *n* = 1, $R^2$ = n-nonyl group) thus prepared had two types of *cis-trans* isomers [isomer formation ratio = 42.7 (GC Retention time 17.64 min): 57.3 (GC Retention time 18.77 min)] at Retention times 17.64 minutes and 18.77 minutes, respectively. The above yields of (4*E*)-4,6-hexadecadienyl acetate (6: *n* = 1, $R^2$ = n-nonyl group) are represented as a mixture of the two types of *cis-trans* isomers.

The configurations of the two types of *cis-trans* isomers of (4*E*)-4,6-hexadecadienyl acetate (6: *n* = 1, $R^2$ = n-nonyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 17.64 minutes was considered to be (4*E*,6*Z*)-4,6-hexadecadienyl acetate, and the *cis-trans* isomer detected at GC Retention time 18.77 minutes was considered to be (4*E*,6*E*)-4,6-hexadecadienyl acetate.

The following are the various spectrum data of (4*E*)-4,6-hexadecadienyl acetate (6: *n* = 1, $R^2$ = n-nonyl group) thus prepared.

Nuclear magnetic resonance spectrum: $^1$H-NMR (500 MHz, $CDCl_3$): δ 0.88 (3H, t, J = 6.9 Hz), 1.27-1.36 (14H, m),

1.68-1.76 (2H, m), 2.03 (1.8H.s), 2.04 (1.2H, s), 2.09-2.21 (4H, q, m), 4.06 (1.2H, t, J = 6.5 Hz), 4.07 (0.8H, t, J = 6.5 Hz), 5.32 (0.4H, dt, J = 14.7, 5.5 Hz), 5.50-5.65 (1.6H, m), 5.91-6.04 (1.6H, m), 6.32 (0.4H, dd, J = 15.0, 11.0 Hz) ppm.

$^{13}$C-NMR(126 MHz, $CDCl_3$): δ 14.09, 20.94, 22.65, 27.70, 28.30, 28.87, 29.17, 29.19, 29.26, 29.31, 29.36, 29.50, 29.56, 29.68, 31.87, 32.57, 126.50, 128.20, 129.94, 130.26, 130.83, 131.30, 132.56, 133.21, 171.11 ppm.

Spin coupling constant between double bonded hydrogen nuclei determined from $^1$H-NMR and two-dimensional NMR $^{13}$C non-irradiated HSQC slice data: (4E,6Z)-form: $J_{4,5}$ = 15.1 Hz (positions 4-5), $J_{6,7}$ = 11.0 Hz (positions 6-7); (4*E*,6*E*)-form: $J_{4,5}$ = 15.0 Hz (positions 4-5), $J_{6,7}$ = 14.7 Hz (positions 6-7).

Mass spectrum: EI (70 eV) (GC Retention time = 17.64 min) (4*E*,6*Z*)-form: m/z 280 ($M^+$), 220, 149, 135, 121, 107, 93, 79, 57, 43, 28; (GC Retention time = 18.77 min) (4*E*,6*E*)-form: m/z 280 ($M^+$), 220, 149, 135, 121, 107, 93, 79, 57, 43, 29.

Infrared absorption spectrum: (ATR): ν ($cm^{-1}$) 606, 948, 987, 1042, 1239, 1365, 1387, 1466, 1743, 2854, 2924, 2955, 3017.

Example 3-3: Preparation of (4*E*)-7-phenyl-4,6-heptadienyl acetate (6: *n* = 1, $R^2$ = phenyl group)

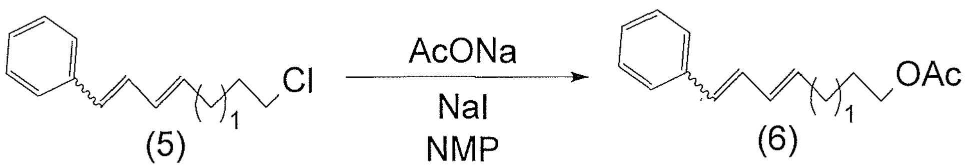

(5) (6)

The procedures of Example 3-1 were repeated, with the proviso that (4*E*)-7-phenyl-4,6-heptadienyl chloride (5: $X^2$ = Cl, *n* = 1, $R^2$ = phenyl group) (206.71 g: 1.00 mol, purity 96.2%) obtained as in Example 2-12 was used instead of (4*E*,10*Z*)-4,6,10-hexadecatrienyl chloride (5: $X^2$ = Cl, *n* = 1, $R^2$ = (3Z)-3-nonenyl group) as the organohalogen compound (5) used in Example 3-1. (4*E*)-7-Phenyl-4,6-heptadienyl acetate (6: *n* = 1, $R^2$ = phenyl group) (211.88 g: 0.92 mol, yield 91.8%, purity 98.0%) was obtained as a result.

It was verified from the gas chromatography analysis that (4*E*)-7-phenyl-4,6-heptadienyl acetate (6: *n* = 1, $R^2$ = phenyl group) thus prepared had two types of *cis-trans* isomers [isomer formation ratio = 57.3 (GC Retention time 17.03 min): 42.7 (GC Retention time 21.59 min)] at Retention times 17.03 minutes and 21.59 minutes, respectively. The above yields of (4*E*)-7-phenyl-4,6-heptadienyl acetate (6: *n* = 1, $R^2$ = phenyl group) are represented as a mixture of the two types of *cis-trans* isomers.

The configurations of the two types of *cis-trans* isomers of (4*E*)-7-phenyl-4,6-heptadienyl acetate (6: *n* = 1, $R^2$ = phenyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 17.03 minutes was considered to be (4*E*,6*Z*)-7-phenyl-4,6-heptadienyl acetate, and the *cis-trans* isomer detected at GC Retention time 21.59 minutes was considered to be (4*E*,6*E*)-7-phenyl-4,6-heptadienyl acetate.

The following are the various spectrum data of (4*E*)-7-phenyl-4,6-heptadienyl acetate (6: *n* = 1, $R^2$ = phenyl group) thus prepared.

Nuclear magnetic resonance spectrum: $^1$H-NMR (500 MHz, $CDCl_3$): δ 1.76 (2H, sep, J = 6.9 Hz), 2.04 (1.8H, s), 2.06 (1.2H, s), 2.21 (2H.sext, J = 6.7 Hz), 4.08 (1.2H, t, J = 6.7 Hz), 4.10 (0.8H, t, J = 6.5 Hz), 5.77-5.87 (1H, dd, J = 15.1, 8.7 Hz), 6.37 (0.4H, J = 11.5 Hz), 6.48 (0.6H, m), 6.18-6.26 (1H, m), 6.34 (0.6H, J = 11.5 Hz), 6.46 (0.4H, J = 15.7 Hz), 6.62 (0.6H, dd, J = 15.2, 11.2 Hz), 6.74 (0.4H, dd, J = 15.7, 10.3 Hz), 7.18-7.25 (1H, m), 7.28-7.39 (4H, m) ppm.

$^{13}$C-NMR (125 MHz, $CDCl_3$): δ 20.93, 20.95, 28.13, 28.19, 29.14, 29.18, 63.81, 63.85, 126.13, 126.72, 127.18, 127.32, 128.17, 128.19, 128.52, 128.84, 128.97, 130.11, 130.58, 131.33, 133.81, 136.00, 137.42, 137.63, 171.09, 171.11 ppm.

Spin coupling constant between double bonded hydrogen nuclei determined from $^1$H-NMR and two-dimensional NMR$^{13}$C non-irradiated HSQC slice data: (4E,6Z)-form: $J_{4,5}$ = 15.2 Hz (positions 4-5), $J_{6,7}$ = 11.5 Hz (positions 6-7); (4*E*,6*E*)-form: $J_{4,5}$ = 15.1 Hz (positions 4-5), $J_{6,7}$ = 15.7 Hz (positions 6-7).

Mass spectrum: EI (70 eV) (GC Retention time = 17.03 min) (4*E*,6*Z*)-form: m/z 230 ($M^+$), 170, 155, 141, 128, 115, 103, 91, 78, 65, 43; (GC Retention time = 21.59 min) (4*E*,6*E*)-form: m/z 230 ($M^+$), 170, 155, 141, 128, 115, 103, 91, 79, 65, 43.

Infrared absorption spectrum: (ATR): ν ($cm^{-1}$) 606, 701, 748, 771, 950, 990, 1042, 1241, 1366, 1387, 1447, 1492, 1596, 1644, 1739, 2954, 3022.

Example 3-4: Preparation of (7*E*)-7,9-tetradecadienyl acetate (6: *n* = 4, $R^2$ = n-butyl group)

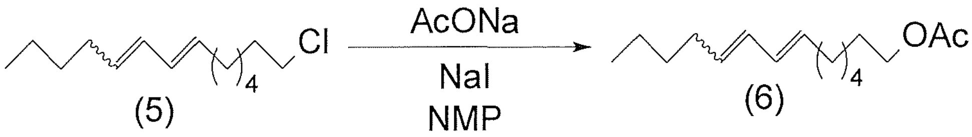


The procedures of Example 3-1 were repeated, with the proviso that (7*E*)-7,9-tetradecadienyl chloride (5: $X^2$ = Cl, *n* = 4, $R^2$ = n-butyl group) (228.80 g: 1.00 mol, purity 95.5%) obtained as in Example 2-13 was used instead of (4*E*,10*Z*)-4,6,10-hexadecatrienyl chloride (5: $X^2$ = Cl, *n* = 1, $R^2$ = (3*Z*)-3-nonenyl group) as the organohalogen compound (5) used in Example 3-1. (7*E*)-7,9-Tetradecadienyl acetate (6: *n* = 4, $R^2$ = n-butyl group) (234.72 g: 0.93 mol, yield 93.8%, purity 97.1%) was obtained as a result.

It was verified from the gas chromatography analysis that (7*E*)-7,9-tetradecadienyl acetate (6: *n* = 4, $R^2$ = n-butyl group) thus prepared had two types of *cis-trans* isomers [isomer formation ratio = 41.1 (GC Retention time 14.30 min): 58.9 (GC Retention time 14.48 min)] at Retention times 14.30 minutes and 14.48 minutes, respectively. The above yields of (7*E*)-7,9-tetradecadienyl acetate (6: *n* = 4, $R^2$ = n-butyl group) are represented as a mixture of the two types of *cis-trans* isomers.

The configurations of the two types of *cis-trans* isomers of (7*E*)-7,9-tetradecadienyl acetate (6: *n* = 4, $R^2$ = n-butyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 14.30 minutes was considered to be (7*E*,9*Z*)-7,9-tetradecadienyl acetate, and the *cis-trans* isomer detected at GC Retention time 14.48 minutes was considered to be (7*E*,9*E*)-7,9-tetradecadienyl acetate.

The following are the various spectrum data of (7E)-7,9-tetradecadienyl acetate *(6: n* = 4, $R^2$ = n-butyl group) thus prepared.

Nuclear magnetic resonance spectrum: $^1$H-NMR (600 MHz, $CDCl_3$): δ 0.89 (1.8H, t, J = 7.7 Hz), 0.90 (1.2H, t, J = 7.7 Hz), 1.28-1.43 (10H, m), 1.59-1.64 (2H.m), 2.13-2.07 (5.4H, m), 2.09 (0.8H, q, J = 7.1 Hz), 2.16 (0.8H, q, J = 6.8 Hz), 4.04 (1.2H, t, J = 6.8 Hz), 4.05 (0.8H, t, J = 6.8 Hz), 5.30 (0.4H, dt, J = 10.7, 7.6 Hz), 5.51-5.60 (1.2H, m), 5.64 (0.4H, dt, J = 14.8, 7.3 Hz), 5.93 (0.4H, t, J = 10.9 Hz), 5.97-6.02 (1.2H, m), 6.30 (0.4H, dd, J = 15.1, 10.9 Hz) ppm.

$^{13}$C-NMR (150 MHz, $CDCl_3$): δ 14.08, 14.10, 21.15, 22.38, 22.46, 25.93, 27.53, 28.68, 28.69, 28.92, 28.95, 29.40, 31.71, 32.03, 32.40, 32.60, 32.89, 64.74, 125.92, 128.65, 130.36, 130.38, 130.65, 132.18, 132.69, 134.45, 171.37 ppm.

Spin coupling constant between double bonded hydrogen nuclei determined from $^1$H-NMR and two-dimensional NMR$^{13}$C non-irradiated HSQC slice data: (7*E*,9*Z*)-form: $J_{7,8}$ = 15.0 Hz (positions 7-8), $J_{9,10}$ = 10.8 Hz (positions 9-10); (7*E*,9*E*)-form: $J_{7,8}$ = 14.5 Hz (positions 7-8), $J_{9,10}$ = 15.0 Hz (positions 9-10).

Mass spectrum: EI (70 eV) (GC Retention time = 14.30 min) (7*E*,9*Z*)-form: m/z 252 ($M^+$), 192, 163, 149, 135, 121, 95, 81, 67, 43; (GC Retention time = 14.48 min) (7*E*,9*E*)-form: m/z 252 ($M^+$), 192, 163, 149, 135, 121, 95, 81, 67, 43.

Infrared absorption spectrum: (ATR): ν ($cm^{-1}$) 606, 728, 948, 987, 1038, 1239, 1365, 1465, 1741, 2857, 2928, 2955, 3015.

Example 3-5: Preparation of (7*E*)-7,9-dodecadienyl acetate (6: *n* = 4, $R^2$ = ethyl group)

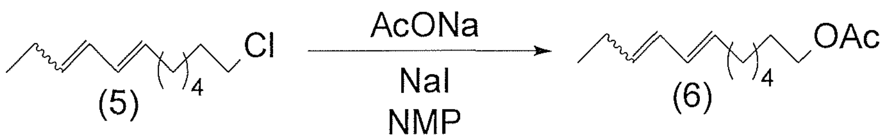


The procedures of Example 3-1 were repeated, with the proviso that (7*E*)-7,9-dodecadienyl chloride (5: $X^2$ = Cl, *n* = 4, $R^2$ = ethyl group) (200.75 g: 1.00 mol, purity 93.4%) obtained as in Example 2-14 was used instead of (4*E*,10*Z*)-4,6,10-hexadecatrienyl chloride (5: $X^2$ = Cl, *n* = 1, $R^2$ = (3*Z*)-3-nonenyl group) as the organohalogen compound (5) used in Example 3-1. (7*E*)-7,9-Dodecadienyl acetate (6: *n* = 4, $R^2$ = ethyl group) (188.45 g: 0.84 mol, yield 83.5%, purity 97.9%) was obtained as a result.

It was verified from the gas chromatography analysis that (7*E*)-7,9-dodecadienyl acetate (6: *n* = 4, $R^2$ = ethyl group) thus prepared had two types of *cis-trans* isomers [isomer formation ratio = 44.9 (GC Retention time 17.53 min): 55.1

(GC Retention time 17.98 min)] at Retention times 17.53 minutes and 17.98 minutes, respectively. The above yields of (7*E*)-7,9-dodecadienyl acetate (6: *n* = 4, $R^2$ = ethyl group) are represented as a mixture of the two types of *cis-trans* isomers.

The configurations of the two types of *cis-trans* isomers of (7*E*)-7,9-dodecadienyl acetate (6: *n* = 4, $R^2$ = ethyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 17.53 minutes was considered to be (7*E*,9*Z*)-7,9-dodecadienyl acetate, and the *cis-trans* isomer detected at GC Retention time 17.98 minutes was considered to be (7*E*,9*E*)-7,9-dodecadienyl acetate.

The following are the various spectrum data of (7*E*)-7,9-dodecadienyl acetate (6: *n* = 4, $R^2$ = ethyl group) thus prepared.

Nuclear magnetic resonance spectrum: $^1$H-NMR (600 MHz, $CDCl_3$): δ 0.99 (1.2H, t, J = 7.5 Hz), 1.00 (1.8H, t, J = 7.4 Hz), 1.30-1.43 (6H, m), 1.59-1.64 (2H.m), 2.03-2.11 (6.2H, m), 2.17 (0.8H, quin, J = 7.5 Hz), 4.03 (1.2H, t, J = 6.8 Hz), 4.05 (0.8H, t, J = 6.8 Hz), 5.30 (0.4H, dt, J = 10.7, 7.6 Hz), 5.53-5.66 (1.6H, m), 5.91 (0.4H, t, J = 10.9 Hz), 5.97-6.02 (1.2H, m), 6.29 (0.4H, dd, J = 15.1, 11.1 Hz) ppm.

$^{13}$C-NMR (150 MHz, $CDCl_3$): δ 13.76, 14.44, 21.14, 25.71, 25.92, 28.67, 28.90, 28.94, 29.37, 29.40, 32.59, 32.87, 64.73, 125.78, 128.08, 129.45, 130.63, 131.92, 132.25, 134.14, 134.50, 171.36 ppm.

Spin coupling constant between double bonded hydrogen nuclei determined from $^1$H-NMR and two-dimensional NMR$^{13}$C non-irradiated HSQC slice data: (7*E*,9*Z*)-form: $J_{7,8}$ = 15.1 Hz (positions 7-8), $J_{9,10}$ = 10.8 Hz (positions 9-10); (7*E*,9*E*)-form: $J_{7,8}$ = 14.8 Hz (positions 7-8), $J_{9,10}$ = 15.3 Hz (positions 9-10).

Mass spectrum: EI (70 eV) (GC Retention time = 17.53 min) (7*E*,9*Z*)-form: m/z 224 ($M^+$), 164, 135, 121, 107, 95, 79, 67, 55, 43; (GC Retention time = 17.98 min) (7*E*,9*E*)-form: m/z 224 ($M^+$), 164, 135, 121, 107, 95, 79, 67, 55, 43.

Infrared absorption spectrum: (ATR): ν ($cm^{-1}$) 606, 728, 948, 987, 1038, 1239, 1365, 1387, 1462, 1742, 2856, 2931, 2962, 3016.

Example 3-6: Preparation of (7*E*)-11-methyl-7,9-tetradecadienyl acetate (6: *n* = 4, $R^2$ = 2-pentyl group)

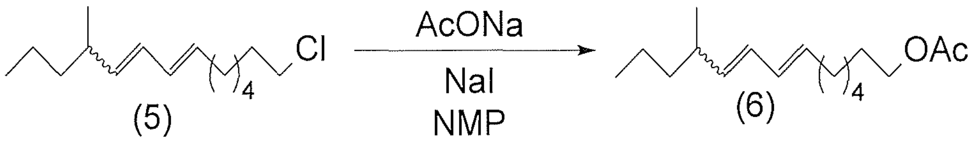


The procedures of Example 3-1 were repeated, with the proviso that (7*E*)-11-methyl-7,9-tetradecadienyl chloride (5: $X^2$ = Cl, *n* = 4, $R^2$ = 2-pentyl group) (242.83 g: 1.00 mol, purity 96.9%) obtained as in Example 2-15 was used instead of (4*E*,10*Z*)-4,6,10-hexadecatrienyl chloride (5: $X^2$ = Cl, *n* = 1, $R^2$ = (3*Z*)-3-nonenyl group) as the organohalogen compound (5) used in Example 3-1. (7*E*)-11-Methyl-7,9-tetradecadienyl acetate (6: *n* = 4, $R^2$ = 2-pentyl group) (245.11 g: 0.92 mol, yield 91.9%, purity 97.2%) was obtained as a result.

It was verified from the gas chromatography analysis that (7*E*)-11-methyl-7,9-tetradecadienyl acetate (6: *n* = 4, $R^2$ = 2-pentyl group) thus prepared had two types of *cis-trans* isomers [isomer formation ratio = 52.1 (GC Retention time 14.06 min): 47.9 (GC Retention time 15.36 min)] at Retention times 14.06 minutes and 15.36 minutes, respectively. The above yields of (7*E*)-11-methyl-7,9-tetradecadienyl acetate (6: *n* = 4, $R^2$ = 2-pentyl group) are represented as a mixture of the two types of *cis-trans* isomers.

The configurations of the two types of *cis-trans* isomers of (7*E*)-11-methyl-7,9-tetradecadienyl acetate (6: *n* = 4, $R^2$ = 2-pentyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 14.06 minutes was considered to be (7*E*,9*Z*)-11-methyl-7,9-tetradecadienyl acetate, and the *cis-trans* isomer detected at GC Retention time 15.36 minutes was considered to be (7*E*,9*E*)-11-methyl-7,9-tetradecadienyl acetate.

The following are the various spectrum data of (7*E*)-11-methyl-7,9-tetradecadienyl acetate (6: *n* = 4, $R^2$ = 2-pentyl group) thus prepared.

Nuclear magnetic resonance spectrum: $^1$H-NMR (600 MHz, $CDCl_3$): δ 0.87 (1.5H, t, J = 7.0 Hz), 0.88 (1.5H, t, J = 7.1 Hz), 0.95 (1.5H, J = 6.7 Hz), 0.97 (1.5H.d, J = 6.7 Hz), 1.18-1.43 (10H, m), 1.59-1.64 (2H, m), 2.03-2.06 (4H, m), 2.08 (1H, q, J = 7.6 Hz), 2.11-2.16 (0.5H, m), 2.55-2.63 (0.5H, m), 4.05 (1H, t, J = 6.8 Hz), 4.06 (1H, t, J = 6.8 Hz), 5.07 (0.5H, t, J = 10.4 Hz), 5.44 (0.5H, dd, J = 14.5, 7.9 Hz), 5.56 (0.5H, dt, J = 14.3, 7.1 Hz), 5.63 (0.5H, dt, J = 14.7, 7.3 Hz), 5.89 (0.5H, t, J = 10.9 Hz), 5.93-6.01 (1H, m), 6.27 (0.5H, dd, J = 15.0, 11.0 Hz) ppm.

$^{13}$C-NMR (150 MHz, $CDCl_3$): δ 14.30, 14.34, 20.57, 20.69, 20.71, 21.15, 21.42, 25.93, 28.68, 28.70, 28.94,

29.36, 29.41, 32.05, 32.63, 32.87, 36.56, 39.49, 39.98, 64.74, 126.14, 127.31, 128.52, 130.75, 132.23, 134.39, 136.81, 138.71, 171.37 ppm.

Spin coupling constant between double bonded hydrogen nuclei determined from $^1$H-NMR and two-dimensional NMR$^{13}$C non-irradiated HSQC slice data: (7*E*,9*Z*)-form: $J_{7,8}$ = 14.9 Hz (positions 7-8), $J_{9,10}$ = 10.7 Hz (positions 9-10); (7*E*,9*E*)-form: $J_{7,8}$ = 14.3 Hz (positions 7-8), $J_{9,10}$ = 14.5 Hz (positions 9-10).

Mass spectrum: EI (70 eV) (GC Retention time = 14.06 min) (7*E*,9*Z*)-form: m/z 266 ($M^+$), 223, 177, 163, 149, 135, 121, 107, 95, 81, 67, 55, 43; (GC Retention time = 15.36 min) (7E,9E)-form: m/z 242 ($M^+$), 199, 171, 158, 143, 123, 109, 95, 81, 67, 55, 41.

Infrared absorption spectrum: (ATR): ν ($cm^{-1}$) 740, 949, 987, 1039, 1239, 1365, 1456, 1742, 2858, 2928, 2956.

Example 3-7: Preparation of (7*E*,11*E*)-7,9,11-tridecatrienyl acetate (6: *n* = 4, $R^2$ = 1-propenyl group)

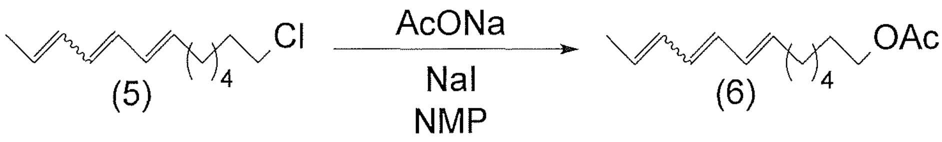


The procedures of Example 3-1 were repeated, with the proviso that (7*E*,11*E*)-7,9,11-tridecatrienyl chloride (5: $X^2$ = Cl, *n* = 4, $R^2$ = 1-propenyl group) (212.76 g: 1.00 mol, purity 96.9%) obtained as in Example 2-16 was used instead of (4*E*,10*Z*)-4,6,10-hexadecatrienyl chloride (5: $X^2$ = Cl, *n* = 1, $R^2$ = (3*Z*)-3-nonenyl group) as the organohalogen compound (5) used in Example 3-1. (7*E*,11*E*)-7,9,11-Tridecatrienyl acetate (6: *n* = 4, $R^2$ = 1-propenyl group) (198.53 g: 0.84 mol, yield 83.9%, purity 98.3%) was obtained as a result.

It was verified from the gas chromatography analysis that (7*E*,11*E*)-7,9,11-tridecatrienyl acetate (6: *n* = 4, $R^2$ = 1-propenyl group) thus prepared had two types of *cis-trans* isomers [isomer formation ratio = 49.3 (GC Retention time 13.15 min): 50.7 (GC Retention time 13.57 min)] at Retention times 13.15 minutes and 13.57 minutes, respectively. The above yields of (7*E*,11*E*)-7,9,11-tridecatrienyl acetate (6: *n* = 4, $R^2$ = 1-propenyl group) are represented as a mixture of the two types of *cis-trans* isomers.

The configurations of the two types of *cis-trans* isomers of (7*E*,11*E*)-7,9,11-tridecatrienyl acetate (6: *n* = 4, $R^2$ = 1-propenyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 13.15 minutes was considered to be (7*E*,9*Z*,11*E*)-7,9,11-tridecatrienyl acetate, and the *cis-trans* isomer detected at GC Retention time 13.57 minutes was considered to be (7*E*,9*E*,11*E*)-7,9,11-tridecatrienyl acetate.

The following are the various spectrum data of (7*E*,11*E*)-7,9,11-tridecatrienyl acetate (6: *n* = 4, $R^2$ = 1-propenyl group) thus prepared.

Nuclear magnetic resonance spectrum: $^1$H-NMR (600 MHz, $CDCl_3$): δ 1.29-1.43 (6H, m), 1.58-1.64 (2H, m), 1.76 (1.5H, J = 6.7 Hz), 1.80 (1.5H, J = 6.8 Hz), 2.04 (3H, s), 2.08 (1H, q, J = 7.7 Hz), 2.11 (1H, q, J = 6.9 Hz), 4.04 (1H, t, J = 6.7 Hz), 4.05 (1H, t, J = 6.7 Hz), 5.61-5.75 (2H, m), 5.80-5.86 (1H, m), 6.00-6.10 (2H, m), 6.45-6.52 (1H, m) ppm.

$^{13}$C-NMR (150 MHz, $CDCl_3$): δ 18.40, 18.52, 21.14, 25.91, 25.92, 28.67, 28.89, 28.94, 29.33, 32.80, 32.95, 64.71, 126.02, 127.28, 127.48, 127.72, 128.98, 130.01, 130.65, 130.71, 130.95, 131.89, 134.24, 135.25, 171.36 ppm.

Spin coupling constant between double bonded hydrogen nuclei determined from $^1$H-NMR and two-dimensional NMR$^{13}$C non-irradiated HSQC slice data: (7*E*,9*Z*,11*E*)-form: $J_{7,8}$ = 15.2 Hz (positions 7-8), $J_{9,10}$ = 11.3 Hz (positions 9-10), $J_{11,12}$ = 14.8 Hz (positions 11-12); (7E,9E,11E)-form: $J_{7,8}$ = 14.6 Hz (positions 7-8), $J_{9,10}$ = 14.6 Hz (positions 9-10), $J_{11,12}$ = 15.0 Hz (positions 11-12).

Mass spectrum: EI (70 eV) (GC Retention time = 13.15 min) (7*E*,9*Z*,11*E*)-form: m/z 236 ($M^+$), 147, 133, 119, 105, 91, 79, 67, 55, 43; (GC Retention time = 13.57 min) (7*E*,9*E*,11*E*)-form: m/z 236 ($M^+$), 147, 133, 119, 105, 91, 79, 67, 55, 43.

Infrared absorption spectrum: (ATR): ν ($cm^{-1}$) 606, 723, 926, 964, 997, 1038, 1240, 1365, 1437, 1740, 2855, 2929, 3013.

Example 3-8: Preparation of (11*E*)-11,13-hexadecadienyl acetate (6: *n* = 8, $R^2$ = ethyl group)

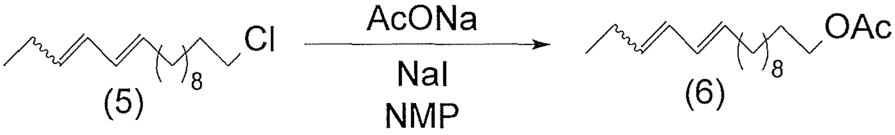

(5) (6)

The procedures of Example 3-1 were repeated, with the proviso that (11*E*)-11,13-hexadecadienyl chloride (5: $X^2$ = Cl, *n* = 8, $R^2$ = ethyl group) (256.86 g: 1.00 mol, purity 88.5%) obtained as in Example 2-17 was used instead of (4E,10Z)-4,6,10-hexadecatrienyl chloride (5: $X^2$ = Cl, *n* = 1, $R^2$ = (3Z)-3-nonenyl group) as the organohalogen compound (5) used in Example 3-1. (11*E*)-11,13-Hexadecadienyl acetate *(6: n* = 8, $R^2$ = ethyl group) (204.73 g: 0.73 mol, yield 73.4%, purity 91.0%) was obtained as a result.

It was verified from the gas chromatography analysis that (11*E*)-11,13-hexadecadienyl acetate (6: *n* = 8, $R^2$ = ethyl group) thus prepared had two types of *cis-trans* isomers [isomer formation ratio = 43.8 (GC Retention time 19.24 min): 56.2 (GC Retention time 19.72 min)] at Retention times 19.24 minutes and 19.72 minutes, respectively. The above yields of (11*E*)-11,13-hexadecadienyl acetate (6: *n* = 8, $R^2$ = ethyl group) are represented as a mixture of the two types of *cis-trans* isomers.

The configurations of the two types of *cis-trans* isomers of (11*E*)-11,13-hexadecadienyl acetate (6: *n* = 8, $R^2$ = ethyl group) were determined by confirming the spin coupling constant between the double bonded hydrogen nuclei from $^1$H-NMR and two-dimensional NMR$^{13}$C non-irradiated HSQC slice data. As a result, the *cis-trans* isomer detected at GC Retention time 19.24 minutes was considered to be (11*E*,13Z)-11,13-hexadecadienyl acetate, and the *cis-trans* isomer detected at GC Retention time 19.72 minutes was considered to be (11*E*,13*E*)-11,13-hexadecadienyl acetate.

The following are the various spectrum data of (11*E*)-11,13-hexadecadienyl acetate (6: *n* = 8, $R^2$ = ethyl group) thus prepared.

Nuclear magnetic resonance spectrum: $^1$H-NMR (600 MHz, $CDCl_3$): δ 0.99 (3H, t, J = 7.5 Hz), 1.24-1.40 (14H, m), 1.61 (2H.quin, J = 7.1 Hz), 2.02-2.10 (6.1H, m), 2.17 (0.9H, quin, J = 7.7 Hz), 4.05 (2H, t, J = 6.9 Hz), 5.29 (0.4H, dt, J = 10.6, 7.7 Hz), 5.53-5.62 (1.2H, m), 5.65 (0.4H, dt, J = 15.0, 7.4 Hz), 5.91 (0.4H, t, J = 10.8 Hz), 5.97-6.03 (1.2H, m), 6.29 (0.4H, dd, J = 14.7, 11.7 Hz) ppm.

$^{13}$C-NMR (150 MHz, $CDCl_3$): δ 13.78, 14.46, 21.14, 21.16, 25.72, 26.04, 28.74, 29.33, 29.36, 29.38, 29.54, 29.57, 29.61, 29.63, 32.74, 33.01, 64.80, 125.62, 128.17, 129.53, 130.45, 131.77, 132.60, 133.99, 134.84, 171.38 ppm.

Spin coupling constant between double bonded hydrogen nuclei determined from $^1$H-NMR and two-dimensional NMR$^{13}$C non-irradiated HSQC slice data: (11E,13Z)-form: $J_{11,12}$ = 14.9 Hz (positions 11-12), $J_{13,14}$ = 10.7 Hz (positions 13-14); (11E,13E)-form: $J_{11,12}$ = 14.9 Hz (positions 11-12), $J_{13,14}$ = 14.9 Hz (positions 13-14).

Mass spectrum: EI (70 eV) (GC Retention time = 19.24 min) *(11E,13Z)-form:* m/z 280 ($M^+$), 251, 220, 177, 149, 135, 121, 95, 82, 79, 67, 55, 43; (GC Retention time = 19.72 min) (11*E*,13*E*)-form: m/z 280 ($M^+$), 251, 220, 177, 149, 135, 121, 95, 82, 79, 67, 55, 43.

Infrared absorption spectrum: (ATR): ν ($cm^{-1}$) 606, 722, 947, 987, 1038, 1237, 1364, 1463, 1742, 2854, 2928, 2962, 3018.

Example 4

The following Example 4-1 describes a preparation of an alcohol compound of the following general formula (7) according to the following reaction formula.

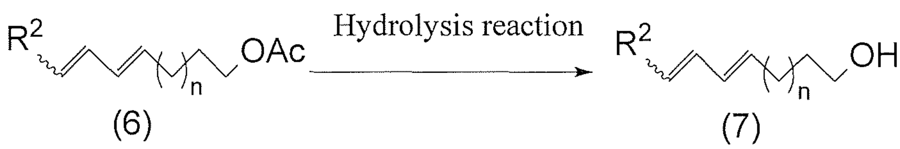

(6) (7)

Example 4-1: Preparation of (4*E*,10*Z*)-4,6,10-hexadecatrienyl alcohol (7: *n* = 1, $R^2$ = (3*Z*)-3-nonenyl group)

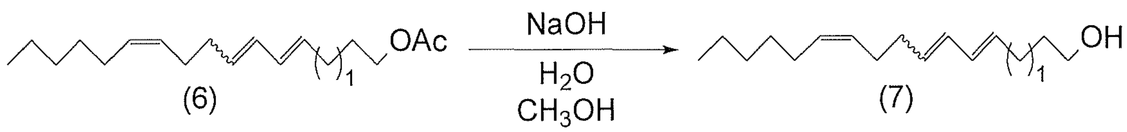

(6) (7)

Air in a reactor equipped with a stirrer, a condenser, and a thermometer was purged with nitrogen. (4*E*,10*Z*)-4,6,10-Hexadecatrienyl acetate (6: *n* = 1, $R^2$ = (3Z)-3-nonenyl group) (278.43 g: 1.00 mol, purity 97.3%) obtained as in Example 3-1 and methanol ($CH_3OH$) (360.0 g) were placed in the reactor and heated to a liquid temperature of 40°C to 45°C. An aqueous solution of 25% by weight sodium hydroxide (188.0 g) was added dropwise to the mixture over 2 hours at a liquid temperature of 45°C to 60°C. After the completion of the dropwise addition, the reaction mixture was stirred for 4 hours at a liquid temperature of 50°C to 60°C. After the completion of the stirring, the reaction mixture was diluted by adding water (200.0 g) to the reactor, and separated into an organic layer and an aqueous layer. The organic layer was washed with an aqueous solution of 20% by weight sodium chloride (250.0 g). The solvent was removed from the organic layer at a reduced pressure, after which the crude product thus obtained was purified by distillation at a reduced pressure to obtain (4*E*,10*Z*)-4,6,10-hexadecatrienyl alcohol (7: *n* = 1, $R^2$ = (3*Z*)-3-nonenyl group) (231.7 g: 0.98 mol, yield 98.6%, purity 97.7%).

It was verified from the gas chromatography analysis that (4*E*,10*Z*)-4,6,10-hexadecatrienyl alcohol (7: *n* = 1, $R^2$ = (3Z)-3-nonenyl group) thus prepared had two types of *cis-trans* isomers [isomer formation ratio = 41.2 (GC Retention time 17.37 minutes: (4*E*,6*Z*,10*Z*)-4,6,10-hexadecatrienyl alcohol): 58.8 (GC Retention time 18.42 minutes: (4*E*,6*E*,10*Z*)-4,6,10-hexadecatrienyl alcohol) at Retention times 17.37 minutes and 18.42 minutes, respectively. The above yields of (4*E*,10*Z*)-4,6,10-hexadecatrienyl alcohol (7: *n* = 1, $R^2$ = (3*Z*)-3-nonenyl group) are represented as a mixture of the two types of *cis-trans* isomers.

The following are the various spectrum data of (4*E*,10*Z*)-4,6,10-hexadecatrienyl alcohol (7: *n* = 1, $R^2$ = (3Z)-3-nonenyl group) thus prepared.

Nuclear magnetic resonance spectrum: $^1H$-NMR (500 MHz, $CDCl_3$): δ 0.82 (3H, t, J = 7.1 Hz), 1.23-1.37 (6H, m), 1.50 (1H, s), 1.63-1.70 (2H, m), 2.02 (2H.quin, J = 6.5 Hz), 2.09-2.23 (6H, m), 3.65 (1.2H, t, J = 6.0 Hz), 3.66 (0.8H, t, J = 6.5 Hz), 5.30-5.41 (2.6H, m), 5.54-5.61 (1H, m), 5.67 (0.4H, dt, J = 14.8, 7.3 Hz) 5.93-6.06 (1.6H, m), 6.34 (0.4H, dd, J = 15.1, 10.9 Hz) ppm.

$^{13}C$-NMR (126 MHz, $CDCl_3$): δ 14.05, 22.54, 27.06, 27.20, 27.28, 28.86, 29.12, 29.34, 29.36, 31.48, 32.25, 32.68, 62.42, 126.19, 128.66, 128.77, 129.77, 130.34, 130.51, 130.61, 130.91, 131.39, 132.21, 133.65 ppm.

Mass spectrum: EI (70 eV) (GC Retention time = 17.37 min) (4*E*,6*Z*,10*Z*)-form: m/z 236 ($M^+$), 177, 147, 125, 107, 91, 79, 67, 55, 41, 29; (GC Retention time = 18.42 min) (4*E*,6*E*,10*Z*)-form: m/z 236 ($M^+$), 177, 147, 125, 107, 91, 79, 67, 55, 41, 29.

Infrared absorption spectrum: (ATR): ν ($cm^{-1}$) 728, 947, 986, 1058, 1448, 2856, 2926, 2954, 3008, 3324.

## Claims

1. A process for preparing an organohalogen compound of the following general formula (5):

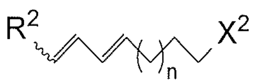

(5)

wherein $R^2$ represents a linear, branched, or aromatic monovalent hydrocarbon group having 1 to 10 carbon atoms, n is the number of methylene groups of 1 to 10, and $X^2$ represents a halogen atom,
the process comprising:
obtaining a reaction product mixture by subjecting a (ω-halo-2-alkenyl)triphenylphosphonium halide compound of the following general formula (1):

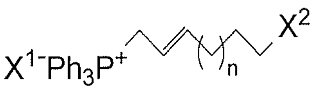

(1)

wherein n is as defined for the general formula (5) above, $X^1$ and $X^2$ represent, independently of each other, a halogen atom, and Ph represents a phenyl group,
to a phosphorus ylide preparation reaction with an alkali metal alkoxide of the following general formula (3):

$R^1OM$ (3)

wherein $R^1$ represents a linear or branched alkyl group having 1 to 6 carbon atoms, and M represents an alkali metal atom,
in the presence of a lithium halide of the following general formula (2):

$LiX^3$ (2)

wherein $X^3$ represents a halogen atom,
and then subjecting the aforesaid reaction product mixture to a Wittig reaction with an aldehyde compound of the following general formula (4):

$R^2CHO$ (4)

wherein $R^2$ is as defined for the general formula (5) above,
to form the organohalogen compound of the general formula (5) above.

**2.** A process for preparing an acetate compound of the following general formula (6):

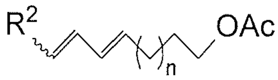

(6)

wherein $R^2$ represents a linear, branched, or aromatic monovalent hydrocarbon group having 1 to 10 carbon atoms, *n* is the number of methylene groups of 1 to 10, and Ac represents an acetyl group,
the process comprising:

the process according to claim 1 for preparing the organohalogen compound (5), and
subjecting the organohalogen compound (5) to an acetoxylation reaction to form the acetate compound (6).

**3.** A process for preparing an alcohol compound of the following general formula (7):

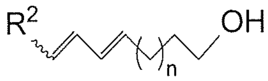

(7)

wherein $R^2$ represents a linear, branched, or aromatic monovalent hydrocarbon group having 1 to 10 carbon atoms, n is the number of methylene groups of 1 to 10,
the process comprising:

the process according to claim 2 for preparing the acetate compound (6), and
subjecting the acetate compound (6) to a hydrolysis reaction to form the alcohol compound (7).

**4.** A process for preparing a (ω-halo-2-alkenyl)triphenylphosphonium halide compound of the following general formula (1):

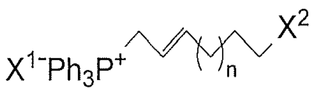

(1)

wherein n is the number of methylene groups of 1 to 10, $X^1$ and $X^2$ represent, independently of each other, a halogen atom, and Ph represents a phenyl group,
the process comprising subjecting a ω-halo-2-alkenyl halide compound of the following general formula (8):

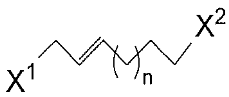

(8)

wherein *n*, $X^1$, and $X^2$ are as defined for the general formula (1) above,
to a substitution reaction with triphenylphosphine to form the (ω-halo-2-alkenyl)triphenylphosphonium halide compound (1).

**5.** A (ω-halo-2-alkenyl)triphenylphosphonium halide compound of the following general formula (1):

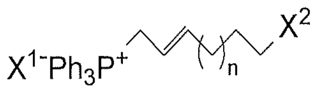

(1)

wherein n is the number of methylene groups of 1 to 10, $X^1$ and $X^2$ represent, independently of each other, a halogen atom, and Ph represents a phenyl group.

**Patentansprüche**

**1.** Ein Verfahren zur Herstellung einer Organohalogenverbindung der folgenden allgemeinen Formel (5):

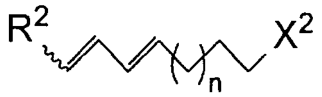

(5)

wobei $R^2$ eine lineare, verzweigte oder aromatische einwertige Kohlenwasserstoffgruppe mit 1 bis 10 Kohlenstoffatomen darstellt, n die Anzahl der Methylengruppen von 1 bis 10 bedeutet und $X^2$ ein Halogenatom darstellt, das Verfahren umfassend:
Erhalten eines Reaktionsproduktgemisches durch Unterwerfen einer (ω-Halogen-2-alkenyl)triphenylphosphoniumhalogenid-Verbindung der folgenden allgemeinen Formel (1):

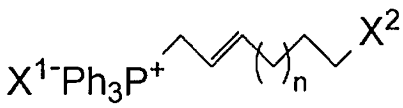

(1)

wobei n wie für die vorstehende allgemeine Formel (5) definiert ist, $X^1$ und $X^2$ unabhängig voneinander ein Halogenatom darstellen und Ph eine Phenylgruppe darstellt,
einer Phosphorylid-Herstellungsreaktion mit einem Alkalimetallalkoxid der folgenden allgemeinen Formel (3):

$R^1OM$ (3)

wobei $R^1$ eine lineare oder verzweigte Alkylgruppe mit 1 bis 6 Kohlenstoffatomen darstellt und
M ein Alkalimetallatom darstellt,
in Gegenwart eines Lithiumhalogenids der folgenden allgemeinen Formel (2):

$LiX^3$ (2)

wobei $X^3$ ein Halogenatom darstellt,
und anschließendes Unterziehen des vorgenannten Reaktionsproduktgemisches einer Wittig-Reaktion mit einer Aldehydverbindung der folgenden allgemeinen Formel (4):

$R^2CHO$ (4)

wobei $R^2$ wie für die vorstehende allgemeine Formel (5) definiert ist,
um die Organohalogenverbindung der vorstehenden allgemeinen Formel (5) zu bilden.

**2.** Ein Verfahren zur Herstellung einer Acetatverbindung der folgenden allgemeinen Formel (6):

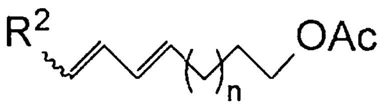

(6)

wobei $R^2$ eine lineare, verzweigte oder aromatische einwertige Kohlenwasserstoffgruppe mit 1 bis 10 Kohlenstoffatomen darstellt, n die Anzahl der Methylengruppen von 1 bis 10 bedeutet und Ac eine Acetylgruppe darstellt,

das Verfahren umfassend:

das Verfahren gemäß Anspruch 1 zur Herstellung der Organohalogenverbindung (5), und
Unterziehen der Organohalogenverbindung (5) einer Acetoxylierungsreaktion, um die Acetatverbindung (6) zu bilden.

**3.** Ein Verfahren zur Herstellung einer Alkoholverbindung der folgenden allgemeinen Formel (7):

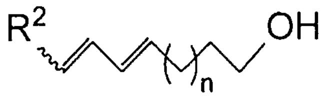
(7)

wobei $R^2$ eine lineare, verzweigte oder aromatische einwertige Kohlenwasserstoffgruppe mit 1 bis 10 Kohlenstoffatomen darstellt, n die Anzahl der Methylengruppen von 1 bis 10 bedeutet,
das Verfahren umfassend:

das Verfahren gemäß Anspruch 2 zur Herstellung der Acetatverbindung (6) und
Unterziehen der Acetatverbindung (6) einer Hydrolysereaktion, um die Alkoholverbindung (7) zu bilden.

**4.** Ein Verfahren zur Herstellung einer (ω-Halogen-2-alkenyl)triphenylphosphoniumhalogenid-Verbindung der folgenden allgemeinen Formel (1):

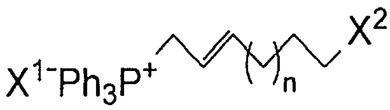
(1)

wobei *n* die Anzahl der Methylengruppen von 1 bis 10 bedeutet, $X^1$ und $X^2$ unabhängig voneinander ein Halogenatom darstellen und Ph eine Phenylgruppe darstellt,
das Verfahren umfassend das Unterziehen einer ω-Halogen-2-alkenylhalogenid-Verbindung der folgenden allgemeinen Formel (8):

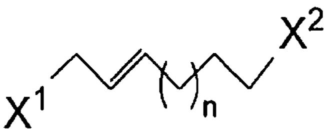
(8)

wobei *n,* $X^1$ und $X^2$ wie für die vorstehende allgemeine Formel (1) definiert sind, einer Substitutionsreaktion mit Triphenylphosphin, um die (ω-Halogen-2-alkenyl)triphenylphosphoniumhalogenid-Verbindung (1) zu bilden.

**5.** Eine (ω-Halogen-2-alkenyl)triphenylphosphoniumhalogenid-Verbindung der folgenden allgemeinen Formel (1):

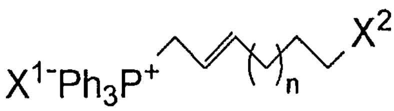
(1)

wobei n die Anzahl der Methylengruppen von 1 bis 10 bedeutet, $X^1$ und $X^2$ unabhängig voneinander ein Halogenatom darstellen und Ph eine Phenylgruppe darstellt.

## Revendications

**1.** Procédé de préparation d’un composé organohalogène de formule générale (5) suivante :

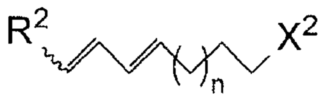

(5)

dans laquelle $R^2$ représente un groupe hydrocarbure monovalent linéaire, ramifié ou aromatique ayant 1 à 10 atomes de carbone, n est le nombre de groupes méthylène de 1 à 10 et $X^2$ représente un atome d'halogène, le procédé comprenant :

l'obtention d'un mélange de produits réactionnels en soumettant un composé halogénure de (ω-halo-2-alcényl)triphénylphosphonium de formule générale (1) suivante :

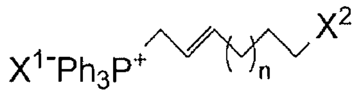

(1)

dans laquelle n est tel que défini pour la formule générale (5) ci-dessus, $X^1$ et $X^2$ représentent, indépendamment l'un de l'autre, un atome d'halogène et Ph représente un groupe phényle, à une réaction de préparation d'ylure de phosphore avec un alcoxyde de métal alcalin de formule générale (3) suivante :

$R^1OM$ (3)

dans laquelle $R^1$ représente un groupe alkyle linéaire ou ramifié ayant 1 à 6 atomes de carbone, et M représente un atome de métal alcalin,
en présence d'un halogénure de lithium de formule générale (2) suivante :

$LiX^3$ (2)

dans laquelle $X^3$ représente un atome d'halogène,
puis la soumission dudit mélange de produits réactionnels à une réaction de Wittig avec un composé aldéhyde de formule générale (4) suivante :

$R^2CHO$ (4)

dans laquelle $R^2$ est tel que défini pour la formule générale (5) ci-dessus,
pour former le composé organohalogène de formule générale (5) ci-dessus.

**2.** Procédé de préparation d'un composé acétate de formule générale (6) suivante :

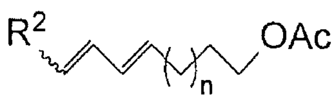

(6)

dans laquelle $R^2$ représente un groupe hydrocarbure monovalent linéaire, ramifié ou aromatique ayant 1 à 10 atomes de carbone, n est le nombre de groupes méthylène de 1 à 10 et Ac représente un groupe acétyle, le procédé comprenant :

le procédé selon la revendication 1 de préparation du composé organohalogène (5), et
la soumission du composé organohalogène (5) à une réaction d'acétoxylation pour former le composé acétate (6).

**3.** Procédé de préparation d'un composé alcoolique de formule générale (7) suivante :

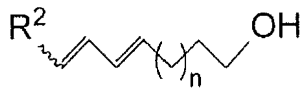

(7)

dans laquelle $R^2$ représente un groupe hydrocarbure monovalent linéaire, ramifié ou aromatique ayant 1 à 10 atomes de carbone, n est le nombre de groupes méthylène de 1 à 10,
le procédé comprenant :

le procédé selon la revendication 2 de préparation du composé acétate (6), et
la soumission du composé acétate (6) à une réaction d'hydrolyse pour former le composé alcoolique (7).

**4.** Procédé de préparation d'un composé halogénure de (ω-halo-2-alcényl)triphénylphosphonium de formule générale (1) suivante :

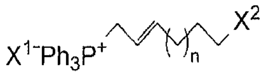

(1)

dans laquelle n est le nombre de groupes méthylène de 1 à 10, $X^1$ et $X^2$ représentent, indépendamment l'un de l'autre, un atome d'halogène et Ph représente un groupe phényle, le procédé comprenant la soumission d'un composé halogénure de ω-halo-2-alcényle de formule générale (8) suivante :

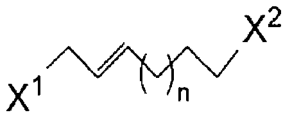

(8)

dans laquelle n, $X^1$ et $X^2$ sont tels que définis pour la formule générale (1) ci-dessus,
à une réaction de substitution avec de la triphénylphosphine pour former le composé halogénure de (ω-halo-2-alcényl)triphénylphosphonium (1).

**5.** Composé halogénure de (ω-halo-2-alcényl)triphénylphosphonium de formule générale (1) suivante :

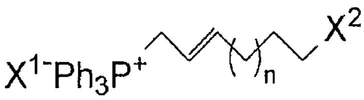

(1)

dans laquelle n est le nombre de groupes méthylène de 1 à 10, $X^1$ et $X^2$ représentent, indépendamment l'un de l'autre, un atome d'halogène et Ph représente un groupe phényle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3556742 A1


### Non-patent literature cited in the description


- **P. S. BEEVOR et al.** *J. Chem. Ecol.*, 1986, vol. 12, 1-23
- **YAO-PIN YEN et al.** *Synth. Commun.*, 1992, vol. 22, 1567-1581
- **E. BUCHTA et al.** *Justus Liebigs Annalen der Chemie*, vol. 640 (1), 29-37